# EUROPEAN PATENT SPECIFICATION

(11) **EP 4 114 490 B1**
(45) Date of publication and mention of the grant of the patent: **14.10.2026**
(21) Application number: 21765270.0
(22) Date of filing: 02.03.2021
(51) Int. Cl.: A61M 16/06

(54) **PATIENT INTERFACE SYSTEM**
PATIENTENSCHNITTSTELLENSYSTEM
SYSTÈME D'INTERFACE PATIENT

(30) Priority: 03.03.2020 US 202062984718 P; 23.11.2020 US 202063117044 P
(43) Date of publication of application: 11.01.2023
(60) Divisional application: 26202398.9 / 4 821 531
(73) Proprietor: Fisher & Paykel Healthcare Limited, East Tamaki Auckland 2013 (NZ)
(72) Inventor: STANLEY, Leon Tyler, Auckland 2013 (NZ); HOWARTH, Brad Michael, Auckland 2013 (NZ); RONAYNE, Michael Paul, Auckland 2013 (NZ); DAVIS, Samuel Rollo Ross, Auckland 2013 (NZ); PEMMARAJU, Hemanth, Auckland 2013 (NZ); MICHELSEN, Larissa Grace, Auckland 2013 (NZ); MCGERTY, Oscar Elliot James, Auckland 2013 (NZ); BECKLEY, Amelia Rhian, Auckland 2013 (NZ); MEECH, Julio Derek, Auckland 2013 (NZ)
(74) Representative: Forresters IP LLP
(86) International application number: PCT/IB2021/051707
(87) International publication number: WO 2021/176338

(56) References cited:
- EP-A2- 2 022 528
- WO-A1-2005/079726
- WO-A1-2007/048174
- WO-A1-2007/048174
- WO-A1-2012/047121
- WO-A1-2016/157040
- WO-A1-2017/037638
- WO-A2-2005/076874
- WO-A2-2010/023590
- US-A1- 2007 000 492
- US-A1- 2011 041 855
- US-A1- 2015 075 531
- US-A1- 2015 335 846
- US-A1- 2016 015 921
- US-A1- 2016 121 068
- US-A1- 2020 023 153

## Description

### FIELD

The present disclosure generally relates to components for medical systems for conveying gases to and/or from a patient. In one particular aspect, the disclosure relates to patient interfaces that form a part of a breathing system.

### BACKGROUND

In assisted breathing, respiratory gases are supplied to a patient through a patient interface via one or more flexible breathing tubes. The patient interface can be a nasal cannula, nasal mask, full face or oro-nasal mask, endotracheal tube, or other known types of interfaces. The gases expired by the patient may be channeled through a similar breathing tube to other equipment (valves, ventilators, pressure devices, or the like) or expelled to the patient's surroundings.

In medical applications, such as assisted breathing, the gases inhaled by a patient are preferably delivered close to body temperature (usually between 33°C and 37°C) and with a high relative humidity (commonly near saturation). In other medical applications, such as continuous positive airway pressure (CPAP) systems or positive pressure ventilations systems that provide patient's suffering obstructive sleep apnea (OSA) with positive pressure breathing gases, the breathing gases may be heated and/or humidified to varying levels to improve user comfort or supplied without heating or humidification.

US 2016/121068 A1 discloses a patient interface device that includes a support member and a sealing assembly attachable to the support member.

In the specification where reference has been made to patent specifications, other external documents, or other sources of information, this is generally for the purpose of providing a context for discussing the features of the disclosure. Unless specifically stated otherwise, reference to such external documents is not to be construed as an admission that such documents, or such sources of information, in any jurisdiction, are prior art, or form part of the common general knowledge in the art.

Further aspects and advantages of the present disclosure will become apparent from the ensuing description which is given by way of example only.

### SUMMARY

It would be advantageous to provide a system for gas delivery treatment with interchangeable patient interface, such as a nasal cannula, nasal mask, or oro-nasal mask. Such an alternative or improved system may further assist with improved compliance and flexibility of gas delivery treatment.

The invention provides a patient interface assembly as defined in the appended independent claim 1.

An aspect of the present disclosure involves a patient interface assembly including a first interface body, a second interface body, and a housing configured to detachably couple with the first interface body or the second interface body. The first interface body and the second interface body are interchangeable, such that the first interface body is coupled to the housing in a first configuration, and the second interface body is coupled to the housing in a second configuration.

In some configurations, the first interface body is a cannula body.

In some configurations, the cannula body comprises one or more nasal prongs.

In some configurations, the one or more prongs are configured to be inserted into nares of a user and form a seal.

In some configurations, the second interface body is a mask body.

In some configurations, the second interface body is a nasal mask.

In some configurations, the second interface body is an oro- nasal mask.

In some configurations, the mask defines a breathing chamber and having a user-contacting surface configured to contact a face of the user and surround at least the nares of the user.

In some configurations, the housing comprises an upper edge and a lower edge.

In some configurations, the upper edge and the lower edge of the housing are symmetrical.

In some configurations, the lower edge of the housing is curved inwardly to the housing.

In some configurations, each of the first interface body and the second interface body comprises a manifold, wherein the manifold is configured to be at least partially received by the housing when the first interface body or the second interface body is detachably coupled to the housing.

In some configurations, the manifold comprises a housing coupling portion configured to be received within the housing when the first interface body or the second interface body is detachably coupled to the housing.

In some configurations, the manifold further comprises a raised portion adjacent the housing coupling portion, wherein the raised portion is configured to inhibit rotation of the manifold about the housing when the first interface body or the second interface body is detachably coupled to the housing.

In some configurations, the housing and/or the manifold comprises a feedback mechanism to provide a visual or audible feedback when the first interface body or the second interface body is detachably coupled to the housing.

In some configurations, the patient interface assembly further includes one or more interface tubes.

In some configurations, the housing comprises one or more tube engaging receiving portions configured to receive interface tubes.

In some configurations, the patient interface assembly further includes a securement member configured to be attached to the housing.

In some configurations, the first interface body can comprise a mask or nasal prongs of a first size and the second interface body comprises a mask or nasal prongs of a second size, wherein the first size is different from the second size.

An aspect of the present disclosure involves a patient interface including, an interface body, a housing, one or more interface tubes, a first securement member and a second securement member extending vertically for providing vertical stability to the patient interface.

In some configurations, the interface body is configured to be engaged on the patient's face and form a seal.

In some configurations, the interface body is a mask body or a cannula body.

In some configurations, the one or more tubes are configured to be coupled to the housing.

In some configurations, the one or more tubes are formed as a single piece with the housing.

In some configurations, each of the one or more tubes comprises an overmoulded grip.

In some configurations, the first securement member includes a central portion, a pair of support pads and a pair of bridging portions. Each of the support pads are configured to engage a securing mechanism at the face of a user on opposing sides of the central portion. Each of the bridging portions connects the central portion and a respective one of the support pads.

In some configurations, the first securement member further comprises a patch assembly coupled to each of the support pads for securing the support pads to the user.

In some configurations, the second securement member is coupled to the housing.

In some configurations, the second securement member is configured to engage the forehead of a user.

In some configurations, the second securement member is constructed from a fabric material

In some configurations, the second securement member further comprises a patch assembly for securing the second securement member to the user.

In some configurations, the interface body has a Shore hardness between 20A-100A.

In some configurations, each of the one or more interface tubes is tapered toward one end.

An aspect of the present disclosure involves a patient interface including an interface body. The interface body includes one or more prongs extending from a base region at the interface body, and one or more openings to allow the flow of respiratory gases into and from the interface body. The one or more prongs are configured to flex at the base region while maintaining seal with the nares of the user.

In some configurations, the interface body comprises a right prong and a left prong.

In some configurations, a thickness of the one or more prongs varies along the length of the one or more prongs.

In some configurations, a thickness of the one or more prongs maintains a constant thickness along the length of the one or more prongs.

In some configurations, the base region has a thickness smaller than the rest of the interface body.

In some configurations, the base region has a surface recessed relative to the adjacent region of the interface body.

In some configurations, the base region has a recessed surface around the one or more prongs.

In some configurations, the base region has a recessed surface extending around two prongs.

In some configurations, the one or more prongs are configured to flex in forward and rear directions.

In some configurations, the one or more prongs are configured to flex in right and left directions.

In some configurations, the one or more prongs are configured to flex in right and left directions.

In some configurations, the one or more prongs have a circular cross-section adjacent at a top end of the prongs.

In some configurations, the one or more prongs are configured to flex while maintaining their diameter and cross-sectional shape along their lengths.

In some configurations, the one or more prongs are configured to flex while maintaining their resistance to flow (RTF).

An aspect of the present disclosure involves a respiratory system including a gas flow source, an inspiratory conduit fluidically connected to the gas flow source, and a patient interface. The patient interface includes an interface body, a housing, one or more interface tubes; and a first securement member.

In some configurations, the respiratory system further includes an expiratory conduit configured to receive gases from the patient interface.

In some configurations, the respiratory system further includes a bubbler device.

In some configurations, the respiratory system further includes a humidifier.

In some configurations, the housing is configured to receive each of a plurality of interface bodies, such that the interface bodies are interchangeable.

In some configurations, the interface body is a mask body or a cannula body.

In some configurations, the one or more interface tubes are configured to be coupled to the housing.

In some configurations, one of the one or more interface tubes is configured to be connected to the inspiratory conduit.

In some configurations, the one or more tubes are formed as a single piece with the housing.

In some configurations, each of the one or more tubes comprises an overmoulded grip.

In some configurations, the first securement member comprises a central portion, a pair of support pads and a pair of bridging portions. Each of the support pads is configured to engage the face of a user on opposing sides of the central portion. Each of the bridging portions connects the central portion and a respective one of the support pads.

In some configurations, the first securement member further comprises a patch assembly coupled to each of the support pads for securing the support pads to the user.

In some configurations, the patient interface further comprises a second securement member is coupled to the housing.

In some configurations, the second securement member is configured to engage the forehead of a user.

In some configurations, the second securement member is constructed from a fabric material

In some configurations, the second securement member further comprises a patch assembly for securing the second securement member to the user.

In some configurations, the respiratory system further incudes a headgear or a bonnet.

In aspect of the present disclosure involves a securement member for a patient interface of a respiratory system including a soft, flexible barb for detachably coupling with a housing of the patient interface.

In some configurations, the barb comprises one or more tabs.

In some configurations, the housing comprises an opening, wherein the one or more tabs are configured to catch on the housing at the opening of the housing.

In some configurations, the barb includes a cutout. The cutout is configured to aid in collapsing of the barb.

In some configurations, the barb comprises an extended top portion that is configured to prevent the barb from being over inserted into the opening of the housing.

In some configurations, the barb is overmoulded onto the securement member.

In aspect of the present disclosure involves a securement member for a patient interface of a respiratory system including a support pad that secures to a patch assembly on a patient, wherein the support pad is hinged relative to the rest of the securement member.

In some configurations, the support pad is flexible.

In some configurations, the securement member further comprising a hinge between the support pad and a portion of the securement member.

In some configurations, the support pad is overmoulded onto the securement member.

In some configurations, the hinge is a 180-degree hinge.

An aspect of the present disclosure involves a securement member for a patient interface of a respiratory system including a first flexible portion connectable with a patient interface component, a second flexible portion connectable to a patient's forehead, and a third rigid portion extending between the first and second portions.

In some configurations, the first flexible portion is a barb.

In some configurations, the second flexible portion is a support pad.

In some configurations, the first and/or second flexible portions are overmoulded onto the third rigid portion.

In some configurations, the second flexible portion is hinged relative to the third rigid portion.

In some configurations, the securement member is detachably connectable to housing of the patient interface.

In some configurations, the component is a housing or interface body.

An aspect of the present disclosure involves a patient interface assembly including an interface body comprising a manifold comprising a front side configured to contact the patient and a rear side opposite the front side, wherein the manifold comprises a raised portion of a housing coupling portion positioned between the rear side of the manifold and the front side of the manifold, the manifold comprising a width that runs along an inner lumen of the manifold and a horizontal axis that runs along the width, wherein the raised portion is angled with respect to the horizontal axis, wherein the angled raised portion of the housing coupling portion is configured to improve the assembly and alignment of the interface body when it detachably couples with a housing via the manifold; a housing comprising a front patient facing side and a rear side opposite the front side, wherein the housing comprises a width that extends from a first tube engaging portion on one end of the housing to a second tube engaging portion on an opposite end of the housing and a horizontal axis that runs along the width, wherein the housing comprises an interface body receiving portion between the front side of the housing and the rear side of the housing, wherein the interface body receiving portion is angled with respect to the horizontal axis, wherein the angled interface body receiving portion is configured to improve the assembly and alignment when the manifold is detachably coupled and received by the housing.

In some configurations, the raised portion of the manifold comprises a first width on the front side of manifold that is greater than a second width on the rear side of the manifold.

In some configurations, the raised portion of the manifold comprises a first width on the front side of manifold that is less than a second width on the rear side of the manifold.

In some configurations, the angled raised portion of the housing coupling portion of the manifold extends at an angle of 120 degrees from the rear side of the manifold.

In some configurations, the angled raised portion of the housing coupling portion of the manifold extends at an angle of 60 degrees from the rear side of the manifold.

In some configurations, the angled raised portion of the housing coupling portion of the manifold extends at an angle of between 30 degrees and 170 degrees from the rear side of the manifold.

In some configurations, the patient interface assembly further comprising an engagement surface where the raised portion of the housing coupling portion and the interface body receiving portion contact each other, wherein a portion of the raised portion of the manifold at the engagement surface is higher relative to the engagement surface of the housing.

In some configurations, the manifold comprises a portion on either side of manifold that is configured to extend within the housing, wherein the portion that extends within the housing has a ridge configured to seal to the housing.

In some configurations, the ridge is an annular ridge.

In some configurations, the ridge is configured to seal with the internal surface of housing.

In some configurations, the manifold has a first distance between the front side of the engagement surface and the annular ridge and a second distance between the rear side of the engagement surface and the annular ridge, wherein the second distance is greater than the first distance.

In some configurations, the front side of the manifold has a first wall thickness and the rear side of the manifold has a second wall thickness, wherein the first wall thickness is thicker than the second wall thickness.

An aspect of the present disclosure involves a patient interface assembly involving a housing that detachably couples with a manifold of an interface body, wherein the housing comprises a side protrusion and a central protrusion, wherein the side protrusion comprises features for coupling a first securement member to the housing and wherein the central protrusion comprises one or more features for coupling a second securement member to the housing.

In some configurations, the housing is a rigid housing.

In some configurations, the one or more features for coupling with a second securement member to the housing are one or more openings.

In some configurations, the second securement member is detachably coupled with the housing.

In some configurations, the one or more openings comprise a lip configured to catch a complementary fitting portion of the second securement member.

In some configurations, the features of the side protrusion comprise an enlarged head and a stem for a push-fit coupling.

In some configurations, the first securement member is detachably coupled with the housing.

In some configurations, the patient interface assembly further comprising a tubing, wherein the tubing is integral with the housing.

In some configurations, the patient interface assembly further comprising a tubing, wherein the tubing is detachably coupled with the housing.

An aspect of the present disclosure involves a securement member for a patient interface of a respiratory system including a central portion, a pair of support pads, each of the support pads configured to engage the face of a user on opposing sides of the central portion, and a pair of bridging portions, each of the bridging portions connecting the central portion and a respective one of the support pads, wherein the bridging portions comprise cutouts for receiving a housing, wherein the bridging portions comprise a thickened portion surrounding the cutouts.

In some configurations, the securement member further comprises a patch assembly coupled to each of the support pads for securing the support pads to the user.

In some configurations, the securement member further comprises openings configured to detachably couple the securement member to protrusions on a housing, wherein the first securement member is push-fit over the protrusions on the housing.

In some configurations, the securement member further comprises a recess on the rear side of the securement member, wherein the recess is configured to accommodate for a central protrusion on the housing when the securement member is coupled to the housing.

An aspect of the present disclosure involves a patient interface assembly of a respiratory system including an interface body; and a housing, wherein the interface body is detachably coupled to the housing; and a feature that limits rotation of the interface body relative to the housing.

In some configurations, the interface body comprises a manifold and the feature comprises a shoulder portion on the rear of the manifold.

In some configurations, the shoulder portion abuts against the housing to limit rotation.

In some configurations, the feature comprises a keyed feature on manifold and housing.

In some configurations, the interface body comprises a cannula body.

In some configurations, the interface body comprises a mask body.

In some configurations, the housing comprises one or more tube engaging receiving portions configured to receive interface tubes.

An aspect of the present disclosure involves a patient interface assembly of a respiratory system including a housing, an inspiratory conduit with a first end and a second end, the first end configured to connect the housing and deliver incoming flow of gases and the second end with locking fingers, and an expiratory conduit with first end and second end, the first end configured to connect a housing and receive flow of expiratory gases and the second end with locking fingers.

In some configurations, the locking fingers at the second end of the expiratory conduit are configured to couple to a downstream component.

In some configurations, the downstream component comprises a bubbler.

In some configurations, the locking fingers at the second end of the inspiratory conduit are configured to couple to an upstream component.

In some configurations, the upstream component comprises a flow source.

In some configurations, the locking fingers extend away from a connector, the locking fingers being spaced apart and narrowing along their length away from the connector.

In some configurations, the locking fingers comprising locking recesses that are formed at least on outer surfaces of each of the locking fingers, the locking recesses being configured to lock with portions of a gas delivery tube connector, the locking fingers being configured to interact with recesses of the gas delivery tube connector to align the connector and the gas delivery tube connector.

An aspect of the present disclosure involves a patient interface assembly of a respiratory system including an interface body, and a housing, wherein at least a portion of an external surface of the housing and/or the interface body comprises a low friction surface coating or finish.

In some configurations, the interface body comprises a cannula body.

In some configurations, the interface body comprises a mask body.

In some configurations, the low friction surface coating or finish is a parylene coating or achieved by a blasting process, such as bead blasting.

In some configurations, the interface body comprises a rear side comprising patient contacting surface and a front side opposite the rear side, wherein the rear side has a low friction surface coating or finish.

In some configurations, the interface body is detachable with the housing and the interface body comprises a manifold with a low friction surface coating or finish that engages with the housing.

An aspect of the present disclosure involves a manufacturing process for a patient interface assembly for a respiratory system including manufacturing an interface body and coating an external surface of the interface body in a low friction surface coating or finish.

In some configurations, the interface body comprises a cannula body.

In some configurations, the interface body comprises a mask body.

In some configurations, the interface body is detachable with a housing.

In some configurations, substantially an entire external surface of the interface body is coated.

In some configurations, a portion of the interface body is coated, wherein the interface body comprises a rear patient facing side of the interface body and a front side opposite the rear side, wherein the coated portion is a rear portion of a manifold of the interface body.

In some configurations, a portion of the interface body is coated, wherein the interface body comprises a rear patient facing side of the interface body and a front side opposite the rear side, wherein the coated portion is a front portion of a manifold of the interface body.

An aspect of the present disclosure involves a patient interface assembly including an interface body including a manifold comprising a front side configured to contact the patient and a rear side opposite the front side, wherein the manifold comprises a raised portion of a housing coupling portion positioned between the rear side of the manifold and the front side of the manifold, wherein the manifold comprising a width that runs along an inner lumen of the manifold and a horizontal axis that runs along the width, wherein the raised portion is angled with respect to the horizontal axis, wherein the angled raised portion of the housing coupling portion is configured to improve the assembly and alignment of the interface body when it detachably couples with a housing via the manifold, and a feature that limits rotation of the interface body relative to the housing, and the housing including a front patient facing side, a rear side opposite the front side, wherein the housing comprises a width that extends from a first tube engaging portion on one end of the housing to a second tube engaging portion on an opposite end of the housing and a horizontal axis that runs along the width, wherein the housing comprises an interface body receiving portion between the front side of the housing and the rear side of the housing, wherein the interface body receiving portion is angled with respect to the horizontal axis, wherein the angled interface body receiving portion is configured to improve the assembly and alignment when the manifold is detachably coupled and received by the housing, a first securement member comprising a central portion, a pair of support pads, each of the support pads configured to engage the face of a user on opposing sides of the central portion, and a pair of bridging portions, each of the bridging portions connecting the central portion and a respective one of the support pads wherein the bridging portions comprise cutouts for receiving the housing, wherein the bridging portions comprise a thickened portion surrounding the cutouts, a second securement member comprising a first flexible portion connectable with a patient interface component, a second flexible portion connectable to a patient's forehead, and a third rigid portion extending between the first and second portions, an inspiratory conduit with a first end and a second end, the first end configured to connect to the housing and deliver incoming flow of gases and the second end with locking fingers, and an expiratory conduit with first end and second end, the first end configured to connect to the housing and receive flow of expiratory gases and the second end with locking fingers.

In some configurations, the interface body comprises a cannula body.

In some configurations, the interface body comprises a mask body.

In some configurations, the inspiratory and expiratory conduit are integral with the housing.

In some configurations, the first and second securement members are connectable with the housing.

In some configurations, the second securement member is connectable with a patch assembly or headgear of a patient.

In some configurations, the second securement member is detachably coupled to the housing.

In some configurations, the interface body is detachably coupled to the housing.

An aspect of the present disclosure involves a patient interface assembly comprising an interface body, and a housing configured to detachably couple with the interface body, the housing comprises one or more tube engaging portions at lateral entry points on opposite ends of the housing, wherein the tube engaging portions are configured to receive interface tubes.

In some configurations, the interface body can comprise a cannula body.

In some configurations, the interface body can comprise a mask body.

An aspect of the present disclosure involves a patient interface assembly comprising a first interface body, a second interface body, and a housing configured to detachably couple with the first interface body or the second interface body, the housing comprises one or more tube engaging portions at lateral entry points of the housing, wherein the tube engaging portions are configured to receive interface tubes, wherein the first interface body and the second interface body are interchangeable, such that the first interface body is coupled to the housing in a first configuration, and the second interface body is coupled to the housing in a second configuration.

An aspect of the present disclosure involves an interface body comprising a mask comprising a breathing chamber and having a user-contacting surface configured to contact a face of the user and surround at least the nares of the user, and a manifold in fluid communication with the breathing chamber, the manifold comprising a first opening on a first lateral side of the manifold and a second opening on the second lateral side of the manifold, wherein the first and second openings permit gas flow into and out of the breathing chamber.

In some configurations, the first and second openings can be configured to be in communication with interface tubes configured to deliver a flow of gas entering and exiting the mask and the first and second lateral side of the manifold.

An aspect of the present disclosure involves an interface body comprising a mask, wherein the mask defines a breathing chamber and having a user-contacting surface configured to contact a face of the user and surround at least the nares of the user, the mask comprising a mask seal, wherein at least part of the mask seal comprises a region of reduced stiffness, wherein the region of reduced stiffness is positioned between a first stiffer region and a second stiffer region, wherein the first and second stiffer regions have a stiffness greater than the stiffness of the region of reduced stiffness, wherein when the first stiffer region is moved toward the second stiffer region, the region of reduced stiffness buckles in a single direction as the first stiffer region continues to move towards the second stiffer region.

In some configurations, the first stiffer region can comprise a structural support portion extending partially around the circumference of the mask.

In some configurations, the second stiffer region can be a front portion of the mask.

An aspect of the present disclosure involves a patient interface assembly comprising an interface body comprising a housing coupling portion, the housing coupling portion comprise a first patient facing side and an opposite second side, wherein the housing coupling portion comprises a shape defined between the first side and the second side of the housing coupling portion, and a housing comprising an interface body receiving portion, wherein the interface body receiving portion is a complementary shape to the shape of the housing coupling portion of the interface body, wherein the housing is configured to detachably coupled with the interface body.

In some configurations, the shape of the housing coupling portion can comprise a trapezoidal shape.

In some configurations, the housing coupling portion of the interface body can comprise a wider base of the trapezoid proximal to the front portion of the housing.

In some configurations, the housing coupling portion of the interface body can comprise a wider base of the trapezoid proximal to the rear portion of the housing.

An aspect of the present disclosure involves an interface body comprising one or more prongs extending from a base region and one or more openings to allow the flow of respiratory gases into and from the prongs, wherein the one or more prongs are configured to flex at the base region while maintaining seal with the nares of the user.

In some configurations, the one or more prongs can comprise a thickness that varies along the length of the one or more prongs.

In some configurations, the one or more prongs can maintain a constant thickness along the length of the one or more prongs.

An aspect of the present disclosure involves an interface body, wherein the interface body can comprise one or more prongs extending from a base region at the interface body, and one or more openings to allow the flow of respiratory gases into and from the interface body, wherein the one or more prongs are configured to flex at the base region while maintaining seal with the nares of the user.

### BRIEF DESCRIPTION OF THE DRAWINGS

Throughout the drawings, reference numbers can be reused to indicate general correspondence between reference elements. The drawings are provided to illustrate example embodiments described herein and are not intended to limit the scope of the disclosure.
FIG. 1 shows an example of a system in which embodiments of the patient interface can be used.
FIG. 2 shows an example of a system in which embodiments of the patient interface can be used.
FIG. 3 is a front, top and side perspective view of a patient interface according to an example embodiment of the present invention.
FIG. 4 is a front, top and side perspective view of the patient interface of FIG. 3.
FIG. 5 is a front, top and side perspective view of a patient interface according to an example embodiment of the present invention.
FIG. 6 is a front, top and side perspective view of the patient interface of FIG. 5.
FIG. 7 is a front, top and side perspective view of a housing.
FIG. 8 is a front view of the housing of FIG. 7.
FIG. 9 is a rear view of the housing of FIG. 7
FIG. 10 is a front, top and side perspective view of a nasal interface.
FIG. 11 is a front view of the nasal interface of FIG. 10.
FIG. 12A is a top view of the nasal interface of FIG. 10.
FIG. 12B is a rear view of the nasal interface of FIG. 10.
FIGS. 13A-13D representation of the nasal prongs of a cannula body.
FIGS. 13E-13I illustrate views of nasal prongs of a cannula body.
FIG. 14A is a schematic drawing of the nasal interface of FIG. 10 showing a thickness analysis.
FIG. 14B is a schematic drawing of a nasal interface showing a thickness analysis.
FIG. 14C is a cross-sectional view of a nasal prong of a cannula body.
FIG. 15 is a rear, top and side perspective view of a patient interface.
FIG. 16 is a front, top and side perspective view of a patient interface.
FIG. 17 is a front view of the patient interface of FIG. 16.
FIG. 18 is a top view of the patient interface of FIG. 16.
FIG. 19 is a rear view of the patient interface of FIG. 16.
FIGS. 20-21 are schematic drawings of the patient interface of FIG. 16.
FIG. 22A is a front, top and side perspective view of a nasal interface.
FIG. 22B is a rear, top and side perspective view of the nasal interface of FIG. 22A.
FIG. 23 is a schematic drawing of the nasal interface of FIG. 22A.
FIG. 24A is a rear, top and side perspective view of a nasal interface.
FIG. 24B is a top, cross-sectional view of a nasal interface.
FIG. 24C is a side, cross-sectional view of a nasal interface.
FIG. 24D is a schematic of a top cross-section of a nasal interface.
FIG. 24E is a schematic of a side cross-section of a nasal interface.
FIG. 25 is a front, top and side perspective view of a patient interface.
FIG. 26 is a top view of the patient interface of FIG. 25.
FIG. 27 is a front, top and side perspective view of a securement member.
FIG. 28 is a front view of the securement member of FIG. 27.
FIG. 29 is a front, top and side view of a securement member and a housing.
FIG. 30 is a rear view of the securement member and the housing of FIG. 29.
FIG. 31 is a schematic drawing of a securement member.
FIG. 32 is a schematic drawing of a securement member.
FIG. 33 is a front, top and side view of a securement member.
FIG. 34 is a front view of the securement member of FIG. 33.
FIG. 35 is a rear view of the securement member of FIG. 33.
FIG. 36 is a front, top, side perspective view of a patient interface assembly according to an example embodiment of the present invention.
FIG. 37 is a top view of a housing.
FIG. 38 is a top perspective view of an interface housing.
FIG. 39A is a front perspective view of a securement member.
FIG. 39B is a rear perspective view of a securement member.
FIG. 39C is a top view of a securement member.
FIGS. 40-42 are front perspective views of a securement member.
FIG. 43A is a front view of a securement member and a housing.
FIG. 43B is a top view of a housing.
FIG. 44 is a front, side perspective view of a securement member.
FIG. 45 is a view of a securement member.
FIG. 46A-46B is a front and rear side perspective view of a securement member.
FIG. 47 is a front side perspective view of a securement member and housing.
FIG. 48A is a rear view of a cannula body.
FIG. 48B is a front view of a mask body.
FIG. 48C is a front view of a schematic of a mask body.
FIG. 48D is a front, side perspective view of a schematic of a mask body.
FIG. 48E is a top view of a schematic of a mask body.
FIG. 49 is a top view of a nasal interface and housing.
FIG. 50 is a partial top view of a nasal interface and housing.
FIGS. 51A-51B is a partial top view of a nasal interface and housing.
FIG. 52A is a top view of a nasal interface and housing.
FIG. 52B is a rear view of the nasal interface and housing of FIG. 52A.
FIG. 53A is a top view of a nasal interface and housing.
FIG. 53B is a partial top view of the nasal interface and housing of FIG. 53A.
FIG. 53C is a partial top view of the nasal interface and housing of FIG. 53A.
FIG. 53D is a partial top view of the nasal interface and housing of FIG. 53A.
FIG. 54 is a side cross section view of an interface body.
FIG. 55 is a side cross section view of an interface body.
FIGS. 56A-56D are views of an interface body and housing.
FIGS. 56E-56F are views of an interface body, housing, and tubing of the interface assembly.
FIG. 57 is a front perspective view of an interface tubing and connector.

### DETAILED DESCRIPTION

Embodiments of systems, components and methods of assembly and manufacture will now be described with reference to the accompanying figures, wherein like numerals refer to like or similar elements throughout. Although several embodiments, examples and illustrations are disclosed below, it will be understood by those of ordinary skill in the art that the inventions described herein extends beyond the specifically disclosed embodiments, examples and illustrations, and can include other uses of the inventions and obvious modifications and equivalents thereof. The terminology used in the description presented herein is not intended to be interpreted in any limited or restrictive manner simply because it is being used in conjunction with a detailed description of certain specific embodiments of the inventions. In addition, embodiments of the inventions can comprise several novel features and no single feature is solely responsible for its desirable attributes or is essential to practicing the inventions herein described.

Certain terminology may be used in the following description for the purpose of reference only, and thus are not intended to be limiting. For example, terms such as "above" and "below" refer to directions in the drawings to which reference is made or directions relative to an orientation of the device as used with the user in an upright position. Terms such as "front," "back," "left," "right," "rear," and "side" describe the orientation and/or location of portions of the components or elements within a consistent but arbitrary frame of reference which is made clear by reference to the text and the associated drawings describing the components or elements under discussion or relative to an orientation of the device as used with the user in an upright position.

### Respiratory System

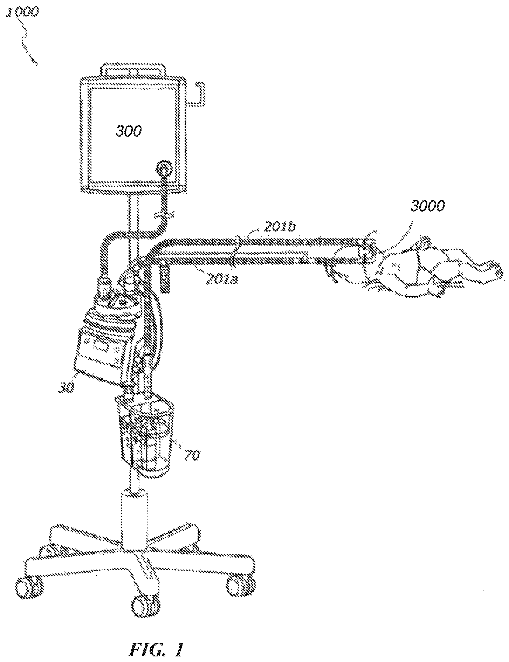

FIG. 1 shows an example respiratory system 1000 in which embodiments of a patient interface 3000 can be used. In the illustrated arrangement, the patient interface 3000 receives an inspiratory flow of gases via an inspiratory conduit 201a. A flow of the expiratory gases can be directed from the interface 3000 via an expiratory conduit 201b to a resistance device, which in the illustrated arrangement is a bubbler device 70. An optional humidifier system 30 is provided to humidify the inspiratory flow of gases. The humidifier system 30 typically includes a chamber sitting atop a heater base, the chamber of which is fed with a source of gases flow from, for example, a hospital or other supply source 300. The humidified inspiratory flow of gases is delivered to the airway of the patient by inspiratory conduit 201a and the patient interface 3000. Excess and expired gases are evacuated from the patient interface 3000 by the expiratory conduit 201b. The resistance device 70 provides resistance to the expiratory flow of the gases exiting the system 1000 to the atmosphere to provide a desirable peak end expiratory pressure (PEEP). One of skill in the art will understand that such a system may include additional and/or replacement components as are known in the art. In some embodiments, the patient interface 3000 includes a nasal cannula. In other embodiments, the patient interface 3000 includes a mask. For example, the patient interface 3000 may include a nasal mask, an oro-nasal mask, an oral mask or a full-face mask. In some embodiments, the resistance device and/or the humidifier is integrated into the supply source 300. Although a water-based resistance device is shown in FIG. 1, it should be appreciated by one of skill that the resistance device may be any other mechanical or electrical resistance device as is known in the art.

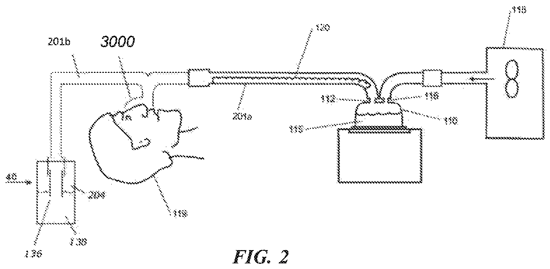

Referring now to FIG. 2, another example of a respiratory system including a bubbler device and humidifier is depicted. A humidified Positive End Expiratory Pressure (PEEP) system is shown in which a patient 119 is receiving humidified and pressurized gases through the patient interface 3000 connected to an inspiratory or inhalatory conduit 201a. It should be understood that the present disclosure, however, is not limited to the delivery of PEEP gases but is also applicable to other types of gases delivery systems and may not necessarily involve humidification. Inspiratory conduit 201a is connected to the outlet 112 of a humidification chamber 110, which contains a volume of water 115. Inspiratory conduit 201a may contain heating means or heater wires 120 that heat the walls of the conduit to ensure a constant humidity profile along the conduit and therefore reduce condensation of humidified gases within the conduit. As the volume of water 115 within humidification chamber 110 is heated, water vapour begins to fill the volume of the chamber above the water's surface and is passed out of the humidification chamber 110 outlet 112 with the flow of gases (for example air) provided from a gases supply means or blower 118 which enters the chamber 110 through inlet 116. The humidified gases pass through the inspiratory conduit 201a to an interface 3000 being worn by the patient 119. The excess gases then flow through an expiratory or exhalatory conduit 201b to a pressure regulator 40.

In some embodiments, the pressure regulator 40 takes the form of discharging the flow of exhalatory gases into a chamber 204 containing a column of water138. The gases flowing through the expiratory conduit 201b are discharged into the body of water 138 from a short conduit 136 which extends from the expiratory conduit 201b into the chamber 204. This results in a bubbling effect, whereby the gases eventually exit the chamber 204 via the outlet port, which can also be used to initially fill the chamber 204 with water. The outlet port includes shielding to prevents liquid aerosols created by the vigorous bubbling on the surface of the water from being expelled. It will be appreciated that the short conduit 136, could equally be integrated into the end of the expiratory conduit 201b.

### Patient Interface Assembly

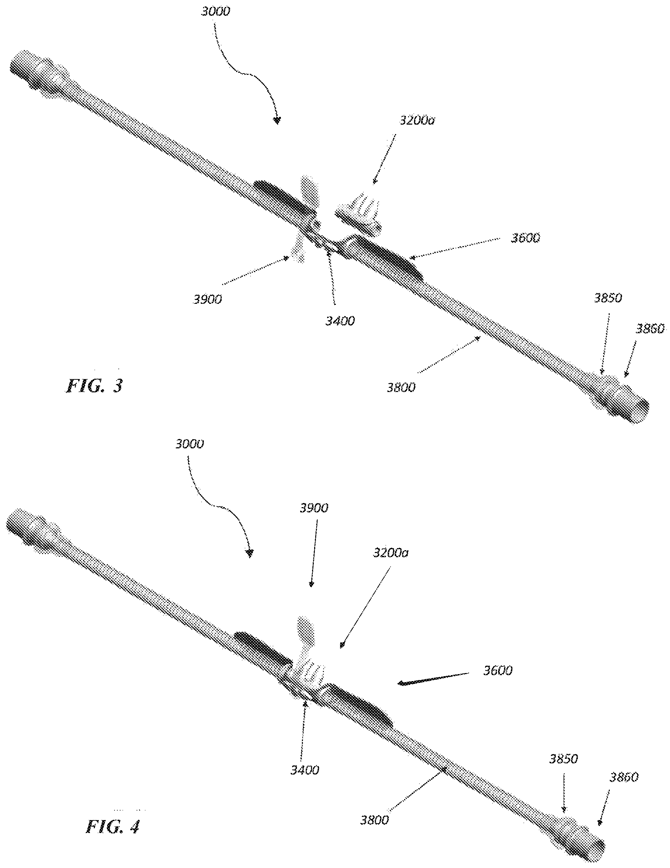

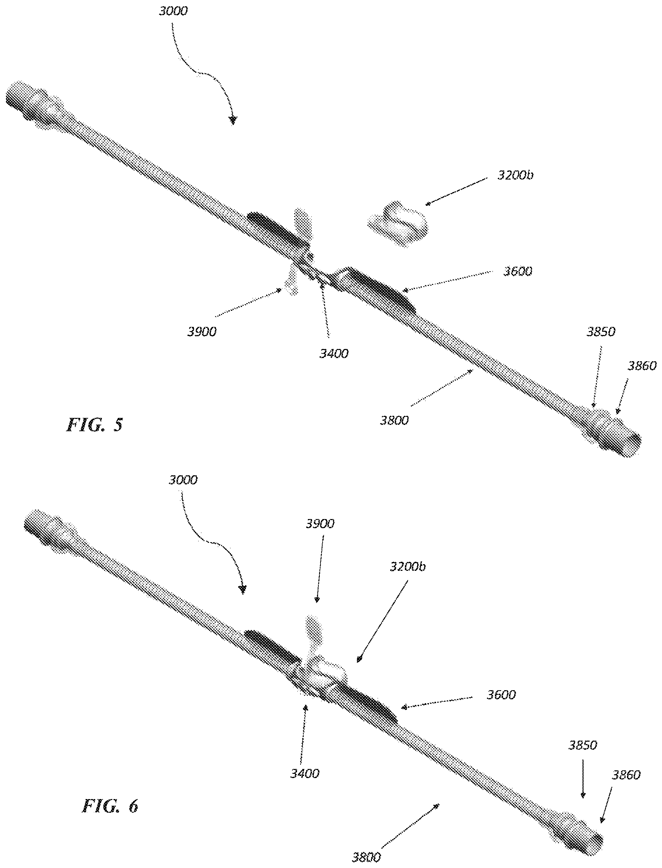

FIGS. 3-6 illustrate embodiments of the patient interface 3000. As shown in FIGS. 3-6, the patient interface 3000 includes an interface body which can form a seal with a user's face and allow gas flow from and to the user, and the interface body can be coupled to a housing 3400. In some embodiments, the housing 3400 can be a frame that can receive a portion of the interface body. In some embodiments, the interface body is a cannula body 3200a that can mate with the housing 3400 to form a nasal cannula 3200, such as illustrated in FIGS. 3-4. The cannula body 3200a can form a seal with nares of the user's nose. In some embodiments, the interface body is a mask body, such as the mask body 3200b illustrated in FIGS. 5-6. The mask body 3200b has a mask or mask cushion that can form a seal with the face of the user. The illustrated embodiment of the patient interface 3000 also includes a first securement member 3600, one or more interface tubes 3800, and a second securement member 3900.

In some embodiments, the housing 3400 can be detachably coupled to the cannula body 3200a, the mask body 3200b, or any other type of interface bodies. The housing 3400 can be detachably coupled with an interface body, for example using mechanisms to be described in further detail herein, such that a user or a clinician can swap between different types of interface bodies (e.g. between the cannula body 3200a and the mask body 3200b) and/or different sizes of interface body. This may assist with better fitting while reusing the same housing without disassembling or removing the entire patient interface 3000 during treatment. For example, the interface body may be coupled to and/or decoupled from the housing 3400 while the first securement member 3600, the interface tubes 3800 and/or the forehead support member 3900 are kept coupled to the housing 3400. In some occasions, a mask body and a cannula body may need to be used alternately during treatment, for example to reduce or prevent skin irritation or damage. The interface body could be swapped while maintaining the housing 3400 connected to a headgear, a bonnet, a chinstrap, or the face of the user. Further, detachable coupling of the interface body and the housing 3400 may be advantageous for cleaning and maintenance. In some embodiments, a kit for treating a patient includes one or more cannula bodies and/or one or more mask bodies compatible with the housing 3400, such that a user or a clinician can choose and use an appropriate interface body. In some embodiments, the kit includes cannula bodies with different sizes and/or mask bodies with different sizes. The different sizes of mask bodies and/or cannula bodies may be indicated by different colours or other means, such as letters and/or symbols. The letters and/or symbols may be moulded, laser marked, printed or otherwise displayed on the mask bodies and/or cannula bodies.

In some embodiments, the housing 3400 may be permanently coupled to the cannula body 3200a, the mask body 3200b, or any other type of interface bodies. A user or a clinician can swap between different types of interface bodies (e.g. between the cannula body 3200a and the mask body 3200b) or the interface body of different sizes for better fitting, by interchanging the assembly with the housing and the interface body. For example, the interface body and the housing 3400 may be coupled to and/or decoupled with the interface tubes 3800. In some embodiments, a kit for treating a patient includes one or more cannula bodies and/or one or more mask bodies attached with the housing 3400, such that a user or a clinician can choose and use an appropriate interface body attached to the housing 3400.

The first securement member 3600 may secure or support the patient interface 3000 relative to the user. The first securement member 3600 may be coupled to the housing 3400 and facilitate securement of the interface body to the user's face and thereby help the interface body to form and maintain a seal with the user's face. In some embodiments, the first securement member 3600 includes means to attach and secure the patient interface 3000 on a headgear, a chinstrap, a patient interface patch or a dermal patch on the patient's face, or otherwise on the patient's face by any suitable mechanisms. For example, the first securement member 3600 may include hook and loop material or adhesive on the user facing side as described in further detail herein. Even though the first securement member 3600 is coupled with the housing 3400 in the illustrated embodiments, the patient interface 3000 may be used without the first securement member 3600. For example, the housing 3400 may be secured on the user's face, for example on cheeks, directly or with other securement means, such as a headgear or chinstrap. As illustrated in FIGS. 3-6, the first securement member 3600 may extend horizontally or substantially horizontally. However, the first securement member 3600 may be positioned in any suitable orientation.

The patient interface 3000 may further include other securement means in addition to or instead of the first securement member 3600. For example, in the illustrated embodiment, the patient interface 3000 includes the second securement member 3900. In some embodiments, the patient interface 3000 includes both the first securement member 3600 and the second securement member 3900. In some embodiments, the patient interface includes one of the first securement member 3600 and the second securement member 3900. As illustrated in FIGS. 3-6, the second securement member 3900 may extend vertically or substantially vertically. However, the second securement member 3900 may be positioned in any suitable orientation.

The patient interface 3000 may further include one or more interface tubes 3800. In some embodiments, the walls of the interface tubes can be made of a material that can allow for the passage of moisture vapor through the walls of the interface tubes. In some embodiments, the one or more interface tubes 3800 are at least partially insulated. The interface tubes 3800 may be coupled to the housing 3400 at one end, and may connect to a CPAP therapy delivering device, for example through the tubes 201a and 201b, at the other end, and allow gas from and to the user to flow through the housing 3400 and the interface body. Each of the interface tubes 3800 may include a connector 3860 for the connection to the respiratory component, such as tubes 201a and 201b. The connector 3860 may be overmoulded to the tube 3800. The connector 3860 may be or comprise a silicone or elastomer material. The connector 3860 may have similar or greater stiffness than the interface tubes 3800. The connector 3860 may be or comprise the same material as a component of the tube 3800, such as a bead of the interface tube 3800, provided in a stiffer geometry relative to the tube 3800. In some embodiments, the connector 3860 includes one or more ports to monitor gas properties such as pressure. In some embodiments, the patient interface 3000 may include one or more sensors to monitor gas properties such as pressure at the one or more ports or any other suitable locations. The connector 3860 may be suitable for receiving a clip, which may be used to clip to bed sheets or clothing to provide additional securement of the patient interface 3000 and the tubes 3800. In some embodiments, each of the interface tubes 3800 further include a grip 3850. The grip 3850 may be directly or indirectly overmoulded to the tube 3800, for example over both the tube 3800 and the connector 3860. In some embodiments, the tube 3800 may be attached with adhesives or threadably attached to the connector 3860 or other regions of the grip 3850. The clinician or the user may hold the grip 3850 to connect or plug-in the connector 3860 to the respiratory component, such as tubes 201a and 201b. In some embodiments, one or more components of the interface tubes 3800 can rotate relative to the other components of the interface tubes 3800. For example, the grip 3850 can rotate relative to the connector 3860, such that the torsion or movement caused by holding of the grip 3850 may not be translated through the rest of the tubes 3800 to the housing 3400 and the interface body.

In some embodiments, the tubes 3800 can be detachably coupled to the housing 3400. In some embodiments, the one or more of interface tubes 3800 are permanently attached to the housing 3400. For example, the one or more of interface tubes 3800 may be directly or indirectly overmoulded to the housing 3400, thereby forming an integral piece. In some embodiments, the tubes 3800 may be attached with an adhesive or threadably attached with the housing 3400. In some embodiments, the tube 3800 can rotate or swivel relative to the housing 3400, such that the torsion or movement of the tubes 3800 may not conduct to the housing 3400 and the interface body. In some embodiments, the interface tubes 3800 have a varying diameter and/or thickness along the length of the interface tubes 3800. For example, each of the interface tubes 3800 may be tapered toward one end. In some embodiments, the interface tubes 3800 have a constant diameter and/or thickness along their lengths. In some embodiments, the interface tubes 3800 may have a circular, semicircular, or D-shaped cross-sectional shape. When the semicircular or D-shaped cross-sectional shape is used, a flat side of the interface tubes 3800 can be patient facing.

### Housing

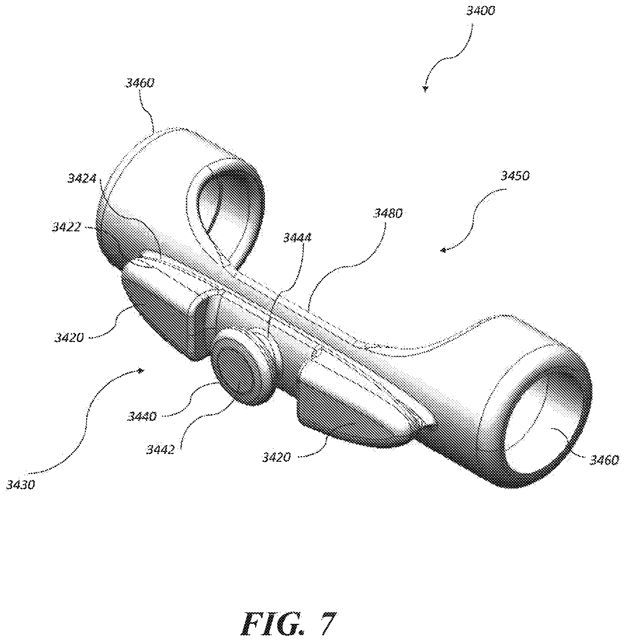

FIGS. 7-9 illustrate an embodiment of the housing 3400. In the illustrated embodiment, the housing 3400 has a front portion 3430 and a rear portion 3450. The rear portion 3450 may be oriented to face the user's face when the patient interface 3000 is applied on the user's face, and the front portion 3430 may be oriented to be opposite the user's face when the patient interface 3000 is applied on the user's face.

In the illustrated embodiment, the front portion 3430 includes one or more attachment elements. The one or more attachment elements may extend outwardly from the front portion 3430. In the embodiment illustrated in FIGS. 7-9, the one or more attachment elements can include a central protrusion 3440 and a front extension 3420. In some embodiments, such as shown in FIGS. 7-9, the one or more attachment elements includes a central protrusion 3440 with a pair of front extensions 3420. Each of the pair of front extensions 3420 can be located laterally on opposing sides of the central protrusion 3440. In other embodiments, the one or more attachment elements can include any arrangement of protrusions or extensions. For example, in some cases, a single attachment element can be used.

In some embodiments, such as shown in FIG. 7, the front extension 3420 can be raised toward a front direction. The front extension 3420 may form a ridge 3422. A channel 3424 may be at least partially defined by the ridge 3422. In some embodiments, the first securement member 3600 can be positioned around the front extension 3420 and secured at the channel 3424, such that the first securement member 3600 can be detachably coupled to the housing 3400 when the patient interface 3000 is assembled.

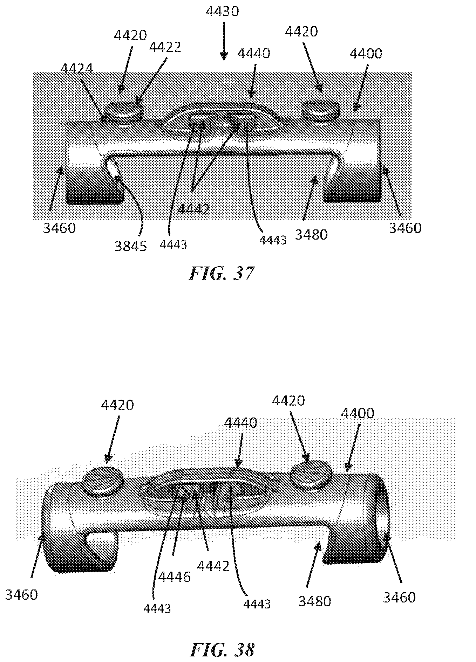

The central protrusion and front extension can be used to secure one or more securement members to the housing 3400. The central protrusion and front extension can be shaped to receive the complementary portion of a securement member. As shown in FIGS. 7-9, in some embodiments, the central protrusion 3440 may include an enlarged head 3442 and a stem 3444. In other embodiments, such as shown in FIGS. 37 and 38, the central protrusion 4440 can have an opening to receive the securement member as described herein with reference to FIGS. 37-38.

As illustrated in FIGS. 7-9, the central protrusion 3440 can receive and retain one or more components with a hole which fits to the enlarged head 3442, for example by push-fit. For example, the second securement member 3900 can be detachably coupled to the housing 3400 at the central protrusion 3440. In some embodiments, the central protrusion 3440 is positioned in the middle or substantially in the middle of the front portion 3430 and between the front extensions 3420.

In some embodiments, the front extensions 3420 can be positioned on either side of the central protrusion 3440 as shown in FIGS. 7-9. The front extensions can have a shape to receive the complementary securement member. As illustrated in FIGS. 7-9, the front extension 3420 may be recessed around the central protrusion 3440, such that the central protrusion 3440 can have a greater height to receive a thicker component, such as the second securement member 3900 without excessively protruding out of the housing 3400. In some embodiments, the front extension 3420 may not be recessed around the central protrusion 3440. In other embodiments, as shown in FIGS. 37 and 38, the front protrusion 4420 can be circular to receive the securement member as described herein with reference to FIGS. 37-38.

In the illustrated embodiment, the rear portion 3450 of the housing 3400 includes an interface body receiving portion 3480 for receiving an interface body, such as the cannula body 3200a or the mask body 3200b. At the interface body receiving portion 3480, an inner surface of the housing 3400 is exposed. The interface body receiving portion 3480 and the inner surface of the housing 3400 may be shaped and/or sized to match the shape and/or size of the interface body, and to allow removal and interchangeability of the interface body relative to the housing 3400. For example, the inner surface of the housing 3400 may have a cylindrical shape, such that it can receive and fit with a cylindrical shaped portion of the interface body.

The housing 3400 may further include one or more tube engaging portions 3460 to receive one or more interface tubes, such as the one or more interface tubes 3800. The tube engaging portions 3460 may be sized and/or shaped to match the shape and/or size of the interface tubes, such that the interface tubes can be coupled to the tube engaging portions 3460 without a leak. The tube engaging portion 3460 may be positioned such that the housing 3400 can receive a gas from the interface tubes through the tube engaging portion 3460, and allow the gas to flow to the interface body coupled to the interface body receiving portion 3480.

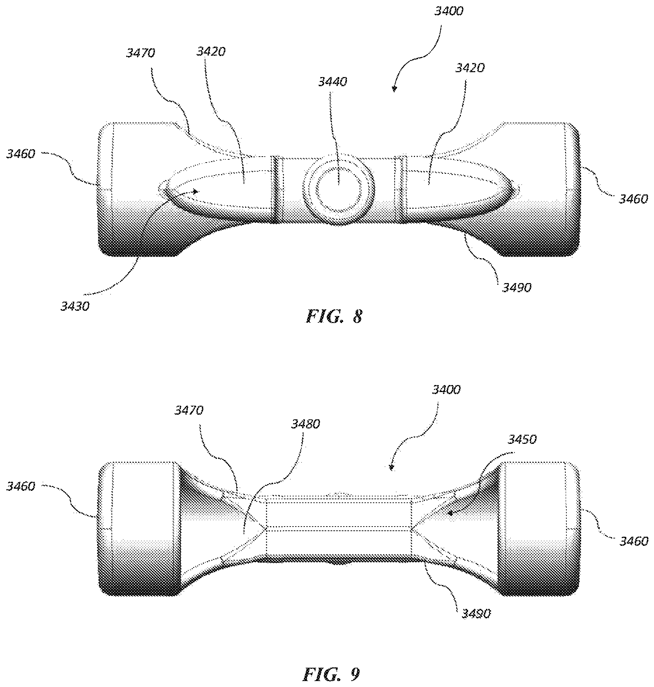

The housing 3400 further includes an upper edge 3470 and a lower edge 3490 opposite the upper edge 3470. In some embodiments, the upper edge 3470 and the lower edge 3490 are symmetrical, such that a user or a clinician can orient the housing 3400 in either direction when assembling the interface 3000. As shown in FIGS. 8-9, the upper edge 3470 and/or the lower edge 3490 may have an inwardly curved profile. The inwardly curved profiles of the upper edge 3470 and/or the lower edge 3490 can allow the user's mouth to be exposed even when the user is wearing the patient interface 3000, for the comfort of the user. If the user is an infant, a pacifier may be used or the user can be breastfed during the treatment. Further, an orogastric tube may be introduced to the patient, and the mouth of the user would be able to be cleaned easily.

In some embodiments, the housing 3400 is constructed from a material having greater stiffness than the interface body, such as the cannula body 3200a and the mask body 3200b, such that the housing 3400 can provide structural stability to the patient interface 3000. For example, the housing 3400 may be constructed from a material having Young's modulus of 0.1 GPa - 7 GPa, 0.5 GPa - 7 GPa, 0.5 GPa - 6 GPa, 0.8 GPa - 6 GPa, 1 GPa - 6 GPa, 1.8 GPa - 6 GPa, 2 GPa - 5 GPa, or 3 GPa - 5GPa. The thickness of the housing 3400 may be then altered to achieve the desired stiffness of each regions of the housing 3400. The housing 3400 may be constructed from thermoplastic elastomers, such as polycarbonate, thermoplastic polyurethane (TPU), polyamide, nylons. The front extension 3420 forms a raised portion with greater thickness as described herein, such that the front extension 3420 contributes to the stiffness of the housing 3400. The size and/or thickness of the front extension 3420 may be adjusted to adjust the rigidity of the housing 3400.

### Interface Body

As described herein, the patient interface 3000 includes an interface body which can form a seal with a user's face and allow gas flow from and to the user. The interface body can be coupled to a housing 3400. In some embodiments, the interface body is a cannula body 3200a that can mate with the housing 3400 and form a nasal cannula that can form a seal with nares of the user's nose. In other embodiments, the interface body is a mask body, such as the mask body 3200b.

### Cannula Body

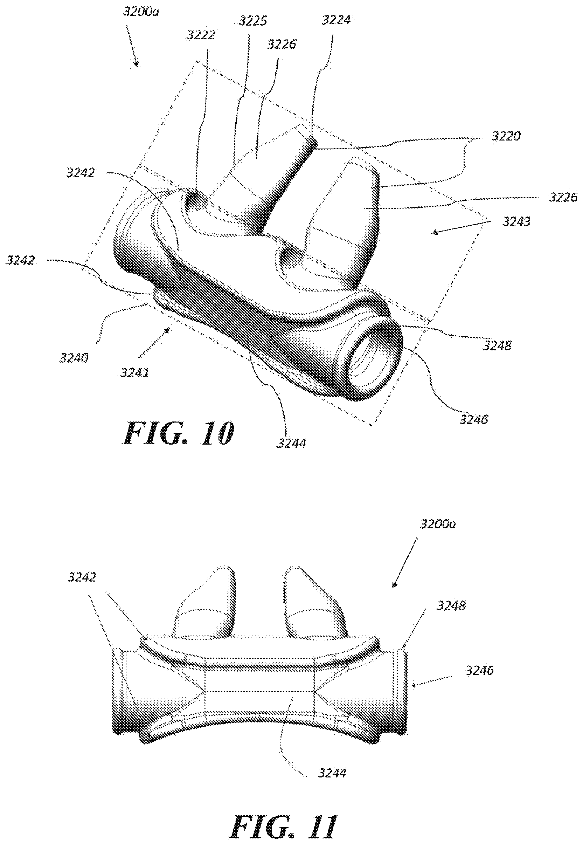

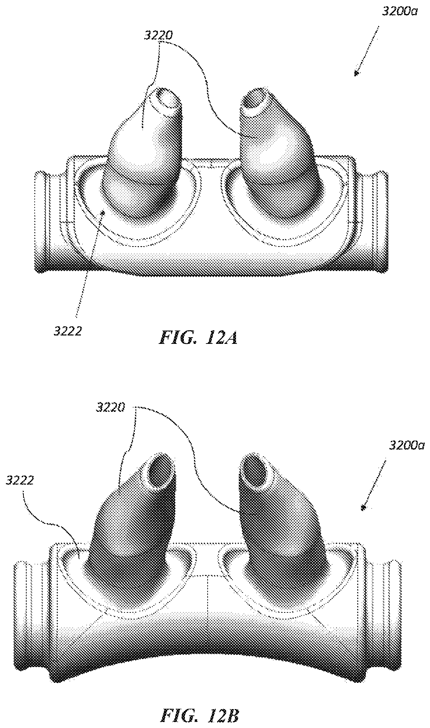

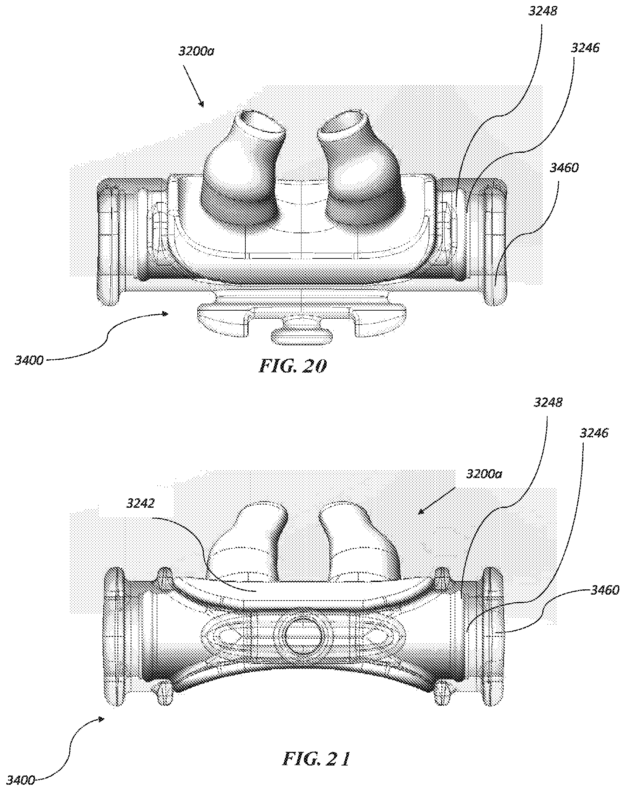

FIGS. 10-12B illustrate an embodiment of the cannula body 3200a. The cannula body described herein in other embodiments can have similar features as described with reference to FIGS. 10-12B. For example, the embodiments of the cannula body described with reference to FIGS. 21, 48A, 49, 52A-B, 53A, and 55 can include features described herein with reference to the cannula body 3200a and nasal prongs described herein. As shown in FIGS. 10-12B, the cannula body 3200a may have at least one nasal prong 3220, such as a pair of prongs 3220. The prongs 3220 may extend from a rear portion 3243 of a manifold 3240 facing the patient. The nasal prongs 3220 can form a seal with nares of a user's nose and allow gas flow from and to the user. The nasal prongs 3220 of the patient interface shown and described herein are shaped for sealing in a patient's nares. In some embodiments, the nasal prongs are shaped for sealing in an infant's nares. However, it should be appreciated that the prongs may be suitable for any patient population with similar nares geometry, and that the prongs may be provided in different absolute sizes for various patient populations. The prongs 3220 can be shaped and formed to minimize tissue compression and kinking of one or more of the prongs 3220 during insertion into a patient's nares.

**In** the illustrated embodiment, the manifold 3240 defines one or more openings 3246, such as a pair of openings 3246 which can be coupled to the tube engaging portion 3460 of the housing 3400 to receive the interface tubes, and allow gas flow from and to the patient via the manifold 3240 and the nasal prongs 3220. The openings 3246 may be in fluid communication with each other via the manifold 3240. Except for the openings 3246 and the lumens of the nasal prongs 3220, the manifold 3240 may be sealed. The openings 3246 can be positioned on the side of the manifold 3240. As shown in FIGS. 10-12B, the manifold 3240 can have two openings 3246 and the openings 3246 can be on opposing sides of the manifold 3240. The placement of the openings 3246 on the side of the manifold allows for lateral flow of fluid into and out of the manifold 3240 through the openings 3246 when the manifold 3240 is coupled to the tube engaging portion 3460 of the housing 3400.

### Prongs of the Cannula Body

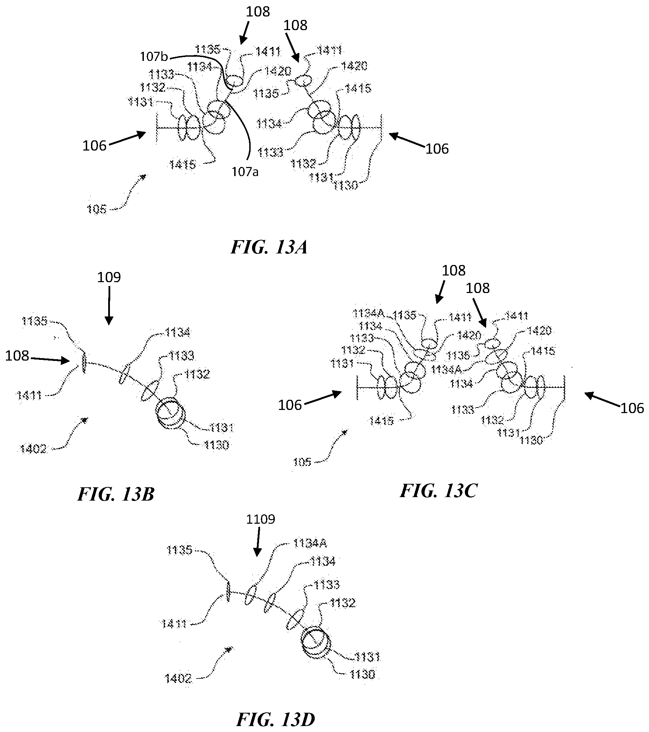

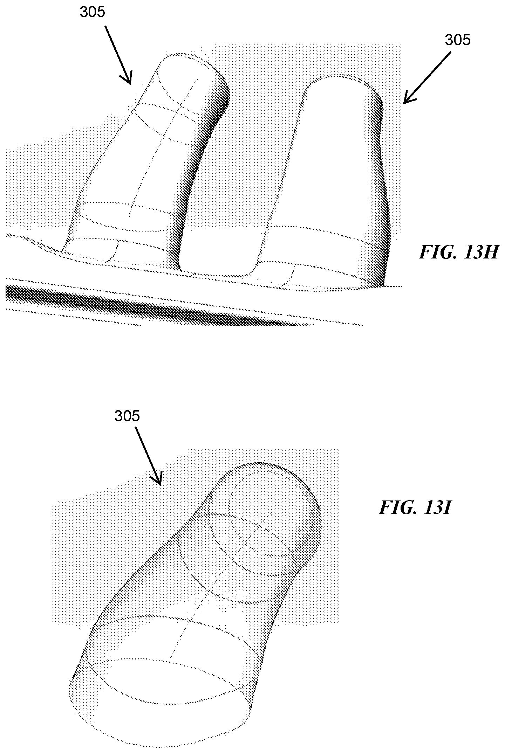

The cannula body 3200a can include nasal prongs with an orientation and/or geometry that may assist with gas flow and delivery of gas to the patient. For example, FIGS. 13A-13D illustrate a geometry of an embodiment of the prongs 105 that can be similar to or the same as the nasal prongs on the cannula body as described herein, such as the nasal prongs 3220 and 4220.

As shown in FIGS. 13A-13B, the prongs 105 may taper inwardly from or between the gases inlet 106 to the gases outlet 108. The cross-sectional area of the gases inlet 106 may be larger than the cross-sectional area of the gases outlet 108. The prong cross-sectional area may gradually diminish from or between the inlet 106 to the outlet 108. This prong tapering may aid in the sealing function of the prongs 105. When the prongs 105 are pushed into the patient's nares, they may seal somewhere along the length of the prong 105 due to tapering in which the prongs 105 widen towards the inlet 106. The tapering may aid in insertion of the prongs 105, as opposed to prongs which are of a constant cross-sectional area, or prongs which widen towards the outlet.

The lumen of the nasal prong 105 may have an internal cross-section that varies along the length of the nasal prong 105. For example, as shown in and described with reference to FIGS. 13A-13D, the internal cross-section of the prong may vary in shape and/or diameter along its length.

As described in more detail herein, the nasal prong 105 may be shaped to substantially align the flow of breathing gas through the gas outlet with a user's upper airways.

The nasal prong 105 may be shaped to extend generally upwardly and rearwardly into a user's nares, the nasal prong 105 having a curvature that includes at least two inflection points, for example, as shown in FIGS. 13A-13D and described herein.

The prongs 105 may extend from the cannula body or manifold, toward the user's septum and curve around the corners of a user's nostrils upwardly and rearwardly into the user's nares. Each prong 105 may extend along a generally inclined posterior trajectory, passing through two mediolateral points of inflection. This geometry may assist to orientate the gas outlet 108 with respect to the user's upper airway passages.

The at least one prong 105 may have a shaped trajectory fitting or comparable with the anatomical shape of the user's nostril. In a first portion (or phase) of the prong 105, the trajectory may move horizontally towards the midline of the face. In a second portion (or phase) of the prong 105, the trajectory may curve upwards into the nostril towards the crown of the head. In a third portion (or phase) of the prong 105, the trajectory may roll backwards into the head, generally following the anatomical curvature of the nostril. In a fourth portion (or phase) of the prong 105, the trajectory may tilt horizontally towards the center of the interface assembly to align the outlet 108 with the user's upper airway.

The prong 105 may have a cross-section that varies along the central trajectory, such as described above. For example, the cross-section may be generally circular at the base of the trajectory and become generally circular or oval or elliptical towards the end of the trajectory or prong 105. The cross-section may be generally circular or oval or elliptical at the base of the trajectory, becoming generally circular towards the end of the trajectory or prong 105.

The cross-sectional diameter may generally decrease along the trajectory from the first portion (or phase) to the end of the fourth portion (or phase).

The geometry of the prongs, such as shown in FIGS. 10-12B as well as FIGS. 48A, 49, 52A-52B, and 53 described herein may be substantially as illustrated in FIGS. 13A-13D, wherein the curved lines represent the prong trajectory, and ellipses 1135 primarily represent the orientation of the lumen within each prong at a particular trajectory (and also show one potential cross-sectional lumen shape). In some embodiments, the ellipses 1135 can be circular or substantially circular. Each prong may generally follow a curved path that is shaped to follow the anatomical geometry/ curvature/ contours of a user's nare. The prongs 105 may be moulded or formed to substantially follow the anatomical shape and curvature of a user's nare.

The prongs 105 may be premoulded or preformed according to the anatomical shape of a nare. In some embodiments, the prongs 105 may include at least one thinned wall section which is conformable so as to limit pressure on the patient's septum. In other embodiments, the prong wall thickness can be uniform throughout.

From a base 1415, each prong 105 may curve generally upwardly or superiorly toward the crown of the user's head (away from the transverse plane) and generally rearwardly or posteriorly (toward the user's coronal plane) with respect to the user's upper lip. Between the ellipses 1131 and 1133 (the second phase) the lumen of the prongs 105 may transition from a generally mediolateral orientation along the user's upper lip to a predominantly inclined posterior orientation. This configuration may direct gas flow toward an upper portion of the back of a user's head. The lumen of the prong 105 may reduce slightly during this phase. The lumen may also become more elliptical, which may utilize the space available within the nare.

In the third phase (between the ellipses 1133 and 1134) the prongs 105 may continue along an inclined posterior trajectory toward the upper back of a user's head (away from the transverse plane and toward the coronal plane). There may be a smooth reduction in the rate of incline in the superior component of the prongs trajectory 1420, which may cause the lumen to move away from the transverse plane. During this phase, the prongs 105 may have negligible or minimal convergence (or mediolateral component) toward the sagittal plane. The prong lumen may reduce further during this phase and may become increasingly elliptical.

In the final phase (between ellipses 1134 and 1135) the prongs 105 may continue along an inclined posterior trajectory with some mediolateral convergence toward the sagittal plane. The mediolateral convergence of the prongs 105 may begin at the illustrated trajectory inflection point 107a at the start of the fourth phase (or slightly prior) adjacent the ellipse 1134.

A second inflection point 107b may be adjacent the final ellipse 1135. The second inflection point may reduce convergence of the prongs 105 and orientate the prong outlet 1411 generally posteriorly (toward the coronal plane) with a slight mediolateral component toward the sagittal plane (represented by the orientation of the final ellipse 1135 in FIG. 13A).

The incline rate of the prong trajectories 1420 may continue to decrease during the fourth phase, until the respective trajectories 1420 are substantially parallel with the transverse plane at the prong outlet 1411 (represented by ellipse 1135). The mediolateral and superior-inferior adjustments of the prong trajectories 1420 adjacent the final ellipse 1135 may position the prong outlet 1411 generally in alignment with the passage of the upper airway. This may assist to reduce soft tissue irritation caused by exiting breathing gases.

The prong lumen may be elliptical at the outlet 1411. The major elliptical axis may be arranged in a generally transverse plane. The prong lumen may be circular or substantially circular at the outlet 1411. The outlet 1411 may direct breathing gases upwardly or superiorly toward the crown of the user's head (away from the transverse plane) and rearwardly or posteriorly (toward the user's coronal plane).

In alternative embodiments the cross-section of the lumens may be triangular, or quadrilateral.

As shown in FIGS. 13C-13D, the nasal prongs may have a sealing region 1109 that is longer compared to a sealing region 109 of the earlier described embodiment described with reference to FIGS. 13A-13B. The longer sealing region 1109 may cause the overall length of the nasal prong of this embodiment to be longer than the overall prong length of previously described embodiments. This may have the additional benefit of the prong being less likely to flick out or otherwise disengage from the patient's nare. With reference to the curved lines that represent the prong trajectory, and the ellipses of FIGS. 13C-13D, a prong sealing region 1109 is between ellipses 1134a and 1135. The prong sealing region 1109 may comprise a tapered region. A cross-section of the exterior surface of the prong 105 near the gas inlet 106 may be larger than a cross-section of the exterior surface of the prong 105 near the gas outlet 108.

In the illustrative embodiment shown, the prong sealing region 1109 tapers from about 5 mm to about 4 mm, for example. The measurements of the prong (5mm and 4mm) refer to an equivalent diameter of an oval. The equivalent diameter may be a diameter calculated from the perimeter of the prong (circular and ovular prongs may have the same equivalent diameter) or it may be the largest width dimension of the prong (circular and ovular prongs may have a different equivalent diameter). The tapered sealing region 1109 may allow the patient interface 1101 to be used for a patient having a nare size that is anywhere between about 4mm and about 5mm. For example, if a patient's nare size is 4.5mm, the prongs 1105 may be inserted in the nares such that the section of the sealing region 1109 having a diameter of 4. 5mm seals with their nares.

The prong sealing region 1109 may have different dimensions. and/or different amounts of taper. The length range for the sealing region may be between about 1mm to about 10 mm. The length of the sealing region may be about 1 mm, 2 mm, 3 mm, 4 mm, 5 mm, 6 mm, 7 mm, 8 mm, 9 mm, or 10 mm. The diameter of the sealing region may be between about 2 mm and about 10 mm. The diameter of the sealing range may be about 2 mm, 3 mm, 4 mm, 5 mm, 6 mm, 7 mm, 8 mm, 9 mm, or 10 mm.

The ratio of the taper from the wider portion to the narrower portion may be about 1.1:1, 1.2:1, 1.3:1, 1.4:1, 1.5:1, 1.6:1, 1.7:1, 1.8:1, 1.9:1, or 2:1.

For example, the prong sealing region may taper from 10mm to 9mm, 9 mm to 8mm, 8mm to 7mm, 7mm to 6mm, 6mm to 5mm, 5mm to 4mm, or 4mm to 3mm, 3mm to 2 mm, for example. The taper may be steeper, for example, the prong sealing region may taper from 10mm to 8mm, 9mm to 7mm, 8mm to 6mm, 7mm to 5mm, 6mm to 4mm, 5mm to 3mm, or 4mm to 2mm, for example. The taper may be less steep, for example, the prong sealing region may taper from 10mm to 9.5mm, 9.5mm to 9mm, 9mm to 8.5mm, 8.5mm to 8mm, 8mm to 7.5mm, 7.5mm to 7mm, 7mm to 6.5mm, 6.5mm to 6mm, 6mm to 5.5mm, 5.5mm to 5mm, 5mm to 4.5mm, 4.5mm to 4mm, or 4mm to 3.5mm, 3.5mm to 3mm, 3mm to 2.5mm, or 2.5mm to 2mm for example.

Different sizes of the patient interface 101, 1101 may be provided, for which different prong sealing region dimensions may be utilized.

The outlet of the prong lumen can be substantially circular. In some embodiments, the outlet of the prong lumen can be substantially elliptical. It should be appreciated by one of skill in the art that any suitable shape of outlet may be used.

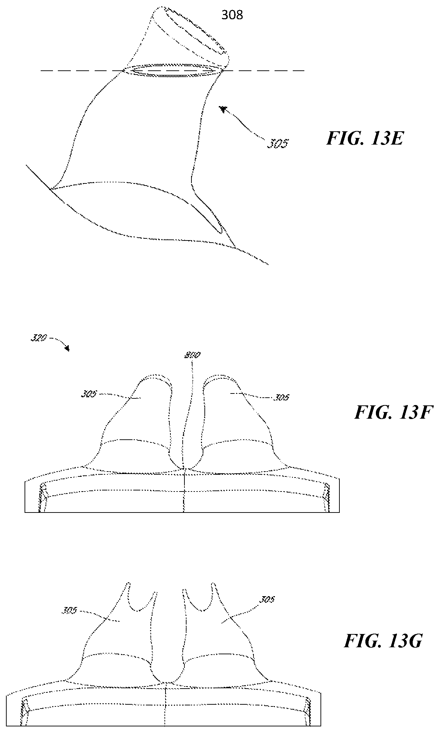

The nasal prong lumen may be flared out at or near the outlet of the lumen. An example of a nasal prong lumen with flared out portions at the outlet or alternative outlet shapes are shown in FIGS. 13E and 13H. As shown in FIG. 13E, ends of the prongs 305 adjacent the prong outlet 308 may be shaped to reduce the resistance to flow of the prongs 305. For example, the prongs 305 may be cut off horizontally or at an angle. This may lower flow resistance in the prong 305. Additionally, or alternatively, when viewing the prongs 305 from above the interface 300 in a same or similar perspective as FIG. 13F, the prong outlet 308 may have a substantially U-shape, as shown in FIG. 13G. These features may assist to provide a lower resistance to flow in use, and/or prevent accidental sealing of the prong outlet 308, which may cause a user or a clinician to falsely determine that the CPAP therapy is delivered to the user. In some embodiments, the nasal prongs may have a thickness that varies in a circumferential direction.

### Base of Nasal Prong

The cannula body can have a base region at and/or adjacent the base of the nasal prong. The base region can form a portion of the cannula body where the base of the nasal prong meets the manifold. The base region can be configured to allow movement of the manifold and/or housing while maintaining the nasal prongs within the patient's nares. The base region may assist to provide comfort and conformability to the patient while also ensuring the flow path is maintained between the manifold and nasal prongs.

In some embodiments, the base region can allow the nasal prongs to bend in different directions without compromising the gas flow path from manifold to prong outlet. For example, the base region can allow the prongs to move relative to the housing/manifold without the prongs kinking. The base region may function to decouple the position of the or each prong from the position of the manifold/housing. In this manner, the housing/manifold can move, e.g. from forces applied to the interface by pulling on tubes, facial movements and/or grasping of the interface, whilst retaining the prongs in position in the nares. The base region may also provide a spring mechanism which may operate to assist in compensating external forces applied to or experienced by the interface. The spring mechanism may also allow for movement of the housing/manifold whilst assisting the prongs to remain in an operational, i.e. sealed position in the nares. In some cases, the base region can include a groove, dip, channel or any other mechanism between the prongs to provide for adequate clearance of the septum.

This portion of the cannula body can be an alternation in the material at or adjacent the base of the prong and/or a specific shape of the base of the prong. For example, the base region can be a groove, sunken or recessed region, a thinning of the nasal prong wall, base or surrounding region of the manifold, a shape of the nasal prong and/or manifold, and/or any other feature that may provide elasticity or conformability. Embodiments of the base region described herein can be used with any of the cannula body embodiments and/or nasal prong embodiments described herein.

In some embodiments, the manifold 3240 may have a base region that can be a sunken or recessed region under the prongs 3220 and/or adjacent the base of the prongs 3220. For example, in the illustrated embodiment of FIGS. 10-12B, the manifold 3240 includes one or more grooves 3222 located under the prongs 3220. The one or more grooves 3222 may support the prongs 3220 relative to a surrounding portion of the manifold 3240. The grooves 3222 as shown in FIGS. 10-12B, can extend substantially around the base of each prong 3220 or at least partially around the base of each prong 3220. The grooves 3222 may assist the prongs 3220 to pivot and move about or relative to the surrounding portion of the manifold 3240.The ability of the prongs 3220 to pivot at or adjacent their base may enable more effective fitting and sealing of the prongs 3220 in the nares of a patient without compromising gas flow. The ability for the prongs 3220 to bend or move relative to the manifold 3240 may decouple the position or orientation of the prongs 3220 from the position of the manifold/housing. In other words, the housing/manifold can move while the prongs 3220 remain in a sealing position in the nares.

The grooves 3222 may allow one size of prongs to fit a wider range of nare sizes. The groove 3222 around each prong 3220 may allow the prong 3220 to move or flex forward and backward when inserting and fitting the prongs 3220 into the patient's nares. This movement can adjust for variation in face and philtrum size of patients. The groove 3222 may also allow for sideways movement to adjust for variation in septum size and/or to assist accommodating movement of the interface 3000, for example caused by cheek movement of the user. The sunken or recessed region such as the grooves 3222 may further make the effective length of the prongs 3222 greater, such that the prongs 3222 can have greater degree of adjustability.

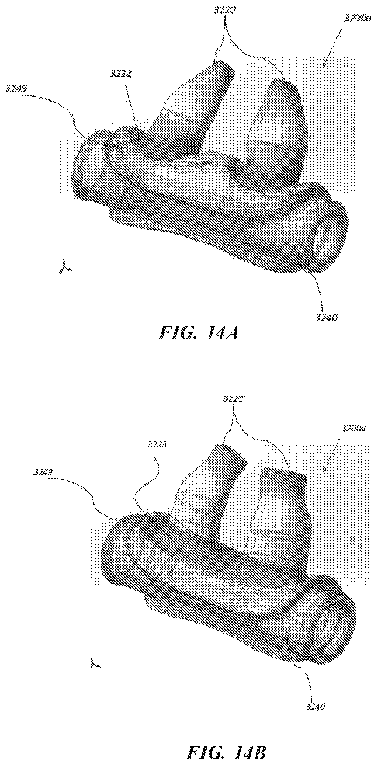

The manifold 3240 may have varying thicknesses at different regions. For example, at least a portion of the grooves 3222 may be thinner relative to the prongs 3220 and the rest of the manifold 3240, as illustrated in FIG. 14A (with the thinner region of material of the prongs shown with darker shading in FIG. 14A and 14B). The grooves 3222 may facilitate movement of the prongs 3220 to assist to maintain seal with the patient's nares. The grooves 3222 may also assist to ensure that the prongs 3220 collapse into the manifold 3240 by the grooves 3222 being folded, bending or flexing, rather than one or more of the prongs kinking, thereby maintaining the diameter and cross-sectional shape of the prongs 3220, and/or maintaining the resistance to flow (RTF) of the prongs 3220.

As illustrated in FIG. 14A, the manifold 3240 may further include a thickened region 3249 adjacent the grooves 3222 shown with the lighter shaded region adjacent the grooves 3222. When the prongs 3220 are placed in the nares of the user, the grooves 3222 having thinner walls, may allow preferential movement or bending of the prongs 3220 into the manifold 3240. In other words, when the prongs 3220 are inserted into the nares, at least part of the thinner walls of the grooves 3222 may flex or deflect inwardly to the manifold 3240. While in the flexed or deflected position, the grooves 3222 may provide an upwards force into the nares of the user to maintain sealing position. On the other hand, the thickened region 3249 may prevent collapse of the manifold 3240 at a region around the grooves 3222, thereby maintaining the structural integrity of the cannula body 3200a. In the illustrated embodiment, the grooves 3222 are separately formed around each prong 3220, being spaced apart from each other.

In some embodiments, the groove or sunken region at the base of the prongs 3220 can be continuous. FIG. 14B illustrates the cannula body 3200a having a sunken region 3223 continuously extending around the bases of the both prongs 3220. Similarly, with the grooves 3222, the sunken region 3223 may have a reduced thickness relative to the prongs 3220 and the rest of the manifold 3240, as illustrated in FIG. 14B. The sunken regions 3223 may provide the prongs 3220 greater flexibility than the grooves 3222, because of the lack of stiffer part between the prongs 3220 which exists between the grooves 3222. Additionally, the sunken regions 3223 may have greater clearance from the septum, such that the cannula body 3200a has smaller chance to interfere and/or damage the septum. The sunken regions 3223 may also make cleaning of the cannula body 3200a easier due to the relative lack of small cavities or spaces. The groove or sunken region may partially or wholly surround the base of the prong. For example, the groove or sunken region can be on one or both sides of each prong to allow adjustment for septum width. The grooves or sunken regions 3222 at the base of the prongs 3220 may allow for a spring-like movement at the base of the prongs to decouple movement of the nasal prong from the movement of the housing and/or manifold and to retain the nasal prongs in the correct sealing position in the nares.

In some embodiments, the base region comprises a thinner region of material at and/or adjacent the base of the nasal prongs. In some embodiments, the groove or sunken region may be absent. The thinner region of material can surround or partially surround the base of each nasal prong. The thinner region of material may be partly or wholly formed in the base of the nasal prongs.

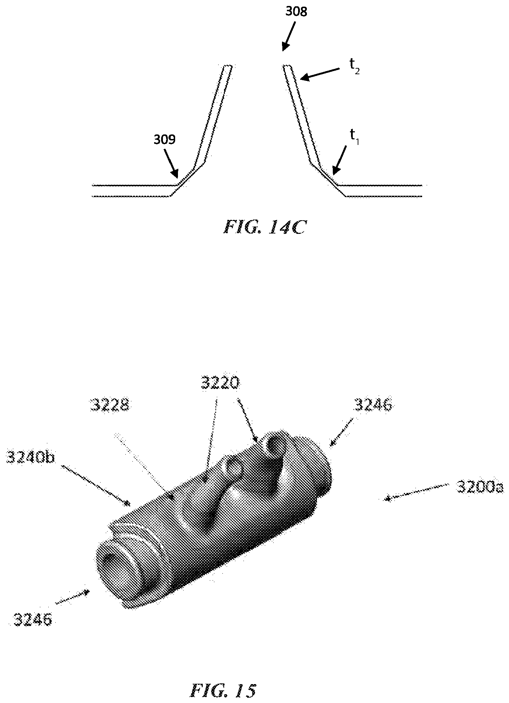

The nasal prong can have a wall thickness that varies from the base of the prong 309 to the outlet 308. As shown in FIG. 14C, the wall at the base of the prong 309 can have a first thickness, t₁, as it extends from the manifold. The wall of the prong can have a second thickness, t₂, as it extends toward the outlet. The first thickness, t₁, can be thinner than the second thickness, t₂. The first thickness, t₁ can be thinner than the material of the manifold that surrounds the base of the prong 309. A thinner wall section t₁ at the base of the prong 309 compared to the remainder of the prong 309 and manifold can allow for prong movement and conformability of the nasal prong to the patient. The thinner wall section at the base of the prong can achieve the same benefits described with reference to the grooves or sunken regions but without having a groove.

The cannula body 3200a can be constructed from a single material or a combination of materials. The cannula body 3200a may be constructed from a material having a Shore hardness of between 20A - 100A. For example, the cannula body 3200a may have a Shore hardness of between 20A-90A, 40A-100A, 40A - 90A, 50A-80A, or 60A-70A. The cannula body 3200a can be constructed from a silicone material. As described herein, the thickness proportions of this material may be then altered to achieve the desired stiffness of each regions of the cannula body 3200a, such as the prongs 3220, the grooves 3222, and the sunken region 3223. The grooves 3222 and the sunken region 3223 may have a thickness between 0.1 mm-1.5 mm, 0.2 mm-1. 5mm, 0.2mm-1.3mm, 0.3 mm-1.3 mm, 0.5 mm- 1.2 mm, or 0.5 mm - 1.0 mm. The wall of the prongs 3220 and thickened region 3249 may have thicknesses greater than the grooves 3222 and the sunken region 3223. For example, the wall of the prongs 3220 and thickened region 3249 may have thicknesses between 0.3 mm - 2.0 mm, 0.5 mm - 2.0 mm, 0.6 mm - 2.0 mm, 0.3 mm-1.8 mm, 0.5 mm -1.8 mm, 0.6 mm - 1.8 mm, 0.7 mm - 1.8 mm, 0.6 mm - 1.5 mm, or 0.7 mm - 1.5 mm.

The prongs 3220 extend from the manifold 3240 and can be shaped and formed to minimize tissue compression and kinking during insertion into a user's nares. As illustrated in FIG. 10, each of the nasal prongs 3220 has a gas outlet 3224 configured to direct a flow of gas towards the nares of the user's nose. Each of the nasal prongs 3220 further includes an exterior surface 3225. At least a portion of the exterior surface 3225 is a sealing surface 3226. The sealing surface 3226 is configured to seal the nares of the user's nose. In one embodiment, the sealing surface 3226 is configured to seal with the nasal valve of the user's nose. The sealing surface 3226 may be configured to seal at or adjacent the entry of the nares of the user's nose. In another embodiment, the sealing surface 3226 is configured to seal between the nares entry and the nasal valve.

In some embodiments, the prongs 3220 have a constant or substantially constant shape of cross section along their lengths. For example, each of the prongs 3220 may have a circular or oval cross-sectional shape along its length. When the prongs 3220 have a constant cross section along the region at the distal end (i.e. end adjacent the outlet) of the prongs 3220, the prongs 3220 may compensate for slight movements in and out of the nares, which may assist to maintain a seal. In some embodiments, the prongs 3220 may have a varying shape along their lengths. For example, the prongs 3200 may have a circular cross-section at a portion closer to the base, while the prongs 3200 may have an oval cross-section at a portion closer to the top of the prongs. The varying cross-section at portions of the prongs can be similar to the varying shapes described herein with reference to FIGS. 13A-13D.

FIG. 15 illustrates a cannula body 3200a with a base region 3228 that may provide a spring action or mechanism at the base of the prongs 3220. The base region 3228 can assist to decouple movement of the housing and/or manifold from the movement of the nasal prongs. The base region 3228 may provide the ability for the prongs 3220 to flex about the base region 3228 whilst avoiding kinking of the prongs 3220. The prongs 3220 may flex whilst maintaining position in the nares. For example, the base region 3228 can provide a spring-like movement at the base of the prongs 3220 and/or the area surrounding the base of the prongs 3220. The base region 3228 can apply a spring load to the prongs to absorb some interface movement whilst maintaining the prongs 3220 in position in the nares. As an example, the base region may initially be in a neutral/relaxed position (i.e. natural state of the cannula body) without any forces applied to any part. When inserted in the nares, the action of pushing the prongs into position, (and sealing) can cause the base region 3228 to flex or deflect downwardly into the manifold. Prong position in the nares may remain constant, particularly once the prongs are in a sealed position. If, in use, the housing and/or manifold is pulled downwards and/or away from the face (e.g. by pulling of tubes), the base region 3228 may move towards the neutral position. There can still be enough elasticity in the base region 3228 to maintain the prong position in the nares. For example, the spring load from the base region 3228 can absorb interface movement such as cheek movement, patient movement, or headgear movement. The base region 3228 can assist the movement of the or each prong 3220 from right to left.

The base region 3228 may assist to accommodate varying septum spacings. The base region 3228 may allow one or both prongs 3220 to flex back and forth to adjust for different facial geometries. The base region 3228 may alternatively or additionally provide an upwards push into the nares to maintain a constant seal.

In some embodiments, the base region 3228 can be a reduced thickness region. The reduced thickness region can allow for the spring at the base of the prongs. The base region 3228 can be a reduced thickness relative to the prongs 3220 and the rest of the manifold 3240. The reduced thickness region can be used in combination with or as an alternative to other base regions. For example, the base region 3228 can be a bellows region that can allow for the spring at the base of the prongs by providing a bellows-like structure or configuration at the base region. In further embodiments, as described previously, the nasal prongs can have grooves or sunken regions at the base of the prongs 3220 that allow for the spring-like movement at the base of the prongs.

FIG. 15 illustrates an embodiment of the base region 3228 at the base of the prongs 3220. The prongs 3220 can have a constant wall section with a constant thickness which transitions to a base region 3228 around the prongs that allows for the spring-like movement of the base of the prongs. The prongs can have a constant wall thickness throughout the prong until the base region 3228. The constant wall thickness of the prongs can assist the prongs to be less prone to kinking because the base region 3228 can absorb movement and the flexing can be localized to the base region 3228.

In some embodiments, the prongs 3220 can have a constant wall section with a constant thickness which transitions towards the base region 3228 around the prongs. The base region 3228 may be thinner than the constant wall thickness of the prongs 3220. This base region 3228 can also have a substantially constant thickness throughout that region. In some embodiments, the thickness of the prong 3220 can be between 0.3 mm - 2.0 mm, 0.5 mm - 2.0 mm, 0.6 mm - 2.0 mm, 0.3 mm-1.8 mm, 0.5 mm - 1.8 mm, 0.6 mm - 1.8 mm, 0.7 mm - 1.8 mm, 0.6 mm - 1.5 mm, or 0.7 mm - 1.5 mm. The thickness of the base region 3228 can be between 0.1 mm-1.5 mm, 0.2 mm-1.5mm, 0.2mm-1.3mm, 0.3 mm-1.3 mm, 0.5 mm- 1.2 mm, or 0.5 mm - 1.0 mm. For example, in one embodiment, the thickness of the prong 3220 can be 0.6 mm and the thickness of the base region 3228 can be 0.3 mm. In such an embodiment, the ratio of the thickness between the prongs 3220 to the base region 3228 can be 1:0.5. The ratio of thickness between the prongs 3220 to the base region 3228 can be any other ratio that provides the desired transition therebetween.

As described herein, the thickness proportions of this material may be altered to achieve the desired stiffness of each regions of the cannula body 3200a, such as the prongs 3220, the base region 3223 and 3228, and grooves 3222 (described with reference to FIG. 14A) of the base region. For example, the base region 3228 may have a thickness between 0.1 mm-1.5 mm, 0.2 mm-1.5mm, 0.2mm-1.3mm, 0.3 mm-1.3 mm, 0.5 mm- 1.2 mm, or 0.5 mm - 1.0 mm. The wall of the prongs 3220 with the constant wall thickness may have thicknesses greater than the base region 3228. For example, the wall of the prongs 3220 can have a section where the prong transitions to the base region with a constant wall thickness that may have thicknesses between 0.3 mm - 2.0 mm, 0.5 mm - 2.0 mm, 0.6 mm - 2.0 mm, 0.3 mm-1.8 mm, 0.5 mm -1.8 mm, 0.6 mm - 1.8 mm, 0.7 mm - 1.8 mm, 0.6 mm - 1.5 mm, or 0.7 mm - 1.5 mm.

The base region 3228 can be a thin localized region at the base of the prongs that allows the manifold and/or housing to move relative to the nasal prongs. For example, when movement from the patient or external forces puts pressure on the prongs, the base region 3228 can at least partially absorb the movement and/or force of the prong. The base region 3228 may accommodate or at least partially absorb the movement and/or force of the prong by flexing or deflecting inward. In some embodiments, the base region 3228 may have greater clearance from the septum than the grooves 3222, such that the cannula body 3200a has smaller chance to interfere and/or damage the septum. The base region 3228 may also make cleaning of the cannula body 3200a easier due to the relative lack of small cavities or spaces.

### Coupling of Interface Body and Housing

Turning back to FIGS. 10-12B, the manifold 3240 may include a front portion 3241 opposite the rear portion 3243. The front portion 3241 may include a housing coupling portion 3244 which can be received by the interface body receiving portion 3480 of the housing 3240. The housing coupling portion 3244 and the rest of the manifold 3240 may be shaped and/or sized to be inserted in and received by the housing 3400 and form a gas tight seal. For example, the manifold 3240 may include a ridge 3248 at each of the openings 3246. The ridge may be an annular ridge 3248. The annular ridge 3248 can provide interference with the housing 3400, forming a gas tight seal with the housing 3400.

In the illustrated embodiment, the manifold 3240 includes a raised portion 3242 which defines a raised surface relative to the housing coupling portion 3244 and the rest of the manifold 3240. The raised surface defined by the raised portion 3242 may be discontinuous with the surface of the housing coupling portion 3244. In some embodiments, the raised portion 3242 is formed both at an upper portion and a lower portion of the manifold 3240, such that the housing coupling portion 3244 is surrounded by the raised portion 3242. In some embodiments, the raised portion 3242 may be formed only at an upper portion or a lower portion of the manifold 3240. In some embodiments, the raised portion 3242 can inhibit or prevent the rotation of the cannula body 3200a about the housing 3400, when the cannula body 3200a and the housing 3400 are assembled, as described in further detail herein. In some embodiments, the manifold 3240 does not include the raised portion 3242, and may have a generally continuous outer surface adjacent the housing coupling portion 3244. In some embodiments, the manifold 3240 may include a band or reinforcing member to increase structural integrity of the manifold 3240. For example, the band or reinforcing member can be formed at least in part by a region of significantly increased thickness relative to the rest of the manifold 3240.

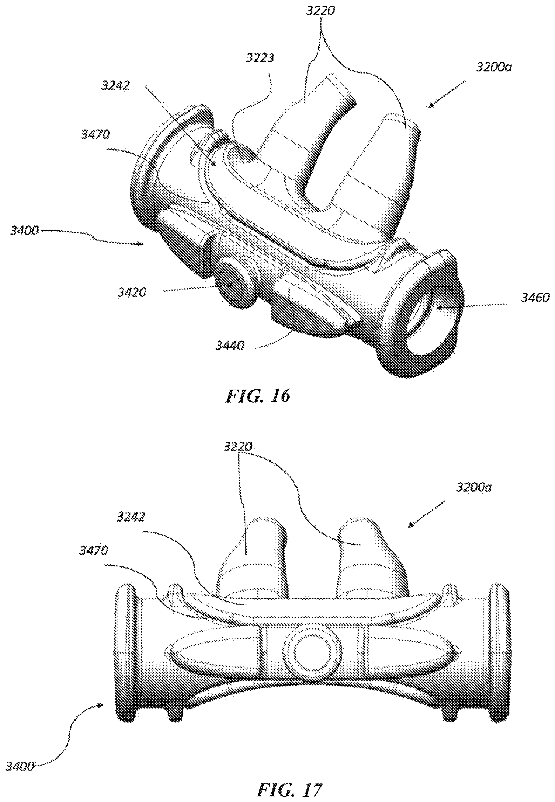

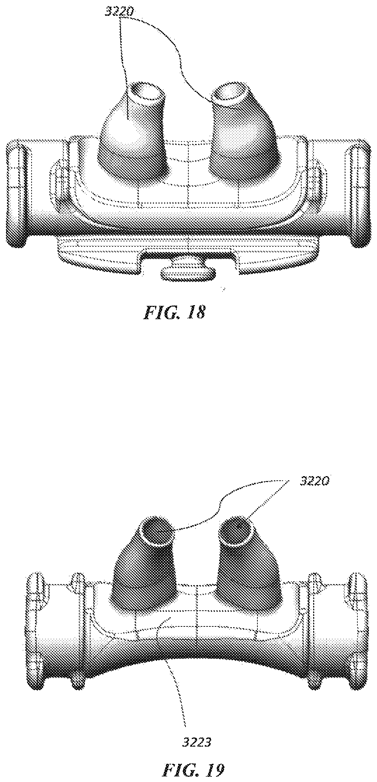

As discussed herein, the cannula body 3200a may be coupled with the housing 3400 by inserting the housing coupling portion 3244 of the manifold 3240 to the interface body receiving portion 3480. FIGS. 16-19 illustrate an embodiment of the cannula body 3200a assembled with the housing 3400. As shown in FIGS. 16-17, the raised portion 3242 of the cannula body 3200a may be exposed when the cannula body 3200a is coupled with the housing 3400. In the illustrated embodiment, the raised portion 3242 matches the upper edge 3470 and/or the lower edge 3490, such that the raised portion 3242 and the upper edge 3470 and/or the lower edge 3490 do not have a gap therebetween when the cannula body 3200a is assembled with the housing 3400. In such embodiments, rotation of the cannula body 3200a about the housing 3400 may be prevented. In some embodiments, the raised portion 3242 and the upper edge 3470 and/or the lower edge 3490 may be shaped such that certain amount of gap exists between them when assembled, to allow certain degree of rotation of the cannula body 3200a about the housing 3400. In some embodiments, the manifold 3240 does not include the raised portion 3242 to allow more free rotation of the cannula body 3200a about the housing 3400, enabling greater degree of adjustment. In some embodiments, the raised portion 3242 may have a thickness similar to the thickness of the housing 3400, such that the raised portion 3242 can form a continuous surface with the housing 3400. In some embodiments, the raised portion 3242 may have a greater thickness or a smaller thickness than the thickness of the housing 3400, such that the raised portion 3242 can protrude relative to the surface of the housing 3400, or be recessed relative to the surface of the housing 3400.

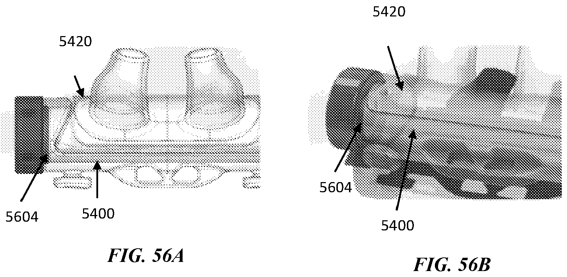

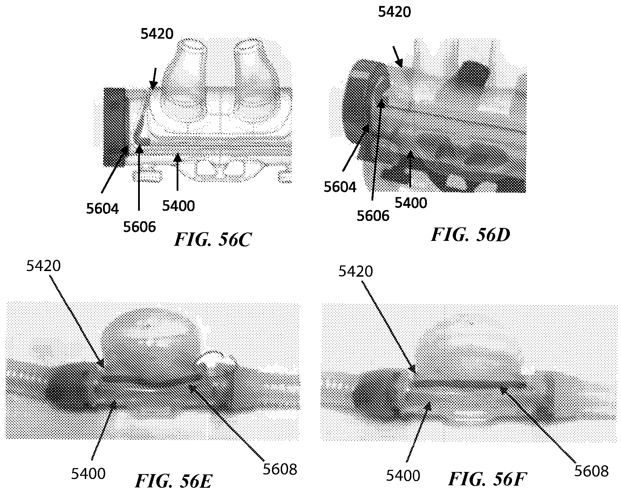

The raised portion 3242 may also assist with visual alignment of the manifold 3240 relative to the housing 3400. For example, the user or the clinician can align the raised portion 3242 with the upper edge 3470 and the lower edge 3490 of the housing 3400 to assemble the manifold 3240 in the correct position. In some embodiments, the manifold 3240 and/or the housing 3400 can include an audible and/or tactile indicator to provide a feedback when the manifold 3240 and the housing 3400 are in the correct position. For example, the manifold 3420 and/or the housing 3400 can make a "click" sound when the manifold 3420 and/or the housing 3400 are correctly positioned and properly assembled. In some embodiments, the manifold 3420 and/or housing 3400 can include a visual indicator that indicates when the manifold 3420 and/or the housing 3400 are correctly positioned and properly assembled. In some cases, the visual indication can be a color indicator. The color indicator can be used with transparent components of the manifold and/or housing. Examples of color indicators are described further herein with reference to FIGS. 56A-56F.

When the housing 3400 and the manifold 3420 are assembled, the housing 3400 may only contact the manifold 3420, and it may not interfere with the prongs 3220. In some embodiments, the sunken region 3223 or the grooves 3222 may be fully or partially exposed, and the movement of the prongs 3220 may not be interfered by the housing 3400.

FIGS. 20A-20B illustrate how the manifold 3240 may be retained within the housing 3400. The internal surfaces of the housing 3400, as shown in FIGS. 20A-20B, may match with the manifold 3240, thereby the manifold 3240 is retained within the housing 3400. As shown in FIG. 20A-20B, the openings 3246 of the manifold 3240 may be retained within the tube engaging portion 3460 of the housing 3400, to receive the interface tubes. In some embodiments, the annular ridge 3248 at each of the openings 3246 of the manifold 3240 can provide interference with the housing 3400, forming a gas tight seal with the inner surface of the housing 3400.

### Mask Body

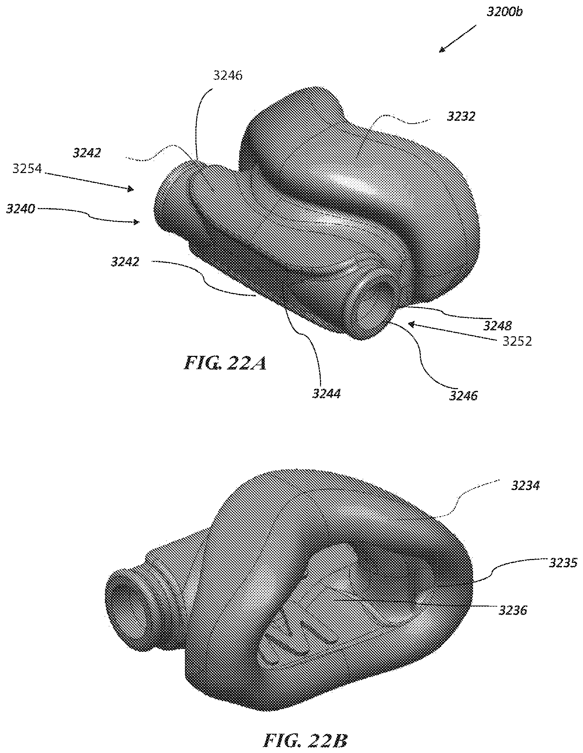

FIGS. 22A-22B illustrate an embodiment of the mask body 3200b. In the illustrated embodiment, the mask body 3200b includes the manifold 3240, which is same or similar to the manifold 3240 of the cannula body 3200a. For example, the manifold 3240 includes the raised portion 3242, the housing coupling portion 3244, the opening 3246, the annular ridges 3248 described in relation with FIGS. 10-13. The manifold 3240 can be coupled with the housing 3400 in substantially the same manner described in relation with the manifold 3240 of the cannula body 3200a. As described herein, the mask body 3200b including the same or similar manifold with the cannula body 3200a, enables swapping between the cannula body 3200a and the mask body 3200b while keeping the housing 3400 same.

In the illustrated embodiment, the mask body 3200b further includes the mask 3232 or mask cushion, extending rearward from the manifold 3240. The mask 3232 may be applied on the face of a patient. In the illustrated embodiment, the mask 3232 is a nasal mask. The nasal mask may form a nasal seal applied over a nose of a patient. The nasal mask may form a seal on or about the nose surrounding the nares of the patient. In some embodiments, the mask 3232 could cover both a nose and a mouth of the patient.

The mask 3232 or mask cushion includes a face-contacting surface 3234. The face-contacting surface 3234 may have an inner peripheral edge 3235 that defines a nose-receiving opening into a mask cavity 3236 for receiving the user's nose in use. In some embodiments, the face-contacting surface 3234 is located generally opposite the manifold 3240. In use, the contacting surface 3234 of the mask 3232 may envelop or circumscribe a portion of the user's nose defining the nares. The contacting surface 3234 may sealingly engage about the user's nose such as against one or more of the cheek surfaces and/or lateral side surfaces of the user's nose, the upper lip region below the user's nose, and across the nasal bridge region or nasal tip region of the user's nose.

The mask 3232 may be substantially hollow and generally shaped to provide or define the mask cavity 3236. The mask cavity 3236 may receive the flow of gases from the manifold 3240. The mask cavity 3236 may be fluidically connected with the manifold 3240 and the openings 3246, such that gas from and to the patient flows through the mask cavity 3236.

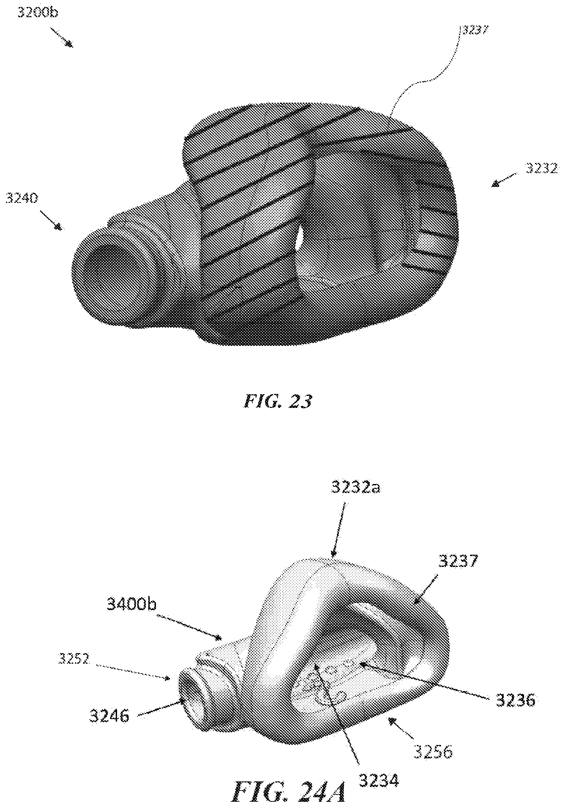

FIG. 23 illustrates a schematic view of the mask body 3200b from the face-contacting side or posterior side of the mask body 3200b. In some embodiments, as shown by a striped region in FIG. 23, a region 3237 of the mask 3232 ("rolling region") may be provided. The region 3237 may be designed such that at least a portion of the region 3237 is configured to roll over onto an outer surface of the mask 3232. The rolling region 3237 may extend at an upper portion and/or side portions of the mask 3232. The rolling region 3237 may include or work in conjunction with, at least part of the face-contacting surface 3234. The rolling region 3237 may assist to relieve pressure applied to the users nose and/or face, such pressure being required to provide a seal adequate for delivery of therapy.

To assist with the rolling of the region 3237, the region 3237 may have a varying thickness or a varying stiffness. The region 3237 can be formed by and/or be coextensive with a reduced stiffness region. In some embodiments, the wall thickness of the mask 3232 may be substantially constant through the rolling region 3237. In some embodiments, the wall thickness of the mask 3232 may vary or alternate between relatively thick and thin regions. The wall thickness of the mask 3232 may be relatively thicker along other portions of the mask, thereby providing support for the structure of the mask 3232. However, other arrangements to induce or facilitate rolling in the rolling region 3237 can also be used. Examples of mask seals comprising rolling portions are disclosed in WO2014/062070.

In some embodiments, a lower or base portion 3256 of the mask 3232 may have a region having greater thickness/stiffness, which may allow greater structural stability of the mask. For example, the base or a portion thereof can be formed at least in part by a region of significantly increased thickness relative to the reduced stiffness region. This base portion 3256 may have greater thickness relative to the face-contacting portions of the mask.

The base portion 3256 may be formed of the same material forming the mask 3232. A region of the base portion having increased thickness may be configured as a thickened band. The base portion 3256 can be positioned on the lower or bottom portion of the mask below the rolling region 3237. The mask can pivot at or about the base portion or thickened band when the rolling region rolls under a force when applying the mask over the patient's face. In other arrangements, the base portion can be or include a component formed of a material that has increased stiffness relative to the silicone or other material(s) forming the mask 3232. This may help with inserting and removing the mask 3232 from the housing 3400 and maintaining a seal in use.

The mask body 3200b can be constructed from a single material or from two or more different materials. The mask body 3200b can be constructed from silicone. The mask body 3200b may be constructed from a material having a Shore hardness of between 15A-80A. For example, the mask body 3200b may have a Shore hardness of between 15A-80A, 20A-80A, 30A-80A, 40A-70A, or 40A - 60A. As described herein, the thickness proportions of this material may be then altered to achieve the desired stiffness of each regions of the mask body 3200b.

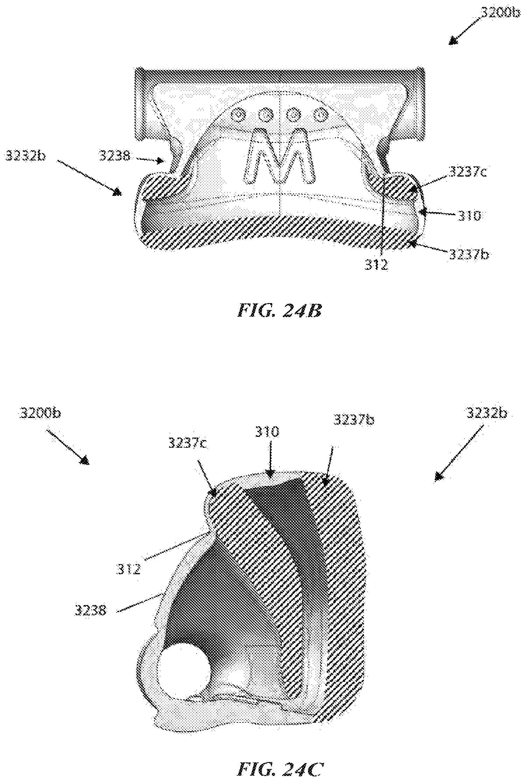

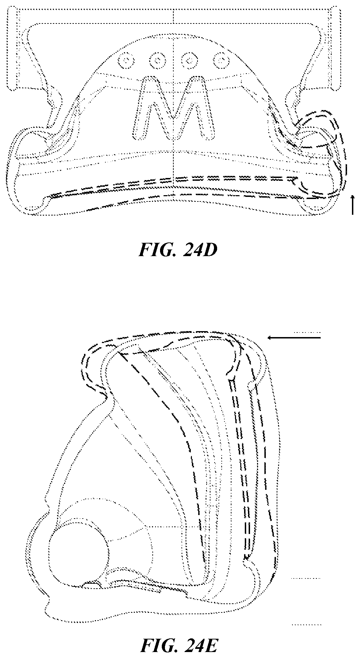

In some embodiments, the mask 3232a can be provided in various or alternative shapes or sizes to achieve the desired fit to achieve comfort, compliance and/or deliver therapy effectively. The mask 3232a illustrated in FIG. 24A is similar to the mask 3232 described in relation to FIGS. 22A-23, however, the mask 3232a may have a different shape. The mask 3232a can have a width measured from a first end 3252 at a first annular ridge 3246 to a second end 3254 (shown in FIG. 22A) at a second annular ridge 3246. The mask 3232a can have a horizontal axis that extends along that width and a vertical axis perpendicular to the horizontal axis. As illustrated in FIG. 24, the mask 3232a can be longer in a vertical direction than mask 3232 described herein. This shape can allow the mask 3232a to seal further up on the bridge of the user's nose than the mask 3232 shown in FIGS. 22-23. This configuration may be more comfortable for the patient. Additionally, sealing higher on the bridge of the user's nose can minimize the risk of occluding the patient airways or otherwise affecting the ability of gas to flow in or out of the patient's airways, particularly on infant patients. For example, by configuring the mask so that the mask can seal higher on the bridge or harder parts of the user's nose as opposed to softer fleshy parts of the nose closer to the tip, the risk of occluding or affecting the flow of gas in or out of the patient airway can be minimized.

### Mask Rolling Region

When the mask 3232 is seated on the face of the user in use, the contacting surface 3234 may lie over the bridge of the nose, sides of the nose and above the upper lip of the user. With a supply of positive pressure air, the contacting surface 3234 may balloon and seal against the face of the user. Leaks may occur where a seal contact pressure between the contacting surface 3234 and the face of the user is less than the pressure of the air being delivered. For therapy to be successfully delivered, the mask contacting surface 3234 pressure should be higher than air pressure around a perimeter of the contacting surface 3234 forming the mask seal.

The mask 3232a can include a rolling region 3237 as described in relation to FIGS. 22A-23. In some embodiments, the rolling region can roll or bend to allow the mask to accommodate facial movement or application of forces on the mask. For example, the rolling aspect at the sides of the mask and the pivot point at the base of the mask can allow the mask to accommodate facial movement or application of forces on the mask. This can allow the mask to maintain the integrity of the seal between the mask and the patient. The thickness of the rolling region 3237 is intended to reduce pressure on the bridge of the nose. In some embodiments, the rolling region 3237 is present at the sides of the mask and can allow the mask to compress into the nasolabial folds to increase the contact between the patient and the mask to form a seal around the nose. This can be useful for example, to accommodate cheek movement. In some embodiments, the rolling region 3227 can allow the mask to form a seal in the fold between the nose and cheeks even if the cheeks are compressed. For example, the cheeks can be compressed during side sleeping or placement on the side, such as during chinstrap use.

To assist with the rolling or bending, the region 3237 can have a varying thickness or a varying stiffness. In the configuration shown in for example FIGS. 24B - 24E, the region 3237 comprises a thin/thick/thin configuration. FIGS. 24B-24E show a mask body 3200b with a mask 3232b with reduced thickness or thin regions 3237b and 3237c that allow for rolling, bending or folding of the mask 3232b. In the embodiment shown, thin region 3237c is configured to roll or bend over relatively thicker or stiffer front portion 3238 of the mask 3232b.

FIGS. 24B-24E show a mask body 3200b with a mask 3232b with thin regions 3237b and 3237c that allow for bending or folding of the mask 3232b.The mask 3232b can have region 3237b with a reduced stiffness at the patient contact surface to conform to the patient's face. The mask 3232b can additionally or alternatively have a region 3237c with a reduced stiffness and/or thickness at the rolling area (top and sides of the mask) to enable a rolling motion with a flattened force-displacement curve. The cross-hatching in FIGS. 24B and 24C illustrate regions 3237b and 3237c which are sections of the mask 3232b that have reduced stiffness. The reduced stiffness may be provided via reduced thickness or thinning of these regions relative to adjacent or other portions of the mask. These regions 3237b, 3237c therefore allow for more bending and flexibility relative to the thicker or stiffer material sections.

The reduced stiffness of the regions 3237b and 3237c can be formed by a thin material forming the section. The patient contacting surface of region 3237b and the region 3237c are shown in FIGS. 24B and 24C. The varying stiffness of the regions can create hinge points or pivot points which may direct movement of the mask 3232b. In the embodiment shown, the hinge points or pivot points are located at or adjacent the base of the mask 3232. When the mask cushion is compressed, such as when the mask 3232 is pushed towards the patient face in use, the region 3237c may roll forwards from a pivot or hinge point at or adjacent the base of the mask 3232. Region 3237c may roll or bend over relatively thicker or stiffer front portion 3238 of the mask 3232b. Region 3237c may therefore be referred to as a rolling region 3237c. The pivot or hinge point may remain at the base of the mask when the mask is used with or without a midline securement member.

The mask 3232b can have a stiffened structural portion 310 in between the two regions 3237b and 3237c. The stiffened structural portion 310 can be stiffer or formed of a thicker material than the regions 3237b and region 3237c. The structural portion 310 may be continuous or unbroken around the circumference of the mask. The structural portion 310 may extend partially around the circumference of the mask. In the embodiment shown in for example, FIG. 24C, the structural portion 310 may extend around the sides and top of the mask. The structural portion 310 may be a band. The band may be located around sides and top of the mask.

The stiffer or thicker structural portion 310 or band may gradually transition into the rest of the rolling region. In other words, the structural portion 310 or band may gradually transition into region 3237b and/or 3237c. This can improve comfort as it reduces likelihood of the band or other such relatively hard section from contacting the user's skin and/or nasal bridge which may occur if the thickened section does not gradually transition. In some embodiments, the gradual transition of the structural portion 310 into the rest of the rolling region 3237 can also improve the ease of cleaning. In some embodiments, the structural portion 310 may gradually transition into the rest of the rolling region 3237.

**In** some cases, such as at higher pressures, the structural portion 310 may help prevent the mask from inflating. The structural portion 310 can also help to prevent folds or creases in the thin material sections of the regions 3237b, 3237c, which may assist to keep the region 3237b conformable to the patient. In some embodiments, the structural portion 310 can make it easier to clean the mask 3232b.

The mask 3232b and the region 3237c may be designed to roll or bend at the top of the mask as well as at the sides of the mask. The rolling at the top is intended to reduce pressure on the bridge of the nose and to better accommodate a range of patient facial geometries. The rolling at the sides is intended to enable the mask to sink down into the cheek (nasolabial) folds and achieve sufficient contact to create a seal. The rolling at the sides can allow the mask to rock side to side while maintaining a seal, thus decreasing the likelihood that side forces will dislodge the seal (e.g. lying in lateral position). In some cases, cheek folds can be increased through patient positioning, the use of chinstraps, and/or tension applied to other patient securing mechanisms or devices.

The structural portion 310 can blend in at the front or patient facing side of the mask 3232b so that there are no hard edges should the region 3237b that contacts the patient buckle. The blending may be less necessary on the opposite or non-patient facing side of the structural portion 310. However, in some cases, the structural portion 310 can be blended on both sides of the structural portion 310. The structural portion 310 can be positioned to affect the force-displacement profile of the rolling region at the top and/or side. For example, the force profile may be relatively flat as the thin material of the reduced stiffness section rolls until the thickness of the rolling material begins to increase (either abruptly or gradually). The increase in the thickness of the rolling material can then ramp up the force-displacement profile. The gradual thinning or gradual reduction in stiffness of the structural portion 310 can be used to allow the rolling region to roll for certain distance before gently resisting displacement rather than going from a low force to a high force when the rolling region stops rolling.

The mask 3232b can roll at the sides and pivot at or adjacent the base of the mask. As shown in FIG. 24B, the rolling region 3237c can be positioned around a point 312 at the base of the mask 3232b. The material thickness of the wall at the base of the mask 3232b can thicken or stiffen as it moves away from point 312 toward the rear of the mask. As shown in FIG. 24B, the region 3237c with reduced stiffness can extend inward and rearward of point 312 which allows the mask 3232b to both roll at point 312 and hinge further inward. This configuration can enable the side of the mask 3232b to be displaced or roll easier when the force is exerted on the mask 3232b. In some embodiments, the mask can have a rolling region or region 3237c that continues around the whole circumference of the mask 3232b. In some embodiments, the mask can have a thickened or stiffened base that can add structure to for example, prevent the mask from folding in half laterally.

To aid with stability it can be useful to have an interface retention or stability apparatus (for example, headgear) attach near the pivot point. The base of region 3237c as shown in FIGS. 24B-24C follows somewhat of a triangular form, but, in some embodiments, the rolling region can be more rounded.

In some embodiments, the reduced stiffness section of material can have a thickness of approximately 0.5mm or less. The reduced stiffness section of material can have a thickness of 0.8 mm or less, 0.7mm or less, 0.6mm or less, 0.5mm or less, 0.4mm or less, or 0.3mm or less. In some embodiments, the ratio of thickness of the structural portion 310 to the reduced stiffness section can be about 20:1, 15:1, 10:1, 5:1, 2:1, or 1.5:1.

The mask 3232b can assist with sealing and the seal contact pressure between the face-contacting surface 3234 and patient can be sufficient to prevent leaks. The seal contact pressure can be applied to the skin at the nasal bone and the region 3237b at the top of the mask 3232b can contact the patient's skin.

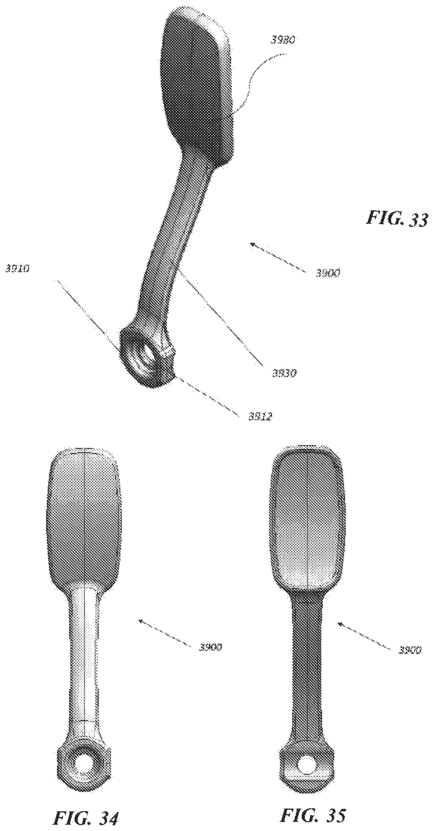

The mask 3232a can be used in combination with one or more securement members to provide stability of the mask on the user. The mask 3232b can be used in combination with a midline securement member (for example, the securement member described with reference to FIGS. 33-35 and 40-47) which is applied to the forehead of the patient. This securement member can compress the rolling region and the contact pressure can be above the delivered air pressure when the mask is sealing. The regions 3237b and 3237c can reduce compression on the bridge and/or sides of the nose while still achieving a proper seal and stabilization of the mask 3232b.

The rolling region of the mask 3232b can roll at the sides as well as the top. FIGS. 24D and 24E illustrate the movement of the mask 3232b as it rolls at the sides and the top. FIG. 24D is a top cross-sectional view of a nasal interface. The top cross-section of the nasal interface shown in FIG. 24D illustrates the side roll of the mask 3232b shown in dashed lines and moving in the direction of the arrow. As shown in FIG. 24D, the mask 3232b pivots at the point 312 as the regions 3237b and 3237c are compressed with forces acting on one side of the mask 3232b. As the mask pivots, region 3237c bends or rolls over the front surface 3238 of the mask. FIG. 24E is a side cross-sectional view of a nasal interface. The side cross-section of the nasal interface shown in FIG. 24E illustrates the top roll of the mask 3232b shown in dashed lines and moving in the direction of the arrow. As shown in FIG. 24E, the mask 3232b hinges at the base of the mask as region 3237c bends or rolls forwards with forces acting on the front top portion of the mask 3232b as shown by the arrow.

The side roll of the mask can allow the seal to be compliant at the sides by allowing the mask to sink in at the nasolabial folds. The side roll of the mask may also help to accommodate cheek movement.

### Entry and Exit Points

In some embodiments, the interface body can have lateral entry and exit points to the mask chamber or nasal prongs so that the flow of gas can enter the interface body from the tubing on each end of the housing. The lateral flow of gas entering and exiting the interface body can result in greater CO₂ clearance when compared to a mask or fluid interface that has an entry and exit point located in the middle or top of the mask. Additionally, the lateral entry and exit points for the flow of gas can reduce dead space outside the flow path in the interface body. In some embodiments, the housing is configured such that gas may enter and/or exit the interface body substantially in line with a horizontal axis of the housing and/or interface body. In some embodiments, ends of the housing at or adjacent the entry/exit points may curve or extend generally downwards. In such embodiments, the entry/exit points may direct gas into the interface body laterally, but at an angle offset from the horizontal.

### Securement Members

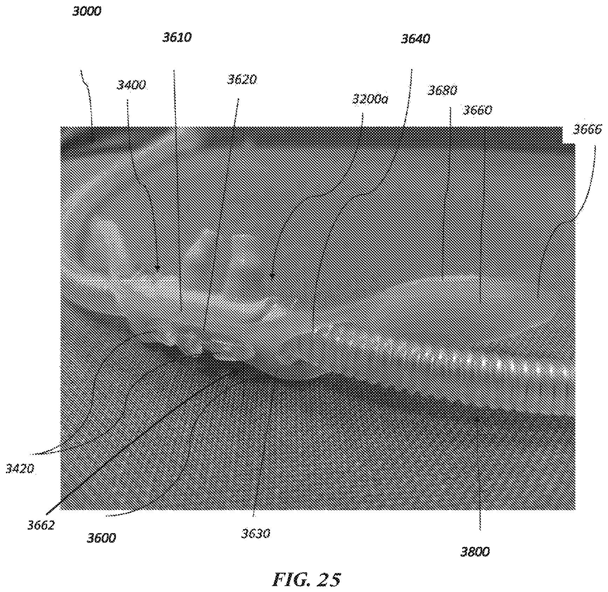

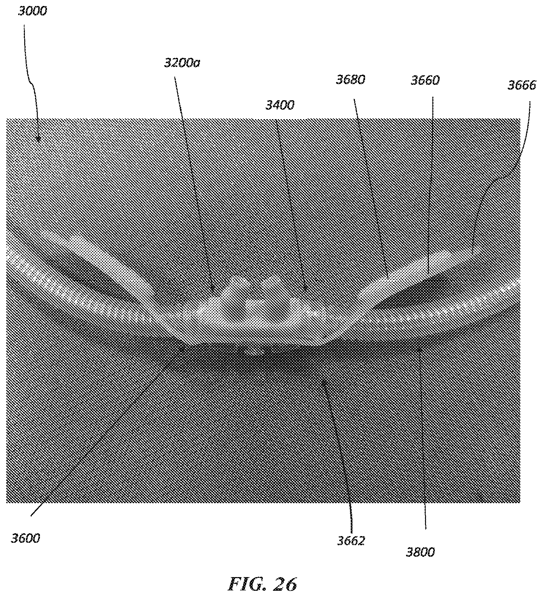

FIGS. 25 and 26 show an embodiment of the patient interface 3000 assembled to form a nasal cannula interface. In the illustrated embodiment, the patient interface 3000 includes the cannula body 3200a, the housing 3400, the first securement member 3600, and the interface tubes 3800. As shown in FIG. 25, the first securement member 3600 includes a central portion 3610, support pads 3660, and bridging portions 3630 extending between the central portion 3610 and the support pads 3660. As shown in FIGS. 25 and 26, the bridging portion 3630 can be positioned on opposing sides of the central portion 3610. The support pads 3660 can extend laterally outward from the bridging portions 3630. In some embodiments, the first securement member 3600 can include a single support pad 3660 extending from a single bridging portion 3630. In some embodiments, the first securement member 3600 can include more than one support pad 3660 extending from bridging portions 3630 on the outer perimeter of the central portion 3610. In some embodiments, the first securement member 3600 can include a pair of support pads 3660. Each support pad 3660 in the pair can extend from bridging portion 3630 at opposing ends of the central portion 3610 as shown in FIGS. 25 and 26.

As illustrated in FIGS. 25-26, the central portion 3610 may have a generally low profile. The central portion 3610 can have a small thickness that allows it to minimally extend outwardly from the patients face. In some embodiments, the central portion 3610 may have a generally planar shape. In some embodiments, the central portion 3610 may have a generally rectangular shape. In some embodiments, the central portion 3610 may be generally curved. In some embodiments, the central portion 3610 may generally curve outwardly from the patients face in use. In some embodiments, the central portion 3610 is permanently or semi- permanently connected with the bridging portions 3630. In some embodiments, the central portion 3610 and the bridging portions 3630 are integrally formed in a single, unitary piece. In some embodiments, the central portion 3610, the bridging portions 3630, and the support pads 3660 may be integrally formed in a single, unitary piece. For example, the central portion 3610, the bridging portions 3630 and the support pads 3660 may be molded as a single piece from a single material, co-molded or over-molded as a single piece from different materials, welded (e.g. ultrasonically welded, heat welded), or otherwise attached by any suitable method to form a single piece. In some cases, the ease of manufacturing may be improved if the central portion 3610 and the bridging portions 3630 are individually formed and subsequently assembled or coupled. In some embodiments, the first securement member 3600 and the second securement member 3900 can be integrally formed.

The support pads (*e.g.,* the support pads 3660) can rest directly or indirectly on a user's face when the patient interface (*e.g*., the patient interface 3000) is worn by the user. For example, the support pads 3660 can be secured to the user's face directly or indirectly by an external force, such as using an adhesive connection, a headgear arrangement, a hook or loop connection as discussed above, or another suitable support structure, such as any of those described herein. In some arrangements, the support pads 3660 can be connected to a headgear and/or a chinstrap for the securement of the interface body. In some embodiments, the support pads 3660 can be connected to one or more facial pads or dermal patches. The facial pads or dermal patches can have a patient side and an interface side. The interface side can have a first fastener element. The first fastener element can removably couple or attach to a complementary fastener element on a patient side of the support pad 3660. In some embodiments, the support pad 3660 may include tabs 3666 at either end of the support pad 3660 to aid in positioning and/or removing the support pad 3660 and/or the first securement member 3600.

Accordingly, the bridging portions (e.g., the bridging portion 3630), which are connected to the support pads, may be moved or deflected when the user's face moves. For example, when a portion of the user's face on which the support pads rest moves, such external force or movement may be conducted to the bridging portion 3630, then to the central portion (e.g., the central portion 3610), and eventually to the housing (e.g., the housing 3400) and the interface body (e.g., the cannula body 3200a, the mask body 3200b). In some instances, the bridging portions themselves may move or deflect when an external force is exerted on them. Accordingly, it is desirable for the bridging portion to be constructed such that the interface body can maintain its seal with the user even when the user's face and/or the support pads move. In some configurations, the deformable configuration of the bridging portion 3630 provides some degree of de-coupling of the support pads 3660 from the interface body. Preferably, the deformation of the bridging portion 3630 at least reduces or possibly eliminates disruption of the seal between the interface body and the user's face as a result of cheek movements of the user's face or external forces exerted on the face of the user. Additionally, the deformation of the bridging portion of the securement members can work in combination with the rolling regions of the mask to maintain a seal between the interface body and the user's face.

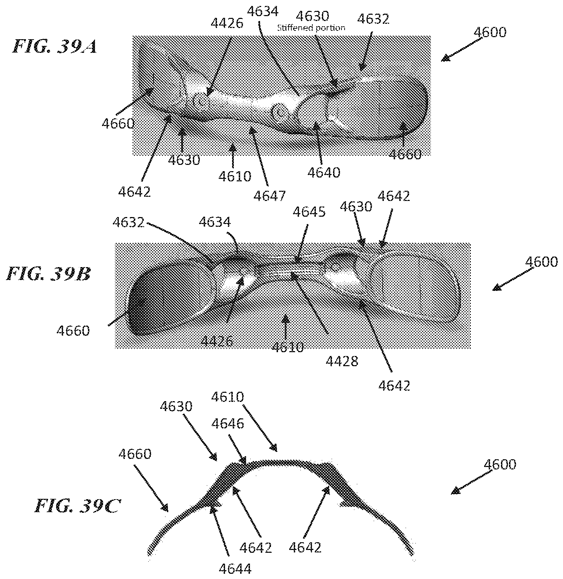

In some embodiments, the first securement member 3600 can accommodate movements of the patient's face with one or more hinge points located further outward from the surface of the patient's face than the location of the support pads 3660. As shown in FIGS. 25 and 26, the hinge point 3662 can be located at a portion of the first securement member 3600 where the first securement member 3600 connects to the housing 3400. In some cases, the hinge points can be located at the connection of the bridging portion 3630 with the central portion 3610 of the first securement member 3600. In some embodiments, such as shown in FIG. 39, the bridging portion 3630 can assist to maintain torsional stability and prevent or minimize movement of the central portion 3610 and the manifold with respect to each other.

In some embodiments, the bridging portion 3630, or the entire first securement member 3600 are made of a flexible material, such that the bridging portion 3630 is flexible or deformable, such as described above. In some embodiments, the bridging portion 3630 are made of the same material as the central portion 3610 in an assembled or unitary construction. In some embodiments, the first securement member 3600 may be made of one or more elastomer materials, such as silicone, rubber, polyethylene, etc. In some embodiments, the first securement member 3600 may be made of a single material. In some embodiments, the first securement member 3600 may be made of two or more materials, such that a region of the bridging portion 3630 has a different flexibility relative to another region. In some embodiments, at least a region of the bridging portions 3630 may be made of flexible but tensile material, such that the bridging portions 3630 are stiff enough to resist torsion forces, while being able to be compressed. This may assist to minimize translation of forces and/or movements from the patient's cheeks to the sealing region. In some embodiments, the first securement member 3600 may be constructed from a same material as the interface body (e.g. the cannula body 3200a, the mask body 3200b) and/or the housing 3400.

The patient facing side of the support pad 3660 can have a fastener element to allow direct attachment to the face (for example, an adhesive) or indirect attachment to headgear or a patient interface patch or dermal patch which can have a complementary fastener element on the non-patient facing side. The user-facing surface 3680 of the support pads 3660 can be initially provided without a user interface patch or a dermal patch. The surface 3680 can receive or retain a patient interface patch or a dermal patch. Such a patient interface patch may be connected to the surface 3680 by an adhesive or other suitable connection as is known in the art (such as by hook-and-loop fastener, ultrasonic welding, and/or co-molding or overmolding). Once the support pad patch is in position, it may be connected to or receive a dermal patch. Alternatively, the patient interface patch may be assembled to the surface 3680 of the support pad 3660 in the same operation as molding the facial pad. In some embodiments, the user-facing surface 3680 is configured such that the user-facing surface 3680 may not cause excessive abrasion or disturbance on the patient's face. For example, the user-facing surface 3680 will include a loop material of the hook-and-loop mechanism, while the user interface patch or the dermal patch includes a hook material of the hook-and-loop mechanism. In some embodiments, the user-facing surface 3680 engages a securement mechanism other than the dermal patch, such as a headgear, a chinstrap or a bonnet.

In some embodiments, the first securement member 3600 may be coupled to the housing 3400 by coupling the central portion 3610 to the housing 3400. The first securement member 3600 may have a mechanism or attachment portion which can be retained by the housing 3400. For example, the first securement member 3600 may include a central opening 3620. The central opening 3620 may be coupled with one or more attachment elements of the housing 3400. For example, the central opening 3620 may be coupled with one or more front extension 3420 and/or central protrusion 6440 of the housing 3400. The central opening 3620 may be sized and shaped to receive and retain the one or more attachment elements of the housing 3400, such as front extension 3420. For example, the central opening 3620 may be sized and shaped to be retained at or around the ridge 3422. In some embodiments, the first securement member 3600 may be detachably coupled to the housing 3400. In some embodiments, the first securement member 3600 may be coupled to the housing 3400 with a clip. In some embodiments, the first securement member 3600 may be permanently coupled to the housing 3400. In some embodiments, the first securement member may not have the central portion 3610 and/or the bridging portion 3630 and may include the support pads 3600 directly coupled with the housing 3400 or the interface body detachably or permanently.

In some embodiments, the bridging portion 3630 may include a cutout 3640. The cutout 3640 may be located to allow passage of interface tubes, such as the interface tube 3800 through the first securement member 3600. In some embodiments, the bridging portion 3630, or any other region of the first securement member 3600 may include a mechanism to further retain the interface tube 3800 to facilitate or assist the management of the interface tube 3800. For example, the bridging portion 3630 may include a hook and loop fastener or an adhesive at the cutout 3640, which may be detachably coupled to the interface tube 3800.

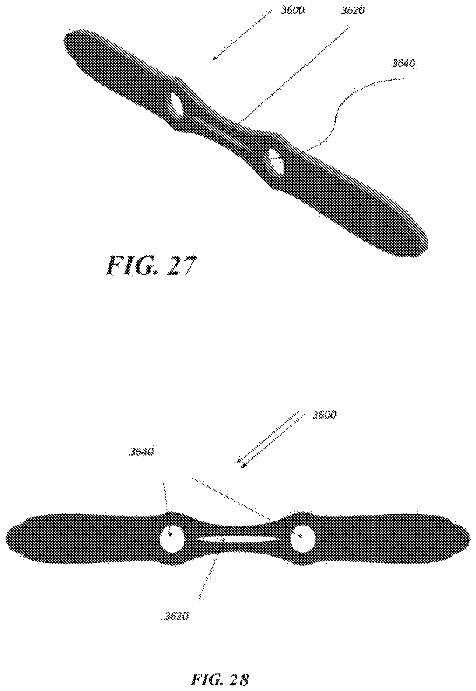

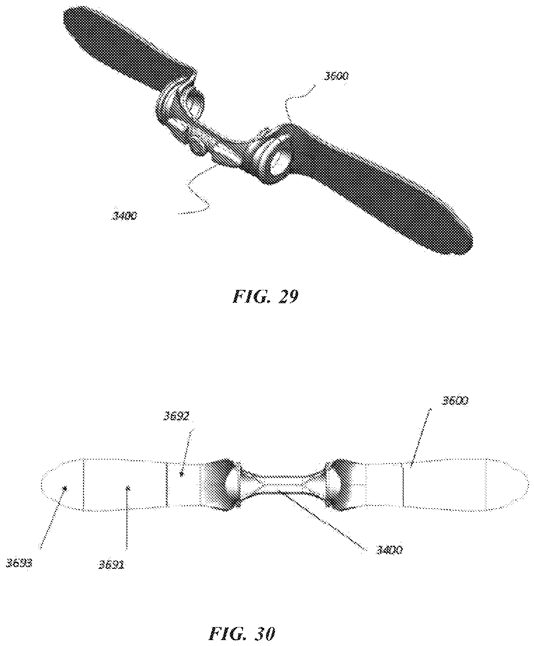

In some embodiments, the first securement member 3600 may be constructed from a fabric material. FIGS. 27-28 illustrate an embodiment of the first securement member 3600 constructed from a fabric material. The first securement member 3600 constructed from a fabric material may be similar with the first securement member 3600 described in relation to FIGS. 25-26, except as described herein. For example, the first securement member 3600 may include a central opening 3620 for coupling with the housing 3400. The first securement member 3600 may include one or more cutouts 3640 to allow the interface tube 3800 to pass through. FIG. 29-30 illustrate the first securement member 3600 coupled to the housing 3400.

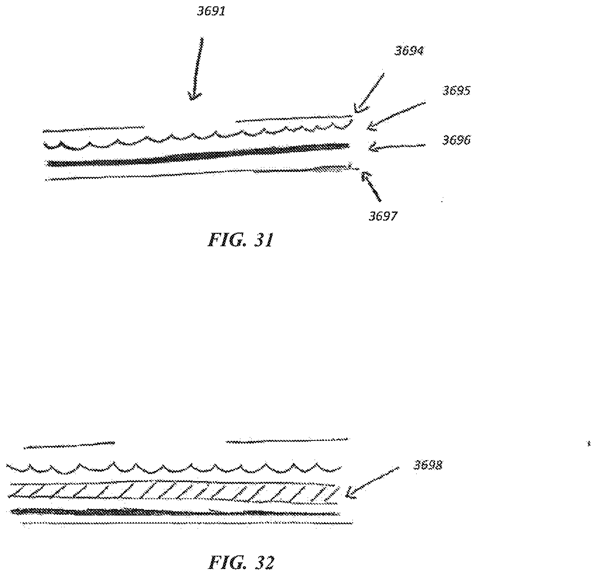

FIGS. 31-32 illustrates a schematic cross-sectional view of the fabric of the first securement member 3600, showing its multi-layer structure. The multi-layer structure may comprise one or more mechanical fastener substrates sandwiched between a pair of film layers. In the illustrated embodiment, an unbroken loop material 3695, and a nylon loop material 3696 may be sandwiched between two outer thin laminate films 3694, 3697. The laminate films 3694, 3697 may allow for easier cleaning and maintenance of the first securement member 3600. The laminate films 3694, 3697 may assist in reducing the amount of moisture and other material that the fabric of the first securement member 3600 can absorb. In some embodiments, the laminate film 3694 at a patient-facing side may be interrupted at an exposed region 3691 to reveal a portion of the unbroken loop material 3695. The exposed region 3691 is also shown in FIG. 30. The exposed unbroken loop material 3695 at the exposed region 3691 may be attached to corresponding hook material on the face of the user, a headgear or a chinstrap, to secure the patient interface 3000. In some embodiments, the unbroken loop material 3695 and the nylon loop material 3696 may be overmoulded with elastomer layers (e.g. thermoplastic elastomer), instead of being laminated with the nylon films 3694, 3697. The nylon films 3694, 3697 may provide the first securement member 3600 stiffness. The nylon films 3694, 3697 may form a non-stretch region 3692 which may assist the stability and support of the patient interface. In some embodiments, the first securement member 3600 may include tabs 3693 at either end of the first securement member 3600 to aid in positioning and/or removing the first securement member 3600. The tab 3693 may be located adjacent the exposed portion 3691. In some embodiments, the first securement member 3600 may further include a foam material 3698 as shown in FIG. 32. The foam material 3698 may be located between the unbroken loop material 3695 and nylon loop material 3696. In some embodiments, the first securement member 3600 may be cut from, for example die-cut, from a preformed multi-layer fabric. In some embodiments, the first securement member 3600 may include perforated strips such that the first securement member 3600 can be cut or torn off at certain positions in order to size the first securement member 3600 for a patient.

FIGS. 33-35 illustrate an embodiment of the second securement member 3900. In the illustrated embodiment, the second securement member 3900 includes a coupling portion 3910, a support pad 3980, and a bridging portion 3930 extending between the coupling portion 3910 and the support pad 3980. The support pad 3980 may be a forehead support pad. The second securement member 3900 may extend generally vertically when the patient interface 3000 is in use on the user's face.

The coupling portion 3910 may be attached to or attachable with the housing 3400 to couple the second securement member 3900 to the patient interface 3000. In some embodiments, the coupling portion 3910 includes a hole or recess 3912. The hole or recess 3912 can be configured to receive the central protrusion 3440, such that the coupling portion 3910 is attached to the housing 3400. In some embodiments, the coupling portion 3910 and/or the housing 3400 may be configured such that the rotation of the second securement member 3900 about the housing 3400 is inhibited. For example, the outer perimeter of the coupling portion 3910 may be multisided and embedded between the central protrusion 3440 and the front extension 3420, such that the front extension 3420 inhibits the rotation of the coupling portion 3910. In some embodiments, the second securement member 3900 may be configured such that it can rotate about the housing 3400. In some embodiments, the central protrusion 3400 may be symmetric, such that the housing 3400 can be symmetric, as described herein. In some embodiments, the second securement member 3900 may be coupled to the housing either in upwardly, or downwardly direction. In some embodiments, the second securement member 3900 may be applied in other orientations, such as sideways or any angled orientations.

In some embodiments, the second securement member 3900 may be detachably attached to the housing 3400. In some embodiments, the second securement member 3900 may be permanently attached to the housing 3400. The second securement member 3900 may be overmoulded and/or formed as an integral piece with the housing 3400. In some embodiments, the second securement member 3900 may be detachably or permanently coupled to the interface body, such as the cannula body 3200a and the mask body 3200b. In some embodiments, the second securement member 3900 may be detachably or permanently coupled to the first securement member 3600. In some embodiments where the secondary securement member 3900 is integral with the first securement member 3600, the secondary securement member 3900 may include perforated strips such that the second securement member 3900 can be cut or torn off from the first securement member 3600. In some embodiments, the first securement member and/or the second securement member can be integral with a headgear of a patient.

The support pad 3980 can be configured to rest directly or indirectly on a user's face. For example, the support pad 3980 can be positioned at or on the forehead, when the patient interface 3000 is worn by the user. For example, the support pad 3980 can be secured to the user's forehead directly or indirectly by an external force, such as using an adhesive connection, a headgear arrangement, a hook or loop connection as discussed above, or another suitable support structure, such as any of those described herein. In some arrangements, the support pad 3980 can be connected to a headgear and/or a chinstrap for the securement of the interface body.

In some embodiments, the second securement member 3980 may be constructed from a fabric material. The fabric material may be the fabric material described in relation to FIGS. 31-32. In some embodiments, the second securement member 3980 may be constructed from a rigid material, such as a thermoplastic or a combination of materials. In some embodiments, the second securement member 3980 can be constructed from the same material as the housing, for example, if the second securement member 3980 is integrally formed with the housing. In some embodiments, the second securement member 3980 may be constructed from one or more elastomer materials, such as silicone, rubber, polyethylene, etc. In some embodiments, the second securement member 3900 may include perforated strips such that the second securement member 3900 can be cut or torn off at certain positions in order to size the second securement member 3900 for a patient.

### Additional Examples of Patient Interface Assembly

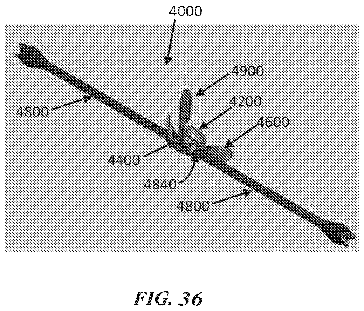

FIG. 36 illustrates an embodiment of a patient interface assembly 4000. As shown in FIG. 36, the patient interface assembly 4000 includes an interface body 4200 which can form a seal with at least part of a user's airway and allow gas flow from and to the user, and a housing 4400 which can be coupled with the interface body 4200. In some embodiments, the interface body 4200 is a cannula body, such as the cannula body 3200a illustrated in FIGS. 3-4 and 10-21 or a mask body, such as the mask body 3200b illustrated in FIGS. 5-6 and 2-24. The cannula body 3200a can form a seal with one or both nares of the user's nose. The mask body 3200b can form a seal with the nose, mouth or nose and mouth of the user. In the embodiment shown in FIGS. 36, the mask body 3200b is a nasal mask. The cannula body 3200a and the mask body 3200b can be interchangeably coupled with the housing. The illustrated embodiment of the patient interface 4000 also includes a first securement member 4600, one or more interface tubes 4800, and a second securement member 4900. The components and features described in these embodiments of the patient interface assembly 4000 can be used with any embodiments of the patient interface assembly and components of the patient interface assembly described previously.

In some embodiments, the interface housing comprises a horizontal axis extending from one tube engaging portion to a second tube engaging portion on the opposing end of the housing. In some embodiments, the interface tubes can engage with the housing at the tube engaging portions and can extend laterally outward along the horizonal axis from the tube engaging portions as shown in FIG. 36. In other embodiments, the interface tubes can engage with the housing at the tube engaging portions and can be biased downward or angled away from the horizontal axis of the housing. In such an embodiment, the first securement member can be positioned above the tubes as opposed to the tubes passing through the first securement member.

In some embodiments, the first securement member 4600 and/or second securement member 4900 may be coupled to or with the housing 4400. In some embodiments, the first securement member 4600 and/or second securement member 4900 may be integral with the housing 4400 and/or the interface body. FIGS. 37 and 38 illustrate a housing 4400 with features for attachment of the first securement member 4600 and/or the second securement member 4900. In the embodiments shown in FIGS. 37 and 38, the features for attachment are located on the front side or front portion 4430 of the housing 4400. The features for attachment may include one or more attachment elements. The one or more attachment elements may be similar to attachment elements described previously such as in relation to embodiments shown in FIGS. 7-9.

In some embodiments, the housing 4400 can be formed from a rigid material. The housing 4400 can be detachably coupled with an interface body as described herein. The housing 4400 can be detachably coupled with a manifold of the interface body. In the illustrated embodiment, the front portion 4430 includes one or more attachment elements in the form of one or more protrusions. The protrusions may include one or more side protrusions 4420 and a central protrusion 4440. The side protrusions 4420 may be raised toward a front direction. The side protrusions 4420 may extend outwardly from the front portion 4430. The side protrusions may have an enlarged head 4422 and a stem 4424. The head 4422 may have a larger cross section than the stem 4424. The side protrusions 4420 can be received by and engage with one or more complementary or receiving components. For example, the or each side protrusion 4420 may be received by and engage with a hole or recess which fits to the enlarged head 4422, for example by push-fit. For example, the first securement member 4600 can be detachably coupled to the housing 4400 at the side protrusions 4420. The first securement member 4600 may have one or more holes or recesses configured to receive or engage with the side protrusions 4420.

The central protrusion 4440 can include a feature to couple the second securement member 4900 to the housing 4400. The central protrusion 4440 can include an opening 4442. In some embodiments, the second securement member 4900 can be coupled to the housing 4400 through the opening 4442 in the central protrusion 4440. In some embodiments, the opening 4442 can have one or more channels 4443 for insertion of the second securement member 4900. In some embodiments, the central protrusion 4440 can include two channels in the opening 4442 as shown in FIGS. 37 and 38. In some embodiments, such as shown in FIG. 38, the central protrusion 4440 can include a lip 4446. The lip 4446 can engage with a complementary fitting portion of a second securement member 4900 (not shown) as described in more detail herein. The features on the housing 4400 assist in securing the first securement member 4600 and/or the second securement member 4900 to the housing 4400. This may position the securement members for subsequent attachment to the patient with suitable mechanisms such as patient interface patch, a dermal patch on the patient's face, an adhesive, any other suitable mechanisms, or through other suitable devices such as a headgear, chinstrap, or bonnet, to stabilize the seal of the mask body or cannula body to the patient. The features on the housing for securing the first securement member 4600 and/or the second securement member 4900 to the housing can have a low profile to minimize the outward extension of the features from the patient's face. This arrangement can minimize the bulk or profile of the patient interface on the patient.

### First Securement Member

FIGS. 39A-39B illustrate views of an embodiment of the first securement member 4600. As shown in FIG. 39A-39B, the first securement member 4600 includes a central portion 4610, support pads 4660, and bridging portions 4630 extending between the central portion 4610 and the support pads 4660. The first securement member 4600 with the central portion 4610, support pads 4660, and bridging portions 4630 can be similar to and formed and used in the same way as the first securement member 3600 with a central portion 3610, support pads 3660, and bridging portions 3630 described in relation to FIGS. 25 and 26, except as described herein. As shown in FIGS. 39A and 39B, the bridging portion 4630 can be positioned on opposing sides of the central portion 4610. The support pads 4660 can extend laterally outward from the bridging portions 4630. In some embodiments, the first securement member 4600 can include a single support pad 4660 extending from a single bridging portion 4630. In some embodiments, the first securement member 4600 can include more than one support pad 4660 extending from bridging portions 4630 on the outer perimeter of the central portion 4610. In some embodiments, the first securement member 4600 can include a pair of support pads 4660 extending from bridging portion 4630 at opposing ends of the central portion 4610 as shown in FIGS. 39A and 39B.

The first securement member 4600 can include a pair of support pads 4660 configured to rest on or engage with the face of a user on opposing sides of the central portion 4610. In some embodiments, the first securement member 4600 includes means to attach and secure the patient interface on a headgear, a chinstrap, a patient interface patch or a dermal patch on the patient's face, or otherwise on the patient's face by any suitable mechanisms.

In some embodiments, the first securement member 4600 may include cutouts 4640 and two arms 4642 bridging the central portion 4610 and the support pads 4660. The cutouts 4640 can allow the interface tube 4800 to pass through between the two arms 4642 and through the cutouts 4640. The cutouts 4640 can receive the interface tubes 4800 through the cutouts 4640. The cutouts 4640 can be fitted over the interface tubes 4800 and secured in place. In some embodiments, the arms 4642 can be positioned around the interface tubes 4800 when the tubes 4800 are extended through the cutouts 4640. In some embodiments, the arms 4642 can be flexed or fitted around the tubes 4800. The arms 4642 may be configured to 'hug' or lie adjacent the tubes 4800. This configuration may assist in reducing the thickness or bulk of the interface assembly while still allowing the bridging portion 4630 the freedom to hinge or flex. The cutouts 4640 of the bridging portion 4630 provide the passage for the interface tubes 4800 to pass through the cutouts 4640 and connect to the housing 4400. In some cases, the interface tubes 4800 can be integral with the housing 4400. In some cases, the interface tubes 4800 can be detachably coupled to the housing 4400.

As shown in FIGS. 39A and 39B, the first securement member 4600 can be curved 4634 where the bridging portion 4630 meets with the central portion 4610. The curve may assist to accommodate the tubular shape of the tube 4800 and/or the housing 4400. FIG. 39C illustrates a top view of the first securement member 4600. The arms 4642 of the bridging portion 4630 can have a first end 4644 that contacts the support pad 4660 and a second end 4646 that contacts the central portion 4610. The arms 4642 can have a horizontal axis that extends from the first end 4644 to the second end 4646. As shown from the top view illustrated in FIG. 39C, the arm 4642 of the bridging portion 4630 can be wider at the second end 4646. The arm 4642 can taper inward as it extends along the horizontal axis toward the first end 4644. However, in some embodiments, the first end 4644 of the bridging portion 4630 that connects to the support pads 4660 can have a thickened region 4632. The thickened region 4632 can be provided with or have a stiffened region of material to provide additional support as shown in FIGS. 39A-39B. FIG. 39A illustrates a front view of the first securement member 4600. As shown in FIGS. 39A-39B, the thickened region 4632 of the first end 4644 can be thicker than the second end 4646 of the arm 4642 when viewed from the front view.

In some embodiments, the cutouts 4640 of the bridging portion 4630 may allow passage of interface tubes, such as the interface tube 4800 through the first securement member 4600 as shown in FIG. 36. In some embodiments, the bridging portion 4630, or any other region of the first securement member 4600 may include a mechanism to further retain the interface tube 4800 to facilitate the management of the interface tube 4800. For example, the bridging portion 4630 may include a hook and loop fastener or an adhesive at the cutout 4640, which may be detachably coupled to the interface tube 4800.

In the illustrated embodiment of FIGS. 39A-39C, the first securement member 4600 includes the opening 4640 formed through the bridging portions 4630. When the user interface patch is applied to the patient facing surface of the support pads 4660, the opening 4640 may expose the user interface patch from the front side opposite the rear-side of the first securement member 4600. Accordingly, if the patient interface patch is connected to the rear side of the support pads 4660 using a double-sided adhesive or a double-sided hook-and-loop fastener, such an adhesive or hook-and-loop fastener will be exposed from the front side opposite the rear side or user-side. In some configurations, such an exposed adhesive or hook-and-loop fastener through the opening 4640 may be used as a tube fastener or otherwise used to organize the interface tubes or conduits.

In some cases, the arms of the bridging portion 4630 may have a recessed region with hook or loop fasteners which can also assist in management of the interface tubes or conduits. In some occasions, it may be desirable that there is some way to hold and manage the interface tubes 4800 of the patient interface assembly 4000. In such arrangements, the tubes 4800 may be flexible enough to promote tube movement and fixation by a clinician or a user. Being able to move the tubes 4800 and fix as desired may allow more comfort for the patient, for example side sleeping.

In some embodiments, the bridging portion 4630 of the first securement member 4600 may have cutout or recessed regions for receiving the tubes 4800 as described herein. The cutout or recessed regions may further include hook or loop material for attaching to a corresponding material on the tubes 4800. In some embodiments, there may be one or more cutout or recessed regions on the bridging portion 4630 or the support pad 4660. The cutout regions may be located at any suitable location along the bridging portion 4630 or the support pad 4660. In some embodiments, there may be a cutout or recessed region having hook or loop on the front side (non-patient facing side) of each support pad 4660 or the bridging portion 4630. In some embodiments, the hook or loop material may be overmoulded onto the bridging portions 4630 or the support pad 4660, or attached by adhesive.

In some embodiments, the rear side and patient facing hook or loop pieces of material may be formed as a single piece to form a double-sided hook or loop material, which can be overmoulded into a cutout or opening in the support pad 4660. The use of the double-sided hook or loop material may be advantageous, since it can be overmoulded more easily to the first securement member 4600 than separate pieces of hook or loop on each side. Further, the support pad 4660 may be more flexible when a single double-sided piece of hook or loop material is used, as opposed to multiple pieces of hook or loop materials, thereby conforming to the user's face better.

The tube 4800 may have a strip of hook or loop material to be attached with a corresponding material on the patient interface assembly 4000. For example, the strip of hook or loop may be wrapped around the outside of the tube 4800 or attached to a part of the tube 4800. In some embodiments, the tube 4800 may include the engagement portion 4840 where the strip of hook or loop or any other attachment mechanism is attached to. The engagement portion 4840 may be wrapped or moulded or placed around the outside of the tube 4800. The piece of loop material may attach to a corresponding hook material on the bridging portions 4630 of the patient interface assembly 4000 to secure the tube 4800. Alternatively, or additionally there may be an overmoulded portion on the tube where part of this portion comprises loop material.

In some embodiments, magnets may be used to hold the conduit or tube 4800 on the patient interface assembly 4000. For example, there may be a magnet on the support pad 4660 or the bridging portion 4630, or placed on a piece of material that wraps around the tube 4800 or overmoulded into the tube 4800. This magnet can interact with a corresponding ferromagnetic material on the other component to secure the tube 4800 and patient interface assembly 4000. Any other suitable detachable coupling mechanisms, such as mechanism described elsewhere herein regarding attachment of the securement members and the interface bodies, may be used to attach the tubes 4800 to the first securement member 4600.

The first securement member 4600 may have a mechanism to be retained by or otherwise coupled to the housing 4400. The first securement member 4600 may be coupled to the housing 4400 by coupling the central portion 4610 to the housing 4400. The first securement member 4600 may be coupled to the housing 4400 by coupling with one or more attachment elements on the housing 4400. For example, the first securement member 4600 may include an opening 4426 for coupling the first securement member 4600 to the housing 4400 at the attachment elements. The opening 4426 may couple to the housing 4400 at the side protrusions 4420. In some embodiments, the first securement member 4600 can be detachably coupled with the housing 4400. The first securement member 4600 may have a mechanism to be retained by or couple with the housing 4400. The opening 4426 may be sized and shaped to be received and retained by or engage with the side protrusion 4420. For example, the opening 4426 may be sized and shaped to be retained at the stem 4424 of the side protrusion 4420.

In some embodiments, the first securement member 4600 can be made of a soft flexible material (e.g. TPE/silicone) or any other material described herein for the securement member. In some embodiments, the first securement member 4600 can have one or more openings or recesses 4426. The first securement member 4600 can have two openings or recesses 4426. The openings or recesses 4426 can be located in a central portion of the first securement member 4600. The openings or recesses 4426 can be pushed over or otherwise engaged with the two side protrusions 4420 of the housing 4400 to secure the first securement member 4600 in place. In some embodiments, the openings 4426 and side protrusions 4420 can allow the first securement member 4600 and the housing to be secured by a push-fit mechanism. The openings 4426 of the first securement member 4600 can be pushed over the enlarged head 4422 of the side protrusion 4420. The inner diameter of the opening 4426 of the first securement member 4600 can sit around the stem 4424 of the side protrusion 4420 when the first securement member 4600 is positioned on the housing 4400. The uniform indentation of the stem 4424 all the way around the side protrusion 4420 can prevent detachment of the first securement member 4600 from the housing 4400.

FIG. 39B illustrates a rear view of the first securement member 4600. As illustrated in FIG. 39B, the first securement member 4600 can include a recess 4428 on the rear side 4645 of the central portion 4610. The recess 4428 can accommodate for the presence of the central protrusion 4440 on the housing 4400 and not increase the bulk of the interface assembly when the first securement member 4600 is in place on the housing 4400. Although two side protrusions are used, the housing 4400 can include only one side protrusion for securing the first securement member 4600. In other cases, more than two side protrusions can be used. For example, the first securement member 4600 can be secured with one or more protrusions on the housing 4400 and corresponding openings on the first securement member 4600.

In some embodiments, the first securement member 4600 may be attached to the housing 4400 through any other mechanism where the side protrusions 4420 can receive and retain or otherwise engage one or more complementary components. For example, the side protrusions 4420 can engage with a hole which fits to the enlarged head of the side protrusion 4420. In some embodiments, the first securement member 4600 may be detachably coupled to the housing 4400. In some embodiments, the first securement member 4600 may be coupled to the housing 4400 with a clip. In some embodiments, the first securement member 4600 may be permanently coupled to the housing 4400. In some embodiments, the first securement member may not have the central portion 4610 and/or the bridging portion 4630 and may include the support pads 4660 directly coupled with the housing 4400 or the interface body detachably or permanently.

In some embodiments, the bridging portion 4630, or the entire first securement member 4600 are made of a flexible material, such that the bridging portion 4630 is flexible or deformable, as described above. In some embodiments, the bridging portion 4630 is made of the same material as the central portion 4610 in an assembled or unitary construction. In some embodiments, the first securement member 4600 may be made of one or more elastomer materials, such as silicone, rubber, polyethylene, or any other flexible material. In some embodiments, the first securement member 4600 may be made of a single material. In some embodiments, the first securement member 4600 may be made of two or more materials, such that a region of the bridging portion 4630 has a different flexibility relative to another region of the first securement member 4600. In some embodiments, at least a region of the bridging portions 4630 may be made of flexible but tensile material, such that the bridging portions 4630 are stiff enough to resist torsion forces and prevent rotation of the central portion with respect to the housing, while being able to hinge or flex to assist to minimize translation of forces and/or movements from the patient's cheeks to the sealing region. In some embodiments, the first securement member 4600 may be constructed from a same material as the interface body (e.g. the cannula body 3200a, the mask body 3200b) and/or the housing 4400.

The first securement member 4600 can have a material thickness that may be defined as the distance from the rear side 4645 of the first securement member 4600 to the front side 4647 of the first securement member 4600. The first securement member 4600 can have different thicknesses at different regions thereof. In some embodiments, the first securement member 4600 can have a thickened region at the bridging portion 4632 where the material thickness at the bridging portion 4632 is greater than the material thickness at other portions of the first securement member 4600. For example, the material thickness at the bridging portion 4632 may be greater than the material thickness at one or more of the support pads 4660, the other portions of the arms of the bridging portion 4630, and/or the central portions 4610 as described previously in relation to FIGS. 39A-39C. This thickened bridging portion 4632 can be located at the arms 4642 that surrounds the cutouts 4640 of the bridging portion 4632 to improve the torsional stability of that region. In some cases, the thickened bridging portion 4632 can be located at the first end 4644 as shown in FIG. 39A.

The torsional stiffness of the first securement member 4600 can resist movement of, and hence disruption of the seal when a force is applied to the interface, e.g. by pulling on tubes or touching of the housing and/or interface body. The torsional stiffness of the first securement member 4600 may assist to resist vertical rotation or movement of the interface body seals, minimizing impact on the seal when a force is applied.

The portion of the first securement member 4600 with or connected to the support pads 4660 may be flexible for attachment of the first securement member 4600 to the patient. The flex of the support pads 4660 can also accommodate the contour of the patient's face.

In some embodiments, the first securement member 4600 can include perforated areas or indications where a clinician may cut off a portion of the support pads 4660 to alter the size of the support pads 4660 e.g. to achieve a smaller size support pad for a smaller patient.

### Second Securement Member

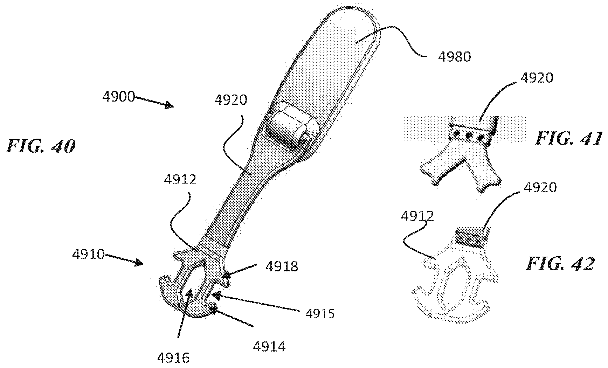

FIGS. 40-42 and 44-47 illustrate embodiments of a second securement member 4900. The second securement member 4900 can include an attachment portion 4910, a support pad 4980 and a central portion 4920 connecting the attachment portion 4910 to the support pad 4980. The support pad 4980 can be a soft connection pad that can rest directly or indirectly on a user's face. For example, the support pad 4980 can be configured to rest at the forehead, when the patient interface 4000 is worn by the user. In some embodiments, the support pad 4980 is a flexible material. For example, the support pad 4980 can be secured to the user's forehead directly or indirectly by an external force, such as using a dermal patch on the patient's face, or any suitable mechanisms such as an adhesive connection, a headgear arrangement, a hook or loop connection as discussed above, or another suitable support structure, such as any of those described herein. In some arrangements, the support pad 4980 can be connected to a headgear and/or a chinstrap for the securement of the interface body.

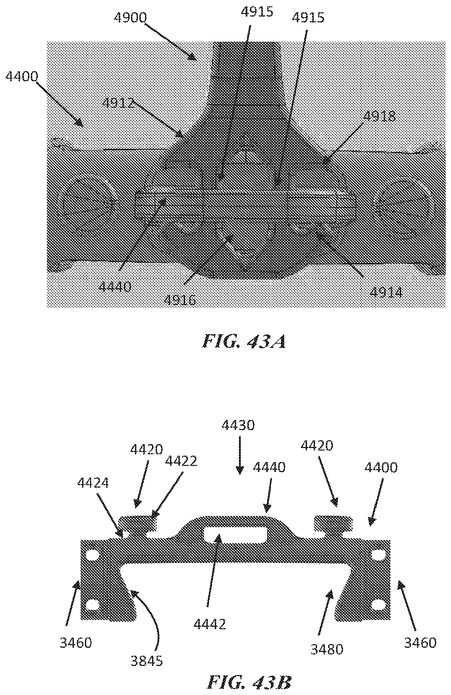

The attachment portion 4910 can be used to attach the second securement member 4900 to the housing 4400. The attachment portion 4910 is formed to couple with a corresponding structure on the housing 4400. In some embodiments, the corresponding structure can be located on the central portion of the housing 4400. The central portion of the housing 4400 can have a central protrusion 4440 such as shown in FIGS. 43A and 43B. The attachment portion 4910 can be formed to couple to an opening 4442 of the central protrusion 4440 (shown in FIGS. 43A and 43B). While the second securement member 4900 described with reference to FIGS. 40-47 is described as coupling to the housing 4400, the second securement member 4900 can be used in combination with any of the housings or patient interface assembly components and embodiments described herein.

In one embodiment, the attachment portion 4910 can be a soft, deformable or flexible material that can be attached or detached from the housing 4400. This may be desired for example, when switching between different interface bodies. The attachment portion 4910 can be a flexible barb 4912. The flexible barb 4912 can have a pair of tabs 4914 on opposing sides of the barb 4912. The pair of tabs 4914 may be configured to catch onto or otherwise engage with the opening 4442 of the housing 4400. The pair of tabs 4914 may be a pair of lower laterally protruding tabs 4914. The flexible barb 4912 can include the pair of lower laterally protruding tabs 4914 and an extended top portion 4918, and a connecting portion 4915 therebetween. The connecting portion 4915 can include a cutout 4916 in the center of the connecting portion 4915. The cutout 4916 can allow for compression or deformation of at least part of the flexible barb 4912 during attachment and detachment. The flexible barb 4912 can be compressed or deformed and the connecting portions 4915 can move inward toward each other. The flexible barb 4912 in the compressed state can be passed through the opening 4442 in the housing 4400. The flexible barb 4912 can then be allowed to relax and the connection portions 4915 move back to their expanded resting position when the laterally protruding tabs 4914 are through the opening 4442. After insertion, the pair of lower laterally protruding tabs 4914 may be located on one side of the central protrusion 4440 and the extended top portion 4918 may be on the other side of the central protrusion 4440 as shown in FIG. 43A.

The flexible material of the flexible barb 4912 can allow for a low insertion force and a strong separation force unless intentionally being removed from where it is attached. The flexible barb 4912 can be compressed and collapsed or otherwise deformed easily to be pushed and fit through the opening 4442 then returning to original configuration when the flexible barb 4912 is through the opening 4442. The cutout 4916 in the center of the flexible barb 4912 promotes this compression and collapsing. In some cases, the attachment portion 4910 can be made of a soft or flexible material, for example, the attachment portion 4910 can be made of TPE or silicone.

FIG. 43A illustrates the interconnection between an embodiment of the second securement member 4900 and the housing 4400. The flexible barb 4912 can fit within the opening 4442 (shown in FIG. 43B) of the central protrusion 4440 of the housing 4400. The flexible barb 4912 can fit within the central protrusion 4440 by each of the laterally protruding tabs 4914 catching onto the underside edges of the central protrusion 4440 of the housing 4400 as shown in FIG. 43. The flexible barb 4912 may collapse or otherwise temporarily deform to fit through the opening 4442. The flexible barb 4912 can be designed to collapse or deform to fit through the opening 4442 and does not have to be an interference fit. In other embodiments, the flexible barb 4912 can be fit with an interference fit. While FIG. 43B does not have a central protrusion with a lip, in some embodiments, the central protrusion 4440 can include a lip similar to the lip 4446 shown in FIG. 38. In such embodiments, the lip can catch the laterally protruding tabs 4914 of a second securement member 4900.

In some embodiments, the flexible barb 4912 can have a low insertion force and strong separation force. The flexible barb 4912 can be easy to disassemble from the housing 4400. The flexible barb 4912 can be removed by moving or pivoting the second securement member 4900 to one side or laterally and pulling the second securement member 4900 and flexible barb 4912 away and out from the housing 4400. In some embodiments, the flexible barb 4912 can be removed by collapsing in a similar but opposing manner to the insertion. In some embodiments, the length, size and/or configuration of the protruding tabs 4914 can be chosen such that when the second securement member 4900 is moved laterally to a certain degree, the tabs 4914 can fold in and allow the second securement member 4900 to be removed. In some embodiments, the protruding tabs 4914 can have a length that is 1.5 mm (about 1.5 mm) long. In some embodiments, the protruding tabs 4914 can have a length that is between 0.5 mm - 10.0 mm, 1.0 -5.0 mm, 1.0 - 2.5, 1.0 - 2.0 mm, 1.5 - 5.0 mm, 1.5 mm - 2.5 mm, or 1.5 mm - 2.0 mm.

The flexible barb 4912 can have an extended top portion 4918 that extends outwardly from the flexible barb 4912. The extended top portion 4918 can prevent the flexible barb 4912 from being over-inserted into the opening 4442 of the housing 4400. The extended top portion 4918 can extend outwardly from the connecting portion 4915 of the flexible barb 4912 and can sit above the opening in the housing 4400 such as shown in FIG. 43. The extended top portion 4918 can prevent over insertion of the second securement member 4900 into the opening 4442. The width of the housing opening 4442 can be less than the width of the extended top portions 4918 measured from the tip of one side of the extended top portion to the tip of the other side of the extended top portion 4918. The width of the housing opening 4442 can be less than the width between the pair of tabs 4914 measured from the tip of one tab 4914 to the tip of the opposing tab 4914.

In some cases, the flexible barb 4912 can be beneficial as it can prevent rigid, sharp, or potentially damaging portions from potentially interacting with the patient. Additionally, the flexible barb 4912 can withstand the compressive forces for assembly and disassemble without breaking.

The central portion 4920 can be a rigid central portion that connects the attachment portion 4910 to the support pad 4980. In some embodiments, the attachment portion 4910 can be integrally formed or molded as one integral piece with the central portion 4910. In other embodiments, the attachment portion 4910 can be formed separately and attached to the central portion 4920. In some embodiments, the attachment portion 4910 can be overmoulded onto central portion 4920 of the second securement member 4900. In some embodiments, the attachment portion 4910 can be attached to the central portion 4920 through crimping or deforming one or both of the portions to join them together. Additionally, in some embodiments, the support pad 4980 can also be overmoulded. In some embodiments, the support pads 4980 can be flexible. In some embodiments, the support pads 4980 can be rigid. The rigid central portion can have a Shore hardness between 30A to 100D. In some embodiments, the attachment portion 4910 and/or a support pad 4980 can have a Shore hardness between 20A - 80A. In some embodiments, the Shore hardness of the central portion 4920 can be the same or greater (or even below) then the Shore hardness of the attachment portion 4910 and/or a support pad 4980.

FIGS. 41 and 42 illustrate other embodiments of the interface between the attachment portion 4910 and the central portion 4920. FIGS. 41 and 42 illustrate joining the attachment portion 4910 and the central portion 4920. In some embodiments, the attachment portion 4910 can be overmoulded onto the central portion 4920. FIG. 41 illustrates the portion of the central portion 4920 that the attachment portion 4910 is overmoulded to. FIG. 42 illustrates the resulting portion of the second securement member after the attachment portion 4910 is overmoulded onto the central portion 4920. Although a particular structure is shown in FIG. 41 extending from the central portion 4920 that the attachment portion is overmoulded to, any structure or geometry can be used.

In some embodiments, the central portion 4920 can work in tension and compression. In some embodiments, the central portion 4920 can prevent over-rotation upwards of the interface body and housing 4400 which can add further added pressure to the nose when compared to a flexible second securement member. In some embodiments, the central portion 4920 can be formed from an optically clear or transparent material which can assist to allow visibility of the patient's face. In some embodiments, the profile of the second securement member 4900 may be shaped to avoid the interface body in use which may give the second securement member 4900 and particularly the central portion 4920 some slight curvature. However, the central portion 4920 can be generally relatively straight. In some embodiments, the central portion 4920 can be rigid. In other cases, the central portion 4920 can be flexible.

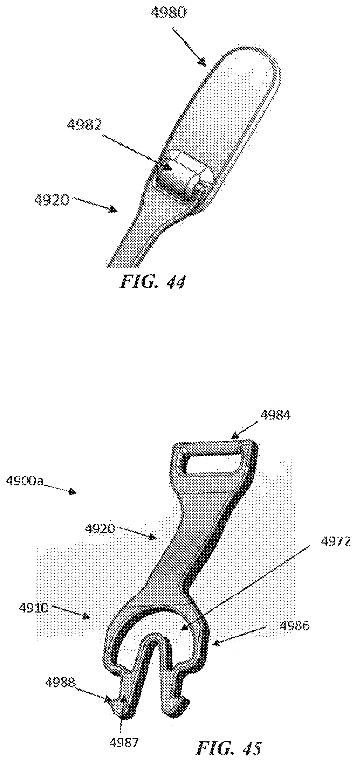

FIG. 44 illustrates an embodiment of the support pad 4980 and the central portion 4920 of the second securement member 4900. The support pad 4980 can be connected to the central portion 4920 with a hinging or pivoting connection 4982. In some embodiments, the central portion 4920 can hinge relative to the attachment portion 4910 due to a difference in material hardness and/or a difference in thickness, or could have a hinge connection similar to the hinged connection 4982 between the support pad 4980 and central portion 4920.

Pivoting of the support pad 4980 relative to the central portion 4920 can help provide the intended vertical stability of the second securement member 4900 by allowing adjustment of the position of the support pad 4980 of the second securement member 4900. This adjustment can be important as a support pad 4980 may connect to a patient at, for example the patient's forehead, or may connect to different devices (for example, headgear) which may locate the support pad 4980 at a different height from the forehead of the patient. The second securement member 4900 can therefore accommodate for these different heights (for example, headgear bulk) and different forehead angles by having the hinging or pivoting connection 4982 of the support pad 4980. The compensation for the different heights and/or forehead angle can assist to minimize impacting the seal of the interface body with the patient. In some embodiments, the support pad 4980 can be flexible. The support pad 4980 can have a means to attach and secure to a headgear, a chinstrap, a patient interface patch or a dermal patch on the patient's face, or otherwise on the patient's face by any suitable mechanisms.

The second securement member 4900 can have a hinge connection between the support pads 4980 and the rest of the second securement member 4900. In some embodiments, the hinging or pivoting connection 4982 can permit a 180 degree pivot, a greater than 180 degree pivot, or any other pivot angle that can allow the second securement member 4900 to be maintained on the patient (and also the rest of the assembly) whilst a caregiver or clinician is tending to a patient or removing/moving the assembly, for example, cleaning the patient's nares. For example, the housing 4400 and interface body can be flipped up and above the support pad 4980 via the pivot or hinge 4982 to move it out of the way. With this configuration, assembly of the housing 4400 and interface body do not have to be fully removed. This can be beneficial because the assembly of the housing 4400 and interface body can be easily flipped up and down. For example, if during care the patient begins to desaturate and requires therapy again, the mask can easily be flipped back down and into place.

In other embodiments, the second securement member 4900 may not have a pivotable connection. The support pad 4980 may instead be integral with the second securement member 4900 as described in relation to FIGS. 33-35. In some embodiments, the support pad 4980 is overmoulded onto the central portion 4920.

FIG. 45 illustrates an embodiment of a second securement member 4900a. The second securement member 4900a can have a pivot or hinge portion handle 4984 for connecting to the support pad (not shown). The second securement member 4900a can include an attachment portion 4910, a support pad (not shown), and a central portion 4920 similar to the second securement member 4900 described in relation to FIGS. 40-44, except as described herein. The attachment portion 4910 can be formed of a rigid material and can have a wide base 4986, a cutout profile 4972 in the wide base 4986, a barbed finger 4987, and a barb catch 4988.

The wide base 4986 can be compressed to bring the barbed fingers 4987 closer together. The cutout profile 4972 of the wide base 4986 can contribute to the deformation of the base 4986 when compressed. The barb catch 4988 can catch on the central protrusion 4440 and/or a lip of the central protrusion 4440 of the housing 4400. The wide base 4986 above the barbed fingers 4987 can be pinched to compress the attachment portion 4910 and to fit the barbed fingers 4987 into the opening 4442 (or openings) of the housing 4400. The attachment portion 4910 can provide tactile feedback to the user.

Similar to the attachment of the second securement member 4900, the second securement member 4900a can be attached by compressing and subsequent expansion of the barb. Once compressed and fit through the opening, the compression force is released and the barbed fingers 4987 can spring out and catch on the central protrusion 4440 of the housing 4400 and remain in place.

To remove the second securement member 4900a a similar mechanism can be used. The attachment portion 4910 can have a low insertion force but high removal force. The removal of the attachment portion 4910 can require the user to actively compress the barb to remove it easily. The second securement member 4900a has an attachment rod 4984 that allows connection of a support pad or any other pad or patch that can be attached to a device for securing the assembly to the patient (such as a headgear or patches) similar to the attachment described in relation to FIG. 44. In some embodiments, the attachment rod 4984 can allow for connection to a dermal patch that can assist to secure the assembly to the patient. In other embodiments, the second securement member 4900a may not have the pivotable connection and the support pad may instead be integral with the second securement member 4900 as described in relation to FIGS. 33-35.

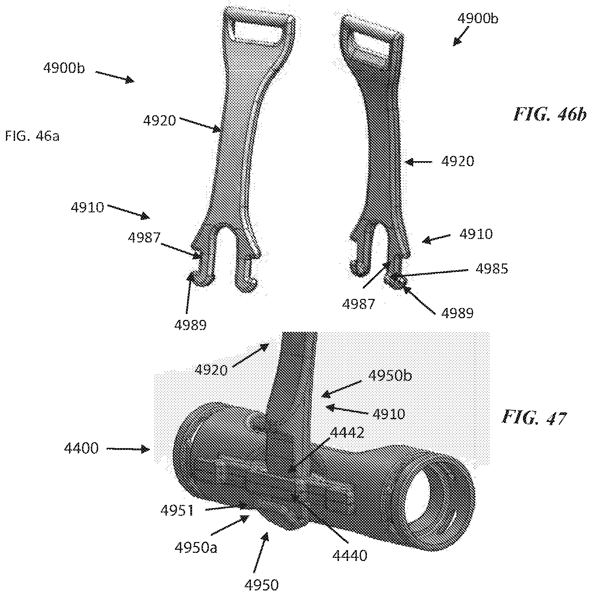

FIGS. 46A and 46B illustrate an embodiment of a second securement member 4900b. FIG. 46A illustrates a front view of a second securement member 4900b. FIG. 46B illustrates a rear view of a second securement member 4900b. The second securement member 4900b can include an attachment portion 4910, a support pad (not shown), and a central portion 4920 similar to the second securement member 4900a described in relation to FIG. 45, except as described herein. The second securement member 4900b can have an attachment portion 4910 with two barbed fingers 4987 and tabs 4989. When pushing the attachment portion 4910 through the opening or openings 4442 in the central protrusion 4440 of the housing 4400, the barbed fingers 4987 can move and compress together. Once received in the opening or openings the tabs 4989 of the barbed fingers 4987 can catch on the side of the central protrusion 4440.

Additionally, second securement member 4900b can have a protrusion 4985 on the rear side of each barbed finger 4987 which can provide an additional catchment which can secure the second securement member 4900b in the central protrusion 4440 of a housing, for example, the housing 4400 described in relation to FIGS. 37-38, by catching on the lip 4446 (shown in FIG. 38).

FIG. 47 illustrates a further embodiment of an attachment portion 4910 of a second securement member 4900b. In this embodiment, attachment portion 4910 includes one or more anterior barb portions 4950. The one or more anterior barb portions 4950 may be located and extend outwardly from a front surface of the attachment portion 4910. A first or lower anterior barb portion 4950a may be located at a lowermost or distal end of the attachment portion 4910. In the embodiment shown, the first anterior barb portion 4950a includes an upwardly extending hook portion 4951. The hook portion 4951 may be flexible or resiliently deformable so that it can be inserted into a corresponding attachment feature on the housing 4400. In the embodiment shown, the hook portion 4951 engages with the opening 4442 in the central protrusion 4440 of the housing 4400.

A second or upper anterior barb portion 4950b may be located on the attachment portion 4910 above the first anterior barb portion 4950a. In the embodiment shown, the second barb portion 4950b includes a protrusion extending outwardly from a front surface of the attachment portion 4910. The protrusion may be configured such that it is unable to pass through the opening 4442. The second barb portion 4950b thus prevents the attachment portion 4910 and hence second securement member 4900 from being over-inserted into the opening 4442.

In some embodiments, the housing may have one opening in the central protrusion at the front side of the housing 4400 (shown in FIG. 43B) for receiving both of the barbed fingers 4987 or may have two openings (as shown in FIGS. 37-38), one for each finger 4987.

The attachment portion 4910 can provide tactile feedback to the user. With tactile feedback of the attachment portion 4910 the user can tell when the attachment portion 4910 is inserted properly within the housing 4400 or under the first securement member 4600. In some cases, the attachment portion 4910 can control the angle of the second securement member 4900 to the seal assembly to the patient.

In some embodiments, the attachment portion 4910 can be rigid. In some embodiments, the attachment portion 4910 can be a flexible material.

The embodiments of the second securement member 4900 described in relation to FIGS. 40-47 can be inserted into the housing 4400 by vertical force, by the user pushing the attachment portion down into the opening of the central protrusion of the housing 4400 until it is received within the opening. This can be preferred over the second securement member 3900 described in relation to FIGS. 33-35 because the second securement member 3900 which attaches to the central protrusion of the housing 3400 with a push-fit attachment could be attached by providing a force that pushes into the patient's face. That force could cause discomfort to the patient thus requiring the assembly to first be removed to attach this second securement member 3900.

### Seal between Housing and Interface Body

The patient interface assembly includes an interface body which can form a seal with at least part of the user's airway and allow gas flow from and to the user, and a housing which can be coupled with the interface body. The housing and the interface body can be coupled in various configurations. The mating of these two components can be optimized for the functionality of the patient interface assembly and/or carer usability. The interface body can be in sealing engagement with the housing to allow the gas flow from and to the user.

In some embodiments, the coupling of the interface body with the housing can be complementary fit as described herein. The coupling of the interface body and housing can allow for assembly and disassembly of the patient interface as well as switching between different interface bodies. The coupling is configured to also assist in preventing unintentional disconnect of the interface body from the housing. The housing and the interface body can be a complementary fit that is difficult to assemble incorrectly and/or provides visual indication of an incorrect and a correct assembly. The interface body can have a manifold 4240 that connects to the interior cavity of the housing 4400 at the tube engaging portion 4460 of the housing 4400.

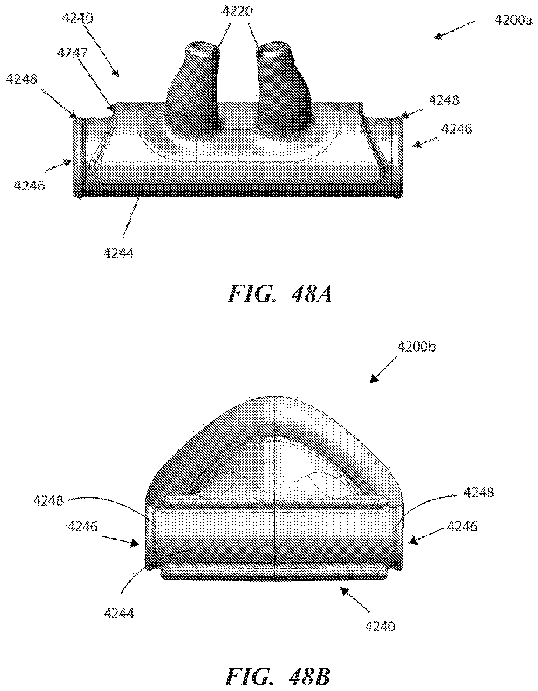

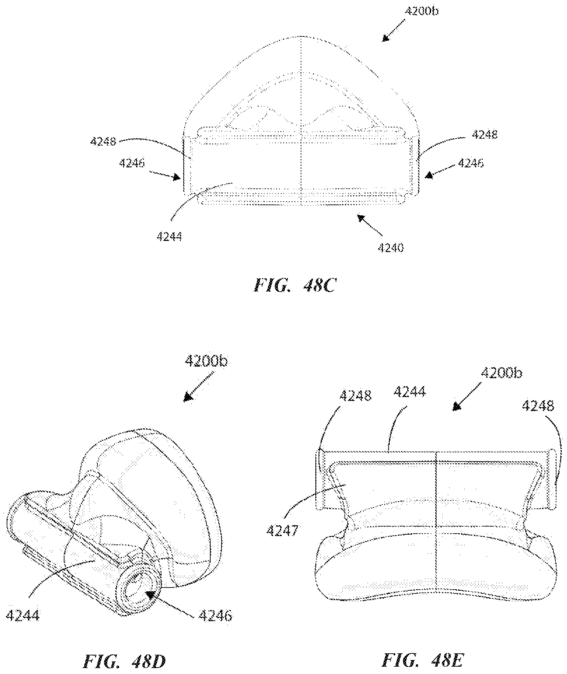

FIG. 48A illustrates an embodiment of a cannula body 4200a interface body. The cannula body 4200a can have a manifold 4240 and at least one nasal prong 4220, such as a pair of prongs 4220. The prongs 4220 can extend from a rear portion of the manifold 4240 facing the patient in use. The cannula body 4200a can be similar to the cannula body described in relation to FIGS. 10-13 and FIG. 21 except as described herein. The manifold 4240 shown in FIGS. 21 and 48A can be similar to the manifold described in relation to FIGS. 10-13, but the manifold 4240 and annular ridges 4248 are positioned closer to the bottom side of the interface body. For example, this can be done by locating the manifold and annular ridges closer to the bottom side of the interface body (as opposed to the center line or substantially the center of the interface body described in relation to FIGS. 10-13). This alignment can help improve the stability of the patient interface.

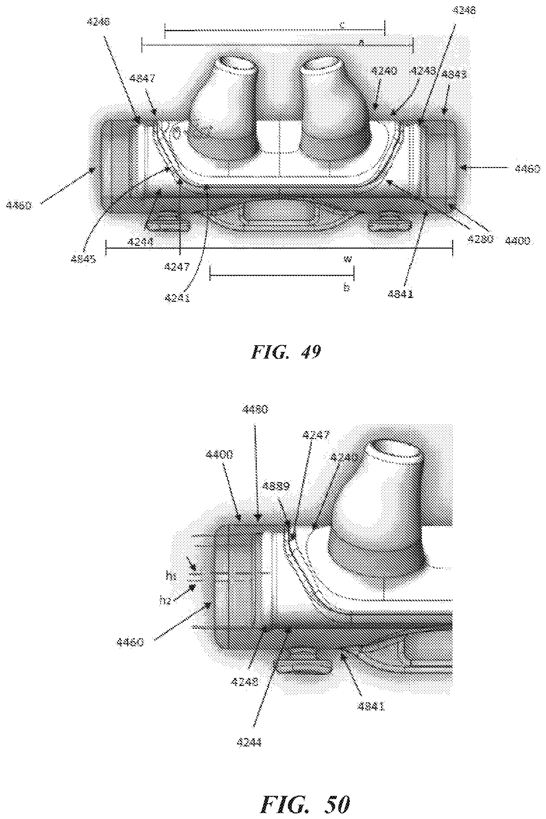

The manifold 4240 may include a housing coupling portion 4244 on the rear side of the manifold 4240. The housing coupling portion 4244 can be received by the interface body receiving portion 4480 (shown in FIGS. 37-38) of the housing. The housing coupling portion 4244 can have a raised portion 4247. The raised portion 4247 may be adjacent the interface body receiving portion 4480 of the housing 4400 when the manifold 4240 and housing are coupled. The housing coupling portion 4244 and the rest of the manifold 4240 may be shaped and/or sized to be inserted in and received by the housing 4400 and form a gas tight seal. For example, the manifold 4240 may include an annular ridge 4248 at each of the openings 4246. The annular ridge 4248 can provide interference with an inner surface of the housing at or adjacent the openings in the housing 4400 forming a gas tight seal with the housing 4400 as shown in FIGS. 49-53D. The annular ridges 4248 of the manifold 4240 are in contact with the inner surface of the housing 4400. All other features of the manifold are in clearance to reduce the fictional forces when assembling the manifold 4240 with in the housing 4400. The lower frictional forces can aid in assembly of the components and decrease the risk of rotational misalignment between the two components.

The interface body and the housing can have an engagement surface 4889 (shown in FIG. 50) where the interface body and the housing meet after assembly. The engagement surface 4889 of the interface body can include the raised portion 4247 of the housing coupling portion 4244 as shown in FIG.49. The raised portion 4247 of the housing coupling portion 4244 can be adjacent the interface body receiving portion 4480 when the housing 4400 and interface body are assembled. The engagement surface 4889 can continue around the entire perimeter of the raised portion 4247. In other embodiments, the engagement surface 4889 can include only a portion of the raised portion.

FIGS. 48B-48E illustrate a mask body 4200b interface body. The mask body 4200b is similar to the mask body 3200b described herein but it includes manifold features described with reference to FIG. 48A.

The manifold 4240 of the cannula body 4200a and the mask body 4200a may define one or more openings 4246 (shown in FIG. 48A-48E), such as a pair of openings 4246. The openings 4246 can be coupled to the tube engaging portion 4460 of the housing 4400 (shown in FIGS. 37-38) to receive the interface tubes, and allow gas flow from and to the patient via the manifold 4240 and the interface body. The positioning of the openings 4246 at both ends of the housing 4400 can create lateral entry points for gas flow to and from the interface. The lateral entry points can assist in clearing CO2 and create less dead space outside of the flow path as described herein with reference to the lateral entry and lateral exit points of the interface body. For example, the lateral flow of gas entering and exiting the interface body, such as a mask cavity of the mask body, can result in greater CO2 clearance when compared to a mask or fluid interface that has an entry and exit point located in the middle or top of the mask. Additionally, the lateral entry and exit points for the flow of gas can reduce dead space outside the flow path in the interface body.

The lateral openings 4246 may be in fluid communication with each other via the manifold4240. When the cannula body is engaged with the housing, except for the openings 4246 and the lumens of the nasal prongs 4220, the manifold 4240 may be sealed when coupled with the housing. When a mask body is engaged with the housing, except for the openings in the manifold and the mask cavity, the manifold may be sealed when coupled with the housing.

As shown in FIG. 49, the housing 4400 may comprise a width, w, that is measured from a first tube engaging portion 4460 on a first end of the housing 4400 to a second tube engaging portion 4460 on the second end of the housing 4400. The interface body can have a width, a, that runs along or is measurable along the lumen of the manifold 4240 and is measured from a first annular ridge 4248 on a first end of the manifold 4240 to a second annular ridge 4248 on a second end of the manifold 4240. In some embodiments, it can be beneficial to reduce the housing profile and minimize overall bulk of the interface. In some embodiments, a single housing size and style can be used for all sizes of cannula bodies or mask bodies. This can allow for a single and constant housing or frame to be used with multiple interface bodies. The interface bodies can be of different types, such as both cannula bodies and mask bodies can be used. The different interface bodies can use different sizes of mask on the different mask bodies or different sizes of nasal prongs on different cannula bodies. This can avoid waste and enable ready changing and adapting of the interface body as required by the clinician.

FIGS. 49-50 illustrates an embodiment of the housing coupling portion 4244 of the manifold 4240 positioned within the interface body receiving portion 4480 of the housing 3400. FIGS. 49-53 show the housing 4400 as transparent which allows the manifold 4240 to be visible within the housing 4400. The housing 4400 and the manifold 4240 can have an engagement surface 4889 where a raised portion 4247 of the housing coupling portion 4244 of the manifold 4240 and the interface body receiving portion 4480 of the housing 4400 mate or abut each other. The housing 4400 can have a width, w, that extends or is measurable from one tube engaging portion 4460 to a second tube engaging portion 4460 as shown by the line "w" in FIG. 49. The interface body receiving portion 4480 can include an opening. The opening can be an angled opening 4845 as shown in one design in FIG. 43B without the manifold attached, and in another design in FIG. 49 with the manifold 4240 located in the housing 4400. The embodiment in FIG. 43D and the embodiment in FIG. 49 have different angled openings that can mate with differently shaped manifolds of the interface body. The angled opening 4845 can include a portion of the housing 4400 that can receive the manifold 4240 of the interface body. The angled opening 3845 can be an angle that extends along the opening in the housing that runs from the front portion 4841 of the housing to the rear portion 4843 of the housing along the top of the housing as shown in FIG. 49. In some embodiments, the housing coupling portion 4244 of the manifold 4240 can have a raised portion 4247 with an angled face that is a complementary fit to the angled opening 4845 of the interface body receiving portion 4480. The angled face of the raised portion 4247 can extend at an angle along the raised portion from the front portion 4241 of the manifold 4240 to the rear portion 4243 of the manifold 4240 along the top of the manifold as shown in FIG. 49. The complementary fit of the angled interface body receiving portion 4480 and angled raised portion 4247 of the housing coupling portion 4244 can improve assembly and alignment when the manifold 4240 is detachably coupled and received by the housing 4400.

In some embodiments, the housing coupling portion 4244 of the interface body and manifold 4240 can have a width measured from one end of the raised portion 4247 of the housing coupling portion 4244 to a second end of the raised portion 4247 of the housing coupling portion 4244 along the lumen of the manifold 4240. When the raised portion 4247 of the housing coupling portion 4244 is an angled raised portion 4247 as shown in FIG. 49, the raised portion 4247 of the housing coupling portion 4244 can have varied widths. For example, the raised portion 4247 of the housing coupling portion 4244 can have a first width, b, on the front portion 4841 of the manifold 4240 and a second width, c, on the rear portion 4843 of the manifold 4240. In some cases, the first width, b, on the front portion of the manifold 4240 is less than the second width, c, on the rear portion of the manifold 4240 as shown in FIG. 49. In some cases, the first width, b, on the front portion of the manifold 4240 is greater than the second width, c, on the rear portion of the manifold 4240.

The angle 4847 of the opening 4845 can be balanced with the width w of the housing. The angled opening 4845 can prevent and discourage incorrect orientation of the manifold 4240 within the housing 4400. As shown in FIG. 49, the angled opening is not a straight edge but instead the shape and configuration of the opening can have curved or trapezoidal sides. In other embodiments, the angled opening can have a straight edge. In other embodiments, the angled opening can have one or more curved edges.

In some embodiments, the angled opening 4845 can help with the alignment and removal of the manifold 4240 from the housing 4400. In some embodiments, a narrow or smaller angle at the angled opening (for example, from 0 to 30 degrees, from 0 to 45 degrees, or from 0 to 60 degrees) could discourage incorrect assembly. A smaller angle could require the width of the housing to be extended due, for example, to the fixed location of the prongs on the interface body. In some embodiments, the angled opening 4845 and the raised portion 4247 of the housing coupling portion 4244 with a larger angle (for example, from 30 to 180 degrees, from 45 to 180 degrees, from 60 to 180 degrees) can allow the housing width to be shorter. A larger angle may, however, lose the advantage of the angle preventing or discouraging incorrect orientation. In some embodiments, at a 90-degree angle, the orientation assistance from the angle can be lost. Therefore, it can be desirable to have the housing 4400 as narrow as possible to improve patient comfort when they are lying on their side, as well as reducing bulkiness and improving aesthetics. However, the width of the housing 4400 is desirably balanced with an angle that can assist to prevent incorrect orientation of the manifold 4240 within the housing 4400. In some embodiments, the raised portion 4247 of the housing coupling portion 4244 can have an angled opening with a 60-degree angle 4847 measured from the rear portion 4843 of the manifold 4240 as shown in FIG. 49. In some embodiments, the angled raised portion 4247 of the housing coupling portion 4244 can extend at an angle of between 30 degrees and 170 degrees from the rear side of the manifold. In some embodiments, the angled raised portion of the housing coupling portion can extend at an angle of between 60 degrees and 120 degrees from the rear side of the manifold. In some embodiments, the angled raised portion of the housing coupling portion can extend at an angle of between 30 degrees and 90 degrees from the rear side of the manifold. In some embodiments, the angled raised portion of the housing coupling portion can extend at an angle of between 90 degrees and 170 degrees from the rear side of the manifold.

FIG. 50 shows a portion of the manifold 4240 coupled to the housing 4400. FIG. 50 shows the housing coupling portion 4244 of the interface body coupled to the interface body receiving portion 4480 of the housing 4400. As shown in FIG. 50 the manifold 4240 is positioned off-center of a horizontal axis, h₂, that runs through the center of the inner lumen of the housing 4400 from a first tube engaging portion 4460 to a second tube engaging portion 4460. This alignment of the manifold 4240 relative to the housing 4400 can allow for a larger manifold internal diameter which can reduce the resistance to flow within the housing 4400 and manifold 4240. This offset arrangement can allow the front portion 4841 of the housing 4400 to be thicker which can increase the stiffness of the housing 4400. The increased thickness of the front portion 4841 of the housing 4400 can improve stability and improves the ease of assembly and/or disassembly of the interface body with the housing 4400. This arrangement can also improve the structural integrity of the housing 4400.

In some embodiments, the manifold 4240 can have a horizontal axis, hi, that extends through the center of the inner lumen of the manifold 4240. The housing 4400 can have a horizontal axis, h₂, that extends through the housing 4400 through the center of the tube engaging portions 4460 on each side of the housing 4400. In some cases, the horizontal axis, hi, can be the same as the horizontal axis, h₂. In other embodiments, the horizontal axis, hi, can be off-set or off-center from the horizontal axis, h₂, of the housing 3400. Therefore, the annular ridge 4248 of the manifold 4240 can be off-center of the horizontal axis h₂. The annular ridge 4248 of the manifold 4240 may be substantially the same size or smaller in size than the inner diameter of the housing 4400.

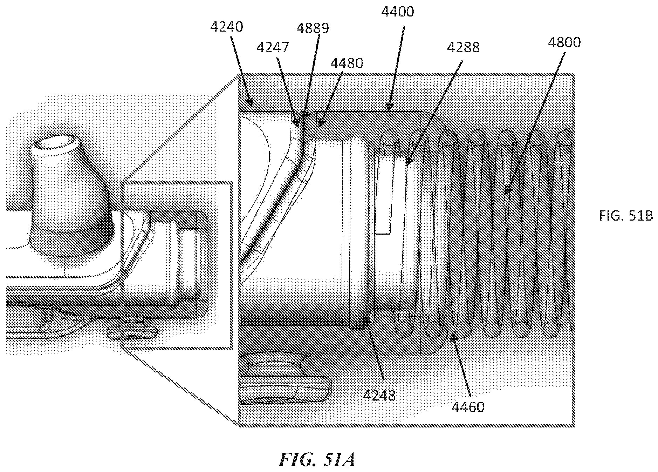

FIGS. 51A-51B show a portion of another embodiment of the manifold 4240 coupled to the housing 4400. FIG. 51B is an enlarged view of an end of the manifold 4240 and the housing 4400 denoted with the box in FIG. 51A. The manifold 3240 can include an extended portion 4288 extending outwardly from each opening 4246 of the manifold 4240. Each extended portion 4288 can extend a distance past the annular ridge 4248. The extended portion 4288 can have a diameter smaller than the outer diameter of the annular ridge 4248. The extended portion 4288 can have an outer diameter that is comparable with the inner diameter of the interface tubes 4800. The extended portions 4288 may be configured in use to extend from the manifold 4240 into the tubing 4800. The extended portions 4288 can extend inside the tube engaging portion 4460 of the housing 4400. In some embodiments, the extended portions 4288 can extend within the interface tubes 4800 of the patient interface assembly. The extended portions 4288 can increase the necessary manifold removal force to remove the manifold from the housing 4400. The extended portions 4288 can reduce the likelihood of the annular ridge 4248 (for example, an annular ridge including an o-ring) catching on the housing 4400 when the user is assembling the two components. When the extended portions 4288 is used, misalignment of the manifold and housing 4400 can be more pronounced and more noticeable.

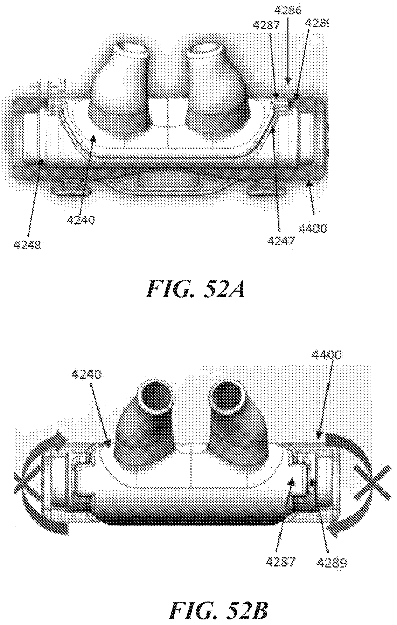

FIGS. 52A-52B show an embodiment of the manifold 4240 coupled to the housing 4400 with a feature designed to prevent or limit rotation of the interface body within the housing. The manifold / housing interface can include a key feature 4286. The key feature 4286 can include a protrusion 4287 on the manifold and an indentation 4289 on the housing. The protrusion 4287 and the indentation 4289 can have a complementary shape so that the protrusion 4287 can mate with the indentation 4289 with a complementary fit as shown in FIGS. 52A-52B. In some embodiments, the key feature 4286 can prevent misalignment between the manifold and the housing 4400. The key feature 4286 can prevent rotation between the two sides of the manifold as shown in FIG. 52B. This can be beneficial as rotation of the manifold can affect the geometry of the prongs or mask. Limiting or preventing the rotation of the manifold can allow for more consistent sealing surface between the manifold and the housing which can improve the ability of the assembly to maintain a seal between the two components.

The key feature 4286 is shown on the rear side (or patient facing side) of the housing 4400. However, the key feature 4286 can be positioned on the front or rear side of the housing 4400 or any location that can allow for a complementary fit between a protrusion and corresponding indentation. Additionally, while the manifold is shown with the protrusion and the housing 4400 is shown with the indentation, a key feature can be used where the protrusion is present on the housing and the indentation is present on the manifold. The key feature shown in FIGS. 52A-52B is rectangular in shape, however, the key feature can be any shape such as a square, rectangular, circular, or any portion of these shapes or irregular shaped.

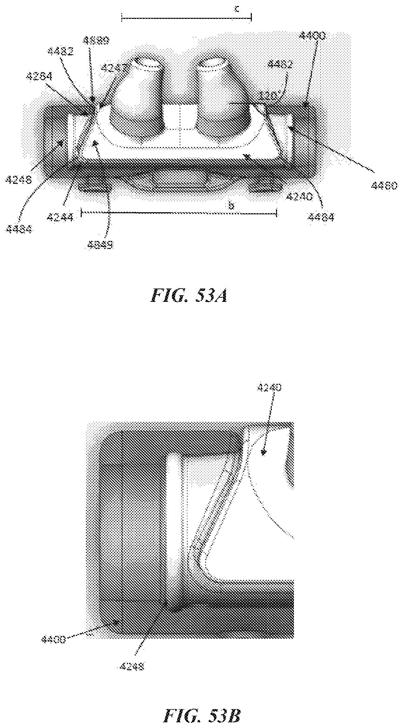

FIGS. 53A-53D illustrate another embodiment of a manifold 4240 coupled to a housing 4400. The raised portion 4247 of the housing coupling portion 4244 of the manifold 4240 can have an angled face 4849 that is a complementary fit to an angled opening 4284 of the interface body receiving portion 4480 of the housing 4400. The angled opening 4284 and raised portion 4247 can be similar to the angled opening described in relation to FIG. 49 except as described herein. The angled opening 4384 of the housing can be a larger angle than the angled opening described in relation to FIG. 49 when measure in the same way. The angled opening 4284 can have a shape and configuration that assists to create a self-aligning geometry with the housing coupling portion 4244 of the manifold 4240. The angled opening 4284 as shown in FIGS. 53A-53B has a similar or same geometry as the angled opening of the interface body receiving portion 3480 of the housing 4400 shown in FIGS. 37 and 38. The angled raised portion 4247 of the housing coupling portion 4244 and angled interface body receiving portion 4480 can control the orientation between the manifold and the housing and reduce likelihood of incorrect placement of the manifold in the housing. The angled raised portion 4247 of the housing coupling portion 4244 and angled interface body receiving portion 4480 can also reduce the chance of rotational misalignment between the manifold and the housing.

The angled raised portion 4247 of the manifold can be a substantially trapezoidal configuration when viewed from the top view of the housing 4400 and manifold 4240 as shown in the top view in FIG. 53A. As shown in FIG. 53A, the trapezoidal configuration of the angled raised portion 4247 is such that a wider base of the trapezoid is proximal the front portion of the housing 4400. In other embodiments, the raised portion of the interface body comprises a wider base of the trapezoid proximal to the rear portion of the housing.

The housing 4400 can have an angled interface body receiving portion 4480 with a complementary configuration, as shown in FIGS. 37, 37, and 53A. As illustrated in the top view of the interface assembly in FIG. 53A, the angled raised portion 4247 can be a trapezoidal shape when viewed from the top. The trapezoidal shape outlined by the angled raised portion 4247 of the housing coupling portion 4244 and angled interface body receiving portion 4480 when viewed from the top view as illustrated in FIG. 53A can have four corners with angles other than 90 degrees.

The angled raised portion 4247 of the housing coupling portion 4244 and angled interface body receiving portion 4480 as illustrated in FIG. 53A can have corners 4482 with angles greater than 90 degrees in the two corners 4482 formed by the rear side of the manifold. The angled raised portion 4247 of the housing coupling portion 4244 and angled interface body receiving portion 4480 as illustrated in FIG. 53A can have corners 4484 with angles of less than 90 degrees in the corners 4484 on the front side of the raised portion of the manifold and interface body receiving portion of the housing. For example, the angled raised portion 4247 of the housing coupling portion 4244 and angled interface body receiving portion 4480 as illustrated in FIG. 53A can have a 120-degree angle at the two opposing corners on the rear side of the manifold.

As shown in FIGS. 53A and 53B, the angle 4482 of the angled raised portion 4247 has an angle greater than 90 degrees when measured from the rear portion of the manifold. The angle illustrated in the embodiment of the housing 4400 shown in FIG. 49 has an angle less than 90 degrees when measured from the rear portion of the manifold. However, any angled opening can also be used to allow assessment of correct attachment and prevent rotational misalignment of the components. In some embodiments, the angled raised portion 4247 of the housing coupling portion 4244 can extend at an angle of between 30 degrees and 170 degrees from the rear side of the manifold. In some embodiments, the angled raised portion of the housing coupling portion can extend at an angle of between 60 degrees and 120 degrees from the rear side of the manifold. In some embodiments, the angled raised portion of the housing coupling portion can extend at an angle of between 90 degrees and 120 degrees from the rear side of the manifold. In some embodiments, the angled raised portion of the housing coupling portion can extend at an angle of between 90 degrees and 170 degrees from the rear side of the manifold.

The manifold 4240 can be folded, flexed, bent or otherwise suitably manipulated while being inserted into the housing 4400. For example, upon insertion, the manifold 4240 can be folded through the center of the housing 4400 in order to manipulate the annular ridge 4248 through the narrowest point of the housing 4400. Once the annular ridges 4248 are concentrically located in the housing, the manifold can straighten and return to the resting shape. During this process, the angled faces 4849 and angled openings 4284 can act to align the manifold 4240 in the correct orientation relative to the housing 4400.

FIG. 53B shows a portion of the manifold 4240 and housing 4400 shown in FIG. 53A. The annular ridge 4248 of the manifold 4240 can be in contact with the housing 4400. The annular ridge 4248 can be in contact with an inner surface of the housing 4400 to create a gas seal. Other features on the manifold 4240 can avoid or have minimal contact with the housing in order to reduce the frictional forces when assembling the two components. Lower frictional forces can aid assembly for the user and decrease the risk of rotational misalignment between the two components. In some cases, the annular ridge 4248 can have an interference fit to prevent leaks.

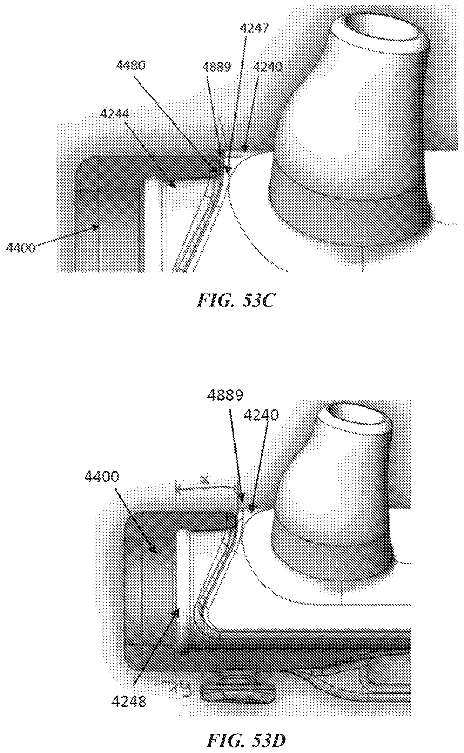

FIG. 53C shows a portion of the manifold 4240 and housing 4400 shown in FIG. 53A. The manifold 4240 of the interface body and the housing 4400 can each have an engagement surface 4889 where the manifold 4240 and the housing 4400 meet or are otherwise contiguous when assembled. A side wall of the raised portion 3247 of the housing coupling portion 3244 can form at least part of the engagement surface 4889 of the interface body. A portion of the raised portion 4247 on the manifold 4240 can be higher relative to the engagement surface 4889 on the housing 4400. This arrangement results in a portion of the raised portion 4247 of the manifold 4240 remaining clear of contact with the housing as shown in FIG. 53C. For example, as illustrated in FIG. 53C, the raised portion 4247 of the rear side of the manifold 4240 is extended beyond the rear side of the housing 4400 at the engagement surface 4889. This difference in rearward (or patient facing) extension of these components can allow the relatively softer manifold material of the interface body to contact the patient's face before or preferentially to the relatively harder or more rigid housing material.

FIG. 53D shows a portion of the manifold 4240 and housing 4400 in an assembled configuration. Disassembly of the manifold 4240 from the housing 4400 can be managed or optimized at least partly based on the materials and/or configurations of the manifold and housing. The disassembly force required to disassemble the components can be at least partly controlled by distance "x" as shown in FIG. 53D, this being a horizontal distance between outer edge of annular ridge 4248 and sealing surface or engagement surface 4889 of the manifold 4240. Disassembly of the manifold 4240 from the housing 4400 can occur when the annular ridge 4248 is moved past the engagement surface 4889 of the housing 4400, unseating the manifold 4240 from the interface body receiving portion 4480 of the housing. The user can overcome this force to swap between interface bodies, for example, between masks, prongs, or between different sizes.

In some embodiments, the seal between the manifold 4240 and the housing 4400 can be broken when the annular ridge 4248 moves beyond distance "y" as shown on FIGS. 53D, distance "y" being a distance between outer edge of annular ridge 4248 and raised portion 4247 of the manifold. With the annular ridge 4248 at or past this point, there can be a clear flow path between the housing and the annular ridge 4248, leading to a leak which is likely unacceptable for delivery of therapy. In some embodiments, the angled opening can affect both of these dimensions ("x" and "y"), as can the overall width of the housing.

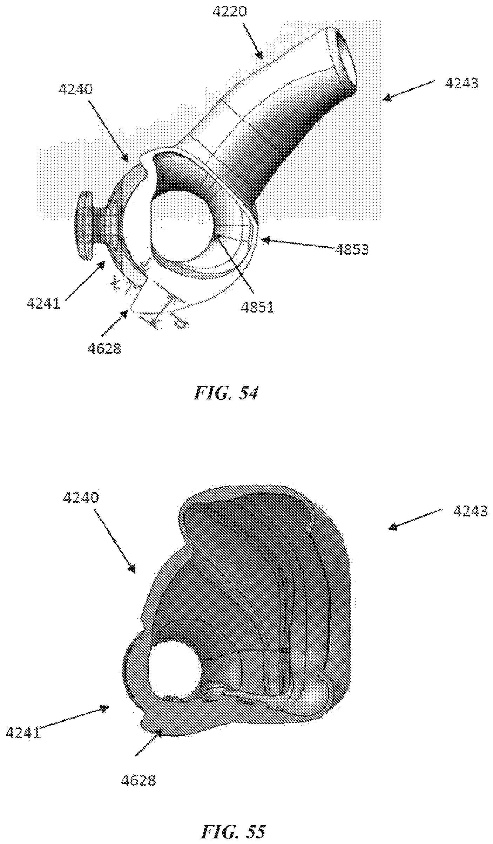

FIGS. 54-55 illustrate a side cross section view of the interface body. FIG. 54 illustrates a side cross section view of the interface body with a cannula body. FIG. 55 illustrates a side cross section view of the interface body with a mask body. The manifold 4240 and cannula body and mask body are similar to the manifold and cannula body and mask body described in relation to FIGS. 10-24 except as described herein. The manifold 4240 can have an inner diameter of the inner surface 4851 of wall of the manifold and an outer diameter of the outer surface 4853 of the wall of the manifold and a wall thickness therebetween. The rear portion 4243 or patient facing portion of the manifold 4240 can have a wall thickness that is thinner compared to the wall thickness on the front portion 4241 of the manifold 4240. The thinner wall thickness can allow for more flexibility which can be more comfortable for the patient. The wall thickness of the front portion 4241 of the manifold 4240 that is received by the housing can be generally thicker compared with the rest of the manifold 4240. This can help with assembly and removal of an interface body from the housing by a user or clinician. For example, when the interface assembly is assembled or the interface body is removed, the interface body can fold inwards when forces are applied on the sides of the interface body. This folding is at least partly enabled from the wall thickness on the rear portion 4243 of the manifold 4240 being thinner than the front portion 4241 of the manifold 4240. The thinner material can collapse inwards when force is applied to the or each side of the manifold 4240 during insertion and removal of the interface body.

The manifolds 4240 of both the mask body and cannula body have annular ridges 4248 on both sides of the manifold 4240. The annular ridges 4248 can be an integral sealing o-ring portion. These annular ridges 4248 of the manifold 4240 can fit in the housing 4400 and seal with the housing 4400 so that either side is respectively in fluid communication with one of the tubes. The annular ridges 4248 are the intended point of contact between the manifold 3240 and the housing 4400. Having a single point of contact can reduce friction of the manifold 4240 with the housing 4400 so that the interface body can slide in easier when fitting to the housing and self-aligns easier. The amount of rotation of each side of the interface body or each annular ridge relative to each other can be controlled by the amount of interference between the components (for example, the amount of interference between the housing 4400 and manifold 4240). This rotation (and thus the interference) is ideally minimized because a small amount of rotation of one side can distort the seal between the housing 4400 and manifold 4240. Therefore, it can be important to assure and confirm proper alignment of the manifold 4240 within the housing 4400. The manifold 4240 can include a self-aligning feature to help to prevent this rotation and maintain each side of the manifold 4240 in the same location in the housing 4400 relative to the other.

FIGS. 54-55 illustrate a manifold with a feature to prevent or at least minimize rotation of the interface body within the housing. In some cases, the feature can be a shoulder portion 4628. The shoulder portion 4628 can be any structure that has a surface large enough to engage with the bottom edge of the opening of the housing (not shown) and prevent or reduce rotation. For example, the shoulder portion 4628 can be a thickened portion of the wall of the manifold 4240 as shown in FIGS. 54 and 55. The shoulder portion 4628 can help to self-align the manifold 4240 within the housing. As shown in FIGS. 54 and 55, the shoulder portion 4628 can be a thickened portion at the bottom of the housing attachment portion of the manifold. Once assembled, the shoulder portion 4628 can overhang the housing. In other cases, the shoulder portion does not overhang the housing but can abut the surface of the opening in the housing. The shoulder portion 4628 catches and abuts on the edge of the housing and may prevent or limit rotation and movement of the interface body relative to the housing. Limiting or preventing the rotation of the manifold can allow for more consistent sealing surface between the manifold and the housing which can improve the ability to maintain a seal between the two components. The shoulder portion 4628 can be a thickened portion of the manifold that can help to prevent insertion of the interface body into the housing incorrectly. The shoulder portion 4628 can allow for visual assessment of the incorrect or correct configuration of the interface body within the housing. The user could visually observe that the manifold is misaligned and correct the alignment of the manifold with the housing.

As shown in FIG. 54, a distance "d" illustrates the width at the shoulder portion 4628 which is wider than the remainder of the manifold wall. The thickness "t" of the manifold wall is illustrated in FIG. 54 and is thinner than the distance "d" of the width of the area of the manifold with the shoulder portion 4628. In some embodiments, the width "d" of the shoulder portion can be 2.5 mm and the wall thickness of the manifold "t" is 1.0 mm. The width "d" of the shoulder portion can be between 1.5 mm to 3.5 mm, 1.5 mm to 2.5 mm thick, 2.5 mm to 3.5 mm thick, and 2.0 mm to 3.0 mm.

The width at the shoulder portion 4628 can create a region at the base of the interface body that can act to prevent rotation of the manifold within the housing once assembled. In some embodiments, the shoulder portion 4628 only extends along a portion of the manifold. In some embodiments, the shoulder portion 4628 can extend the width of the manifold from one side of the manifold to the other. The shoulder portion 4628 can also give the nasal prongs or mask some structure or stiffness which can assist with the insertion of the device as described previously. Additionally, the increased thickness at this shoulder portion 4628 can also increase the necessary removal force thereby reducing the chance of accidental disassembly.

In some embodiments, the surface finish of the material of the housing and interface body or manifold can assist with the manifold's placement within the housing and thus improve fitting of the manifold to the housing. In some embodiments, to achieve this, the surface finish of the housing and manifold or interface body can result in a low friction or slippery surface. In some embodiments, the low friction surface of the housing or interface body can be a characteristic of the material that forms the housing or interface body or a portion of the housing or interface body. In some embodiments, the low friction surface can be achieved during the manufacturing process. For example, a material coating can be added to the material of the housing or interface body. For example, a parylene coating can be applied to the external surfaces of the housing or interface body to achieve a low friction surface. Alternatively, the surface can be treated by a process, such as a blasting process (e.g. bead blasting) to provide the low friction surface. The coating or treatment can reduce the friction and adherence of the housing or interface body to other materials which can improve sliding the interface body into the housing. Furthermore, the low friction surface also reduces friction of the parts of the interface body which contact the patients face and can improve comfort. In some embodiments, other components of the patient interface assembly can also have the low friction surface or be formed from a low friction material.

FIGS. 56A-56F illustrate a manifold 5420 coupled to a housing 5400 that use a visual indicator for indicating correct positioning and proper alignment of the components. The visual indicator can be a color indicator or indicator lines which indicate the correct placement of components.

FIGS. 56A-56D illustrate the visual indicator can be a colored ring 5604 located on or around the annular ridge of the manifold 5420. The colored ring 5604 can be observed through a transparent or translucent portion of the housing 5400. A user or clinician may observe the position and configuration of the colored ring 5604 through the transparent or translucent portion. In the embodiment shown, correct positioning of the components is indicated by observing that the colored ring 5604 is in axial alignment with the housing 5400 and forms a circle. An incorrect positioning of the components is indicated by observing, for example, that the colored ring 5604 is deformed or out of alignment with the housing 5400.

The colored ring 5604 can be provided on both opposing annular ridges of the manifold. In some embodiments, the color indicator can be provided only on the manifold, only on the housing, or on both the manifold and the housing.

Additionally or alternatively, the interface assembly may include a second visual indicator, such as a second color indicator, on an angled corner of either the housing 5400 and/or the manifold 5420. FIGS. 56C-56D illustrate the color indicator 5606 at the angled edge of the manifold 5420 and housing 5400. The color indicator 5606 can provide a visual indication of when the angled edge of these two components are aligned correctly. When the two components are not aligned correctly the user can see the misalignment of the color indicator on the components.

A further embodiment of visual indicator is shown in FIGS. 56E-56F. In this embodiment, the visual indicator is provided as a colored or contrasting bar or line 5608. The colored bar 5608 can be located on a surface of the interface body manifold 5420. In the embodiment shown, the colored bar 5608 is located on the manifold 5420 at or adjacent a surface or edge that mates or abuts the housing 5400. If the manifold 5240 and housing 5400 are incorrectly aligned, the colored bar 5608 can be observed to have a non-linear configuration as shown in FIG. 56E. The colored bar 5608 may observed to have a linear or straight configuration if the components are correctly assembled and aligned, such as shown in FIG. 56F.

In some embodiments, the color indicator on the housing and/or manifold can be used as a sizing indicator as well as an alignment indicator. For example, the housing and manifold can be provided in various sizes and different sizes can use different color indicators so that the correct size housing could be paired with the correct size manifold. This could assist the user in correctly pairing the housing and manifold components. While the color indicators illustrated with reference to FIGS. 56A-56F are provided on the ends of the manifold where it mates with the housing or the angled edge of the manifold and the housing, the color indicator can be provided on any component of the manifold and/or housing, such as any portion of the manifold that mates with the housing to provide a visual indication of the correct positioning and alignment. In some embodiments, any portion or component or the entirety of the components of the interface assembly can be transparent or translucent to allow visibility of the visual alignment indicator. The transparent or translucent material can allow the colored portion of the components to be visible, for example, the transparent or translucent material can allow visibility of the colored portion on the annular ridge of the manifold within the housing.

### Tubing and Connector

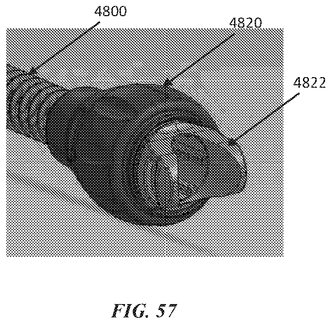

The one or more interface tubes and connectors of the patient interface assembly allows fluid communication to and from the patient from an external device or gas flow source. FIG. 57 illustrates tubing 4800 and a connector 4820 at the end of the tubing. In some embodiments, the walls of the interface tubing 4800 can be made of a material that can allow for the passage of moisture vapor through the walls of the tubing 4800. The connector 4820 can be any type of interlocking or mating connector to couple the tubing to another device or to additional tubing. As shown in FIG. 57 the connector can have locking fingers 4822 to connect and secure the tubing to another device or an additional tubing. Example of the connector and/or the connector assembly can be found in International Application No. PCT/NZ2012/000142, filed August 10, 2012 entitled CONDUIT CONNECTOR FOR A PATIENT BREATHING DEVICE. In other embodiments, the connector can have a tapered fit to connect to another device or an additional tubing.

The housing can connect to the tubing at the tube engaging portion (shown in FIG. 36). In some embodiments, the tubing 4800 can include an inspiratory conduit with a first end that can connect with the housing and deliver incoming flow of gases and a second end with locking fingers 4822. In some embodiments, the tubing 4800 can include an expiratory conduit with a first end that can connect with a housing and receive flow of expiratory gases and a second end with locking fingers 4822. In some embodiments, the tubing can include both an inspiratory conduit and an expiratory conduit. In some cases, the locking fingers 4822 at the second end can be coupled to a downstream component, for example, a bubbler. In some embodiments, the locking fingers 4822 at the second end of the inspiratory conduit can be coupled to an upstream component such as a flow source.

In some embodiments, the locking fingers 4822 can extend away from the connector 4820. The locking fingers 4822 can be spaced apart and narrowing along their length away from the connector. The locking fingers can have locking recesses that are formed at least on outer surfaces of each of the locking fingers. The locking recesses can lock with portions of a gas delivery tube connector or other connector. The locking fingers can interact with recesses of the gas delivery tube connector or other connector to align the connector 4820 and the gas delivery tube connector or other connector.

### Terminology

Unless the context clearly requires otherwise, throughout the description and the claims, the words "comprise", "comprising", and the like, are to be construed in an inclusive sense as opposed to an exclusive or exhaustive sense, that is to say, in the sense of "including, but not limited to". Conditional language used herein, such as, among others, "can," "could," "might," "may," "*e*.*g*.," and the like, unless specifically stated otherwise, or otherwise understood within the context as used, is generally intended to convey that certain embodiments include, while other embodiments do not include, certain features, elements and/or states. Thus, such conditional language is not generally intended to imply that features, elements and/or states are in any way required for one or more embodiments or that one or more embodiments necessarily include logic for deciding, with or without author input or prompting, whether these features, elements and/or states are included or are to be performed in any particular embodiment.

The term "plurality" refers to two or more of an item. Recitations of quantities, dimensions, sizes, formulations, parameters, shapes and other characteristics should be construed as if the term "about" or "approximately" precedes the quantity, dimension, size, formulation, parameter, shape or other characteristic. The terms "about" or "approximately" mean that quantities, dimensions, sizes, formulations, parameters, shapes and other characteristics need not be exact, but may be approximated and/or larger or smaller, as desired, reflecting acceptable tolerances, conversion factors, rounding off, measurement error and the like and other factors known to those of skill in the art. Recitations of quantities, dimensions, sizes, formulations, parameters, shapes and other characteristics should also be construed as if the term "substantially" precedes the quantity, dimension, size, formulation, parameter, shape or other characteristic. The terms "approximately," "about," and "substantially" as used herein represent an amount close to the stated amount that still performs a desired function or achieves a desired result. For example, in some embodiments, as the context may dictate, the terms "approximately", "about", and "substantially" may refer to an amount that is within less than or equal to 10% of the stated amount. The term "generally" as used herein represents a value, amount, or characteristic that predominantly includes, or tends toward, a particular value, amount, or characteristic. For example, as the context may dictate, the term "generally linear" can mean something that departs from exactly parallel by less than or equal to 15°.

Numerical data may be expressed or presented herein in a range format. It is to be understood that such a range format is used merely for convenience and brevity and thus should be interpreted flexibly to include not only the numerical values explicitly recited as the limits of the range, but also interpreted to include all of the individual numerical values or sub-ranges encompassed within that range as if each numerical value and sub-range is explicitly recited. As an illustration, a numerical range of "1 to 5" should be interpreted to include not only the explicitly recited values of about 1 to about 5, but should also be interpreted to also include individual values and sub-ranges within the indicated range. Thus, included in this numerical range are individual values such as 2, 3 and 4 and sub-ranges such as "1 to 3," "2 to 4" and "3 to 5," etc. This same principle applies to ranges reciting only one numerical value (e.g., "greater than 1") and should apply regardless of the breadth of the range or the characteristics being described.

A plurality of items may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary. Furthermore, where the terms "and" and "or" are used in conjunction with a list of items, they are to be interpreted broadly, in that any one or more of the listed items may be used alone or in combination with other listed items. The term "alternatively" refers to selection of one of two or more alternatives, and is not intended to limit the selection to only those listed alternatives or to only one of the listed alternatives at a time, unless the context clearly indicates otherwise.

Reference to any prior art in this specification is not, and should not be taken as, an acknowledgement or any form of suggestion that that prior art forms part of the common general knowledge in the field of endeavor in any country in the world.

The invention may also be said broadly to consist in the parts, elements and features referred to or indicated in the specification of the application, individually or collectively, in any or all combinations of two or more of said parts, elements or features.

Where, in the foregoing description reference has been made to integers or components having known equivalents thereof, those integers are herein incorporated as if individually set forth.

It should be noted that various changes and modifications to the presently preferred embodiments described herein will be apparent to those skilled in the art. Such changes and modifications may be made without departing from the scope of the invention and without diminishing its attendant advantages. For instance, various components may be repositioned as desired. It is therefore intended that such changes and modifications be included within the scope of the invention. Moreover, not all of the features, aspects and advantages are necessarily required to practice the present invention. Accordingly, the scope of the present invention is intended to be defined only by the claims that follow.

## Claims

1. A patient interface assembly (3000, 4000) comprising:
a first interface body (3200a, 4200);
a second interface body (3200b, 4200);
a housing (3400, 4400, 5400) configured to detachably couple with the first interface body (3200a, 4200a) or the second interface body (3200b, 4200), comprising
a pair of tube engaging portions (3460) at lateral entry points on opposite ends of the housing; and
a pair of interface tubes (3800, 4800), each of the interface tubes (3800,4800) at one end being coupled to one tube engaging portion of the pair of tube engaging portions (3460), and allow gas from and to the user to flow through the respective first interface body (3200a, 4200) or second interface body (3200b, 4200) when detachably coupled to the housing (3400, 4400, 5400),
wherein the first interface body (3200a, 4200) and the second interface body (3200b, 4200) are interchangeable, such that the first interface body (3200a, 4200) is coupled to the housing (3400, 4400, 5400) in a first configuration, and the second interface body (3200b, 4200) is coupled to the housing (3400, 4400, 5400) in a second configuration,
wherein each of the first interface body (3200a, 4200a) and the second interface body (3200b, 4200b) comprises a manifold (3240, 4240, 5240), the manifold (3240, 4240, 5240) configured to be at least partially received by the housing (3400, 4400, 5400) when the first interface body (3200a, 4200a) or the second interface body (3200b, 4200b) is detachably coupled to the housing (3400, 4400, 5400), the manifold (3240, 4240, 5240) comprising a first opening (3246, 4246) and a second opening (3246, 4246) in communication with the interface tubes when the first interface body (3200a, 4200) or the second interface body (3200b, 4200) is detachably coupled to the housing (3400, 4400, 5400) and wherein the positioning of the first and second openings (3246, 4246) defines lateral entry points for gas flow to and from each of the first interface body (3200, 4200) and the second interface body (3200, 4200).

2. The patient interface assembly (3000, 4000, 5400) of claim 1, wherein the first and second openings (3246, 4246) are in fluid communication with each other via the manifold (3240, 4240, 5240).

3. The patient interface assembly (3000, 4000, 5400) of claim 1 or 2, wherein a first interface tube (3800, 4800) of the pair of interface tubes (8800, 4800) comprises or is configured for connection to an inspiratory conduit (201a) of a respiratory system, and a second interface tube (8800, 4800) of the pair of interface tubes (8800, 4800) comprises or is configured for connection to an expiratory conduit (201b) of the respiratory system..

4. The patient interface assembly (3000, 4000) of any one of the preceding claims, wherein the first and second openings (3246, 4246) are on opposing sides of the manifold (3240, 4240, 5240).

5. The patient interface assembly (3000, 4000) of any one of the preceding claims, wherein the first interface body (3200a, 4200) is a cannula body (3200a, 4200a) and/or wherein the second interface body (3200b, 4200) is a mask body (3200b, 4200b).

6. The patient interface assembly (3000, 4000) of any one of the preceding claims, wherein each of the first interface body (3200a, 4200) and the second interface body (3200b, 4200) are configured to form a seal with a user's face.

7. The patient interface assembly (3000, 4000) of any one of the preceding claims, wherein an upper edge (3470) and a lower edge (3490) of the housing (3400, 4400, 5400) are symmetrical.

8. The patient interface assembly (3000, 4000) of any one of the preceding claims, wherein the manifold (3240, 4240, 5240) comprises a housing coupling portion (3244, 4244) configured to be received within the housing (3400, 4400, 5400) when the first interface body (3200a, 4200) or the second interface body (3200b, 4200) is detachably coupled to the housing (3400, 4400, 5400).

9. The patient interface assembly (3000, 4000) of claim 8, wherein the manifold (3240, 4240, 5240) further comprises a raised portion (3242, 4247) adjacent the housing coupling portion (3244, 4244), wherein the raised portion (3242, 4247) is configured to inhibit rotation of the manifold (3240, 4240, 5240) about the housing (3400, 4400, 5400) when the first interface body (3200a, 4200) or the second interface body (3200b, 4200) is detachably coupled to the housing (3400, 4400, 5400).

10. The patient interface assembly (3000, 4000) of claim 8 or claim 9, wherein the housing coupling portion (3244, 4244) and the manifold (3240, 4240, 5240) are shaped and/or sized to be inserted in and received by the housing (3400, 4400) and form a gas tight seal with the housing (3400, 4400).

11. The patient interface assembly (3000, 4000) of any one of claims 8 to 10, wherein the housing coupling portion (3244, 4244) comprises a trapezoidal shape defined between a first patient facing side and an opposite second side of the housing coupling portion (3244, 4244), and the housing comprises a complementary shape to the trapezoidal shape of the housing coupling portion (3244, 4244).

12. The patient interface assembly (3000, 4000) of any one of the preceding claims, wherein the manifold (3240, 4240, 5240) comprises a ridge (3248, 4248) at each of the first and second openings (3246, 4246) that is configured to form a gas tight seal with the housing (3400, 4400).

13. The patient interface assembly (3000, 4000) of claim 12, wherein the ridge is an annular ridge (3248, 4248).

14. The patient interface assembly (3000, 4000) of any one of the preceding claims, wherein the manifold (3240, 4240, 5240) comprises a visual indicator for indicating correct positioning and proper alignment with the housing.

15. The patient interface assembly (3000, 4000) of claim 14 when dependent on claim 12 or 13, wherein the visual indicator comprises a colored ring (5604) located on or around the ridge (3248, 4248) of the manifold (3240, 4240, 5240).

## Patentansprüche

1. Patientenschnittstellenanordnung (3000, 4000), umfassend:
einen ersten Schnittstellenkörper (3200a, 4200);
einen zweiten Schnittstellenkörper (3200b, 4200);
ein Gehäuse (3400, 4400, 5400), das konfiguriert ist, um mit dem ersten Schnittstellenkörper (3200a, 4200a) oder dem zweiten Schnittstellenkörper (3200b, 4200) lösbar gekoppelt zu werden, umfassend
ein Paar von Schlaucheingriffsabschnitten (3460) an lateralen Eintrittspunkten auf gegenüberliegenden Enden des Gehäuses; und
ein Paar von Schnittstellenschläuchen (3800, 4800), wobei jeder der Schnittstellenschläuche (3800, 4800) an einem Ende mit einen Schlaucheingriffsabschnitt des Paares von Schlaucheingriffsabschnitten (3460) gekoppelt ist und zulässt, dass Gas von und zu dem Nutzer durch den jeweiligen ersten Schnittstellenkörper (3200a, 4200) oder zweiten Schnittstellenkörper (3200b, 4200) strömt, wenn sie mit dem Gehäuse (3400, 4400, 5400) lösbar gekoppelt sind,
wobei der erste Schnittstellenkörper (3200a, 4200) und der zweite Schnittstellenkörper (3200b, 4200) derart austauschbar sind, dass der erste Schnittstellenkörper (3200a, 4200) in einer ersten Konfiguration mit dem Gehäuse (3400, 4400, 5400) gekoppelt ist und der zweite Schnittstellenkörper (3200b, 4200) in einer zweiten Konfiguration mit dem Gehäuse (3400, 4400, 5400) gekoppelt ist,
wobei jeder des ersten Schnittstellenkörpers (3200a, 4200a) und des zweiten Schnittstellenkörpers (3200b, 4200b) einen Verteiler (3240, 4240, 5240) umfasst, wobei der Verteiler (3240, 4240, 5240) konfiguriert ist, um mindestens teilweise durch das Gehäuse (3400, 4400, 5400) aufgenommen zu werden, wenn der erste Schnittstellenkörper (3200a, 4200a) oder der zweite Schnittstellenkörper (3200b, 4200b) mit dem Gehäuse (3400, 4400, 5400) lösbar gekoppelt ist, der Verteiler (3240, 4240, 5240) umfassend eine erste Öffnung (3246, 4246) und eine zweite Öffnung (3246, 4246), die in Kommunikation mit den Schnittstellenschläuchen stehen, wenn der erste Schnittstellenkörper (3200a, 4200) oder der zweite Schnittstellenkörper (3200b, 4200) mit dem Gehäuse (3400, 4400, 5400) lösbar gekoppelt ist, und wobei die Positionierung der ersten und der zweiten Öffnung (3246, 4246) laterale Eintrittspunkte für Gasströmung zu und von jedem des ersten Schnittstellenkörpers (3200, 4200) und des zweiten Schnittstellenkörpers (3200, 4200) definiert.

2. Patientenschnittstellenanordnung (3000, 4000, 5400) nach Anspruch 1, wobei die erste und die zweite Öffnung (3246, 4246) über den Verteiler (3240, 4240, 5240) miteinander in Fluidkommunikation stehen.

3. Patientenschnittstellenanordnung (3000, 4000, 5400) nach Anspruch 1 oder 2, wobei ein erster Schnittstellenschlauch (3800, 4800) des Paares von Schnittstellenschläuchen (8800, 4800) eine Inspirationsleitung (201a) eines Beatmungssystems umfasst oder für Verbindung mit dieser konfiguriert ist, und ein zweiter Schnittstellenschlauch (8800, 4800) des Paares von Schnittstellenschläuchen (8800, 4800) eine Expirationsleitung (201b) des Beatmungssystems umfasst oder für Verbindung mit dieser konfiguriert ist.

4. Patientenschnittstellenanordnung (3000, 4000) nach einem der vorstehenden Ansprüche, wobei die erste und die zweite Öffnung (3246, 4246) auf gegenüberliegenden Seiten des Verteilers (3240, 4240, 5240) sind.

5. Patientenschnittstellenanordnung (3000, 4000) nach einem der vorstehenden Ansprüche, wobei der erste Schnittstellenkörper (3200a, 4200) ein Kanülenkörper (3200a, 4200a) ist, und/oder wobei der zweite Schnittstellenkörper (3200b, 4200) ein Maskenkörper (3200b, 4200b) ist.

6. Patientenschnittstellenanordnung (3000, 4000) nach einem der vorstehenden Ansprüche, wobei jeder des ersten Schnittstellenkörpers (3200a, 4200) und des zweiten Schnittstellenkörpers (3200b, 4200) konfiguriert ist, um eine Abdichtung mit einem Gesicht eines Nutzers auszubilden.

7. Patientenschnittstellenanordnung (3000, 4000) nach einem der vorstehenden Ansprüche, wobei eine Oberkante (3470) und eine Unterkante (3490) des Gehäuses (3400, 4400, 5400) symmetrisch sind.

8. Patientenschnittstellenanordnung (3000, 4000) nach einem der vorstehenden Ansprüche, wobei der Verteiler (3240, 4240, 5240) einen Gehäusekopplungsabschnitt (3244, 4244), der konfiguriert ist, um innerhalb des Gehäuses (3400, 4400, 5400) aufgenommen zu werden, wenn der erste Schnittstellenkörper (3200a, 4200) oder der zweite Schnittstellenkörper (3200b, 4200) mit dem Gehäuse (3400, 4400, 5400) lösbar gekoppelt ist, umfasst.

9. Patientenschnittstellenanordnung (3000, 4000) nach Anspruch 8, wobei der Verteiler (3240, 4240, 5240) ferner einen erhabenen Abschnitt (3242, 4247) angrenzend an den Gehäusekopplungsabschnitt (3244, 4244) umfasst, wobei der erhabene Abschnitt (3242, 4247) konfiguriert ist, um eine Drehung des Verteilers (3240, 4240, 5240) um das Gehäuse (3400, 4400, 5400) zu hemmen, wenn der erste Schnittstellenkörper (3200a, 4200) oder der zweite Schnittstellenkörper (3200b, 4200) mit dem Gehäuse (3400, 4400, 5400) lösbar gekoppelt ist.

10. Patientenschnittstellenanordnung (3000, 4000) nach Anspruch 8 oder 9, wobei der Gehäusekopplungsabschnitt (3244, 4244) und der Verteiler (3240, 4240, 5240) geformt und/oder bemessen sind, um in das Gehäuse (3400, 4400) eingesetzt und durch dieses aufgenommen zu werden und eine gasdichte Abdichtung mit dem Gehäuse (3400, 4400) auszubilden.

11. Patientenschnittstellenanordnung (3000, 4000) nach einem der Ansprüche 8 bis 10, wobei der Gehäusekopplungsabschnitt (3244, 4244) eine Trapezform, die zwischen einer ersten patientzugewandten Seite und einer gegenüberliegenden zweiten Seite des Gehäusekopplungsabschnitts (3244, 4244) definiert ist, umfasst und das Gehäuse eine komplementäre Form zu der Trapezform des Gehäusekopplungsabschnitts (3244, 4244) umfasst.

12. Patientenschnittstellenanordnung (3000, 4000) nach einem der vorstehenden Ansprüche, wobei der Verteiler (3240, 4240, 5240) an jeder der ersten und der zweiten Öffnung (3246, 4246) eine Rippe (3248, 4248), die konfiguriert ist, um eine gasdichte Abdichtung mit dem Gehäuse (3400, 4400) auszubilden, umfasst.

13. Patientenschnittstellenanordnung (3000, 4000) nach Anspruch 12, wobei die Rippe eine ringförmige Rippe (3248, 4248) ist.

14. Patientenschnittstellenanordnung (3000, 4000) nach einem der vorstehenden Ansprüche, wobei der Verteiler (3240, 4240, 5240) einen optischen Indikator zum Anzeigen der korrekten Positionierung und einer richtigen Ausrichtung an dem Gehäuse umfasst.

15. Patientenschnittstellenanordnung (3000, 4000) nach Anspruch 14, wenn abhängig von Anspruch 12 oder 13, wobei der optische Indikator einen farbigen Ring (5604), der sich auf der oder um die Rippe (3248, 4248) des Verteilers (3240, 4240, 5240) befindet, umfasst.

## Revendications

1. Ensemble interface patient (3000, 4000) comprenant :
un premier corps d'interface (3200a, 4200) ;
un second corps d'interface (3200b, 4200) ;
un logement (3400, 4400, 5400) conçu pour s'accoupler de manière détachable au premier corps d'interface (3200a, 4200a) ou au second corps d'interface (3200b, 4200), comprenant
une paire de parties venant en prise avec le tube (3460) au niveau de points d'entrée latéraux sur des extrémités opposées du logement ; et
une paire de tubes d'interface (3800, 4800), chacun des tubes d'interface (3800, 4800) au niveau d'une extrémité étant accouplé à une partie venant en prise avec le tube de la paire de parties venant en prise avec le tube (3460), et permettant à du gaz provenant de l'utilisateur et allant vers celui-ci de s'écouler à travers le premier corps d'interface (3200a, 4200) ou le second corps d'interface (3200b, 4200) respectif lorsqu'il est accouplé de manière détachable au logement (3400, 4400, 5400),
dans lequel le premier corps d'interface (3200a, 4200) et le second corps d'interface (3200b, 4200) sont interchangeables, de telle sorte que le premier corps d'interface (3200a, 4200) est accouplé au logement (3400, 4400, 5400) dans une première configuration, et le second corps d'interface (3200b, 4200) est accouplé au logement (3400, 4400, 5400) dans une seconde configuration,
dans lequel chacun du premier corps d'interface (3200a, 4200a) et du second corps d'interface (3200b, 4200b) comprend un collecteur (3240, 4240, 5240), le collecteur (3240, 4240, 5240) étant conçu pour être au moins partiellement reçu par le logement (3400, 4400, 5400) lorsque le premier corps d'interface (3200a, 4200a) ou le second corps d'interface (3200b, 4200b) est accouplé de manière détachable au logement (3400, 4400, 5400), le collecteur (3240, 4240, 5240) comprenant une première ouverture (3246, 4246) et une seconde ouverture (3246, 4246) en communication avec les tubes d'interface lorsque le premier corps d'interface (3200a, 4200) ou le second corps d'interface (3200b, 4200) est accouplé de manière détachable au logement (3400, 4400, 5400) et dans lequel le positionnement des première et seconde ouvertures (3246, 4246) définit des points d'entrée latéraux pour un flux de gaz allant vers chacun du premier corps d'interface (3200, 4200) et du second corps d'interface (3200, 4200) et provenant de ceux-ci.

2. Ensemble interface patient (3000, 4000, 5400) selon la revendication 1, dans lequel les première et seconde ouvertures (3246, 4246) sont en communication fluidique l'une avec l'autre par l'intermédiaire du collecteur (3240, 4240, 5240).

3. Ensemble interface patient (3000, 4000, 5400) selon la revendication 1 ou 2, dans lequel un premier tube d'interface (3800, 4800) de la paire de tubes d'interface (8800, 4800) comprend ou est conçu pour une liaison à un conduit inspiratoire (201a) d'un système respiratoire, et un second tube d'interface (8800, 4800) de la paire de tubes d'interface (8800, 4800) comprend ou est conçu pour être relié à un conduit expiratoire (201b) du système respiratoire.

4. Ensemble interface patient (3000, 4000) selon l'une quelconque des revendications précédentes, dans lequel les première et seconde ouvertures (3246, 4246) sont sur des côtés opposés du collecteur (3240, 4240, 5240).

5. Ensemble interface patient (3000, 4000) selon l'une quelconque des revendications précédentes, dans lequel le premier corps d'interface (3200a, 4200) est un corps de canule (3200a, 4200a) et/ou dans lequel le second corps d'interface (3200b, 4200) est un corps de masque (3200b, 4200b).

6. Ensemble interface patient (3000, 4000) selon l'une quelconque des revendications précédentes, dans lequel chacun du premier corps d'interface (3200a, 4200) et du second corps d'interface (3200b, 4200) sont conçus pour former une étanchéité avec le visage d'un utilisateur.

7. Ensemble interface patient (3000, 4000) selon l'une quelconque des revendications précédentes, dans lequel un bord supérieur (3470) et un bord inférieur (3490) du logement (3400, 4400, 5400) sont symétriques.

8. Ensemble interface patient (3000, 4000) selon l'une quelconque des revendications précédentes, dans lequel le collecteur (3240, 4240, 5240) comprend une partie d'accouplement de logement (3244, 4244) conçue pour être reçue à l'intérieur du logement (3400, 4400, 5400) lorsque le premier corps d'interface (3200a, 4200) ou le second corps d'interface (3200b, 4200) est accouplé de manière détachable au logement (3400, 4400, 5400).

9. Ensemble interface patient (3000, 4000) selon la revendication 8, dans lequel le collecteur (3240, 4240, 5240) comprend en outre une partie surélevée (3242, 4247) adjacente à la partie d'accouplement de logement (3244, 4244), dans lequel la partie surélevée (3242, 4247) est conçue pour empêcher la rotation du collecteur (3240, 4240, 5240) autour du logement (3400, 4400, 5400) lorsque le premier corps d'interface (3200a, 4200) ou le second corps d'interface (3200b, 4200) est accouplé de manière détachable au logement (3400, 4400, 5400).

10. Ensemble interface patient (3000, 4000) selon la revendication 8 ou la revendication 9, dans lequel la partie d'accouplement de logement (3244, 4244) et le collecteur (3240, 4240, 5240) sont façonnés et/ou dimensionnés pour être insérés dans le logement (3400, 4400) et reçus par celui-ci et former une étanchéité aux gaz avec le logement (3400, 4400).

11. Ensemble interface patient (3000, 4000) selon l'une quelconque des revendications 8 à 10, dans lequel la partie d'accouplement de logement (3244, 4244) comprend une forme trapézoïdale définie entre un premier côté faisant face au patient et un second côté opposé de la partie d'accouplement de logement (3244, 4244), et le logement comprend une forme complémentaire à la forme trapézoïdale de la partie d'accouplement de logement (3244, 4244).

12. Ensemble interface patient (3000, 4000) selon l'une quelconque des revendications précédentes, dans lequel le collecteur (3240, 4240, 5240) comprend une cannelure (3248, 4248) au niveau de chacune des première et seconde ouvertures (3246, 4246) qui est conçue pour former une étanchéité aux gaz avec le logement (3400, 4400).

13. Ensemble interface patient (3000, 4000) selon la revendication 12, dans lequel la cannelure est une cannelure annulaire (3248, 4248).

14. Ensemble interface patient (3000, 4000) selon l'une quelconque des revendications précédentes, dans lequel le collecteur (3240, 4240, 5240) comprend un indicateur visuel pour indiquer un positionnement correct et un alignement approprié sur le logement.

15. Ensemble interface patient (3000, 4000) selon la revendication 14 lorsqu'elle dépend de la revendication 12 ou 13, dans lequel l'indicateur visuel comprend un anneau coloré (5604) situé sur la cannelure (3248, 4248) du collecteur (3240, 4240, 5240) ou autour de celle-ci.
